# EUROPÄISCHE PATENTSCHRIFT

(11) **EP 2 436 668 B1**
(45) Veröffentlichungstag und Bekanntmachung des Hinweises auf die Patenterteilung: **12.09.2012**
(21) Anmeldenummer: 11183333.1
(22) Anmeldetag: 29.09.2011
(51) Int. Cl.: C07C 275/60, C07C 275/62, A61K 6/083

(54) **Polymerisierbare Verbindungen umfassend ein polyalicyclisches Strukturelement**
Polymerisable compounds comprising a polyalicyclic structure element
Composés polymérisables comprenant un élément structurel polyalicyclique

(30) Priorität: 30.09.2010 DE 102010041792
(43) Veröffentlichungstag der Anmeldung: 04.04.2012
(73) Patentinhaber: VOCO GmbH, 27472 Cuxhaven (DE)
(72) Erfinder: Stepputtis, Dr. Manfred, 27449 Kutenholz (DE); Blömker, Dr. Tobias, 27472 Cuxhaven (DE); Plaumann, Manfred Thomas, 27474 Cuxhaven (DE); Maletz, Dr. Reinhard, 27472 Cuxhaven (DE); Fontein, Dr. Nils, 27472 Cuxhaven (DE)
(74) Vertreter: Eisenführ, Speiser & Partner

(56) Entgegenhaltungen:
- DE-A1- 3 522 006
- US-B1- 6 670 499

## Beschreibung

Die vorliegende Erfindung betrifft neue polymerisierbare Verbindungen (nachfolgend auch als Monomere bezeichnet) umfassend ein polyalicyclisches Strukturelement und bestimmte funktionelle Gruppen, Mischungen umfassend eine oder mehrere dieser Verbindungen und entsprechende härtbare Gemische und Erzeugnisse sowie deren jeweilige Verwendung als Dentalmaterial bzw. zur Herstellung eines Dentalmaterials. Die Erfindung betrifft ferner ein Verfahren zur Herstellung dieser Verbindungen bzw. Mischungen und ein Verfahren zur Herstellung eines Erzeugnisses, vorzugsweise eines für den Dentalbereich geeigneten Erzeugnisses.

Härtbare Monomere umfassend ein polyalicyclisches Strukturelement sind grundsätzlich bekannt und werden in zahlreichen Anwendungsgebieten, z.B. in der Dentaltechnik, eingesetzt.

Unter (Meth)acryl ist im Rahmen des vorliegenden Textes sowohl Acryl als auch Methacryl zu verstehen.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

DE 4314252 A1 (entsprechend US 5,580,947) betrifft ein Verfahren zur Herstellung von olefinisch ungesättigten Isocyanaten durch Umsetzung von Isophorondiisocyanat und ausgewählten olefinisch ungesättigten Carbonsäuren. Die resultierenden olefinisch ungesättigten Isocyanate eignen sich zur Verwendung als Bindemittel in Beschichtungsmaterialien.

US 3,517,039 beschreibt acylierte Harnstoffpolyisocyanate, hergestellt mittels Reaktion organischer Diisocyanate mit organischen Monocarbonsäuren. Die in US 3,517,039 beschriebenen Produkte können zur Herstellung von wärmestandfesten Schaumstoffen oder von Polyurethanelastomeren sowie insbesondere von elastifizierten Lacküberzügen.

EP 0 611 752 A1 offenbart ein Verfahren zur Herstellung von olefinisch ungesättigten, Urethangruppen aufweisenden Isocyanaten unter Einhaltung eines bestimmten NCO/OH-Äquivalentenverhältnisses. Die gemäß EP 0 611 752 A1 erhältlichen Isocyanate können als Bindemittel für bei Raumtemperatur einkomponentig zu verarbeitende Beschichtungsmaterialien eingesetzt werden.

EP 0 209 700 A2, DE 35 22 006 und DE 35 22 005 beschreiben (Meth)acrylsäureDerivate bestimmter Tricyclodecane mit zweibindigen Brückengliedern aus der Gruppe der Urethane bzw. Harnstoffe, welche im Dentalbereich eingesetzt werden können.

EP 0 000 194 A1 (entsprechend US 4,160,080) beschreibt Polyisocyanate, die Allophanatgruppen enthalten. Diese Allophanatpolyisocyanate können zur Herstellung von Polyurethan-Schaumstoffen, Elastomeren, Duromeren, Beschichtungen, Verklebungen und Lackierungen eingesetzt werden.

EP 0 682 012 B1 betrifft ein Verfahren zur Herstellung von hellfarbigen lichtechten Allophanatgruppen aufweisenden (cyclo)aliphatischen Polyisocyanaten, durch Umsetzung von Urethangruppen aufweisenden organischen Verbindungen mit organischen Polyisocyanaten mit (cyclo)aliphatisch gebundenen Isocyanatgruppen in Gegenwart von Zinn(II)-salzen. Die in EP 0 682 012 B1 beschriebenen Polyisocyanate können als Aufbaukomponente bei der Herstellung von Polyurethankunststoffen verwendet werden.

EP 1 727 846 B1 offenbart ein Verfahren zur Herstellung von Allophanatgruppen enthaltenden Bindemitteln, die unter Einwirkung aktinischer Strahlung mit ethylenisch ungesättigten Verbindungen unter Polymerisation reagierende Gruppen und gegebenenfalls NCO-reaktive Gruppen aufweisen.

EP 0 712 840 B1 betrifft ein Verfahren zur Herstellung von bestimmten Allophanatgruppen aufweisenden Polyisocyanaten durch Umsetzung unter Allophanatbildung von Urethangruppen aufweisenden Verbindungen. Die Verbindungen gemäß EP 0 712 840 B1 können als Bindemittel oder Bindemittelkomponente in Beschichtungsmitteln verwendet werden.

EP 0 867 457 B1 offenbart ein ethylenisch ungesättigtes Polyurethan, das im Wesentlichen frei von Isocyanatgruppen ist, welches das Reaktionsprodukt eines ethylenisch ungesättigten Polyisocyanats, das Allophanatgruppen und β,γ-ethylenisch ungesättigte Ethergruppen enthält, mit einer hydroxyfunktionellen, ethylenisch ungesättigten Verbindung ist, wobei das ethylenisch ungesättigte Polyisocyanat durch Allophanatierung des Urethangruppen-enthaltenden Reaktionsprodukts eines organischen Diisocyanats mit einem β,γ-ethylenisch ungesättigten Etheralkohol hergestellt wird, wobei der β,γ-ethylenisch ungesättigte Etheralkohol eine Verbindung umfasst, die aus der aus Glycerindiallylether, Trimethylolpropandiallylether und Pentaerythtrittriallylether bestehenden Gruppe ausgewählt ist. Die in EP 0 867 457 B1 offenbarten ethylenisch ungesättigten Polyurethane mit Allophanatgruppen können als Bindemittel in Einkomponenten-Beschichtungszusammensetzungen verwendet werden.

DE 10 2007 040 240 A1 und EP1 645 582 A1 beschreiben jeweils Verfahren zur Herstellung von strahlenhärtenden Allophanaten durch Umsetzung von isocyanatgruppenhaltigen Verbindungen und hydroxyfunktionellen Verbindungen, wobei das Verhältnis von NCO-Gruppen zu den OH-Gruppen 1,45 : 1,0 bis 1,1 : 1,0 beträgt. Gemäß DE 10 2007 040 239 A1 werden unter Einsatz von bestimmten Mischungen enthaltend Hydroxyethylacrylat und Hydroxypropylacrylat als hydroxyfunktionellen Verbindungen entsprechende strahlenhärtenden Allophanate erhalten. Die strahlenhärtenden Allophanate gemäß dieser drei Dokumente können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden.

DE 10 2004 060 285 A1 betrifft strahlungshärtbare Zusammensetzungen auf Basis eines Dicidolgemisches (enthaltend zwei oder drei isomere 3,8-, 4,8- und/oder 5,8-Bis(hydroxymethyl)tricyclo[5.2.1.0^{2,6}]decane) mit mindestens einer Verbindung, welche mindestens eine ethylenisch ungesättigte Gruppierung mit gleichzeitig mindestens einer gegenüber Dicidol reaktive Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das Umsetzungsprodukt eines Dicidolgemisches und mindestens einer Verbindung, welche mindestens eine ethylenisch ungesättigte Gruppierung mit gleichzeitig mindestens einer gegenüber Dicidol reaktive Gruppierung aufweist. Die Anwendungsgebiete entsprechen den in DE 10 2004 060 285 A1 genannten.

WO 03/035013 A1 und DE 602 16 951 T2 betreffen Dentalkleberzusammensetzungen zum Binden von Dental-Restaurierungsmitteln an Dentin und/oder Zahnschmelz. In diesen Dokumenten wird unter anderem die Herstellung von 3,(4),8,(9)-Bis(2-propenamidomethyl)tricyclo[5.2.1.0]^{2,6}-decan beschrieben.

US 6,670,499 B1 beschreibt vom Adamantan abgeleitet Diurethane. Die in US 6,670,499 beschriebenen Verbindungen eignen sich als Intermediate für den Einsatz im dentalen Bereich oder zur Herstellung von optischen Materialien (wie beispielsweise Linsen).

Auf dem Gebiet der Dentaltechnik und für diverse andere Anwendungszwecke besteht ein ständiger Bedarf nach weiteren polymerisierbaren Monomeren. Insbesondere besteht ein Bedarf nach Monomeren, die die Herstellung von Erzeugnissen und Polymeren mit verbesserten Eigenschaften, beispielsweise einer erhöhten Hydrophobie und/oder einer höheren mechanischen Stabilität ermöglichen.

Primäre Aufgabe der vorliegenden Erfindung ist es, neue polymerisierbare Monomere anzugeben, die insbesondere für Anwendungen in der Dentaltechnik geeignet sind, ohne dabei auf dieses Einsatzgebiet beschränkt zu sein. Vorzugsweise sollen die unter Verwendung der erfindungsgemäßen Monomeren erhältlichen Polymere eine ausgeprägte Hydrophobie aufweisen, die sich u.a. in einer sehr geringen Wasseraufnahme zeigt. Ebenfalls bevorzugt sollen sich die unter Verwendung der erfindungsgemäßen Monomeren erhältlichen Polymere durch eine hohe mechanische Stabilität auszeichnen, die sich u.a. in einer hohen Biegefestigkeit zeigt. Besonders bevorzugt sollen sich unter Verwendung der erfindungsgemäßen Monomere solche Polymere herstellen lassen, die sowohl eine geringe Wasseraufnahme als auch eine hohe Biegefestigkeit aufweisen.

Diese Aufgaben werden gelöst durch eine Verbindung der Struktur Q(YZₑ)_{b} mit ein, zwei, drei, vier oder mehr funktionellen Gruppen ausgewählt aus der Gruppe bestehend aus N-Acylharnstoff, Allophanat und Biuret, wobei hier und nachfolgend gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten YZₑ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind;
- b ist eine natürliche Zahl ausgewählt aus der Gruppe der natürlichen Zahlen 2, 3, 4;
- jedes Z bedeutet ein Strukturelement, das unabhängig von etwaigen weiteren Strukturelementen Z ausgewählt ist aus der Gruppe bestehend aus

   -O-(C=O)-CH=CH₂, -O-(C=O)-C(CH₃)=CH₂,

   -(C=O)-CH=CH₂ und -(C=O)-C(CH₃)=CH₂,
- jeder Index e ist eine natürliche Zahl, die unabhängig von etwaigen weiteren Indizes e ausgewählt ist aus der Gruppe der natürlichen Zahlen 1, 2, 3 und 4;
- jedes Y bedeutet ein Strukturelement, welches in der Struktur Q(YZₑ)_{b} das polyalicyclische Strukturelement Q mit e Strukturelementen Z verbindet;
wobei die Verbindung herstellbar ist durch Umsetzung eines ersten Umsetzungsproduktes, welches das Umsetzungsprodukt ist aus einer ersten Reaktion von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH,
   wobei gilt:
   - R bedeutet jeweils unabhängig von etwaigen weiteren R ein Wasserstoffatom oder einen Alkylrest;
   - m ist eine natürliche Zahl ausgewählt aus der Gruppe der natürlichen Zahlen von 0 bis 10,
   - jeder Index n ist eine natürliche Zahl, die unabhängig von etwaigen weiteren Indizes n ausgewählt ist aus der Gruppe bestehend aus 0 und 1,
   wobei die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
   oder
   die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Im Bereich der Dentaltechnik werden an dentale Polymere besondere Anforderungen gestellt, wie beispielsweise eine gute Bioverträglichkeit, eine geringe Toxizität des Monomeren (für den Fall, dass es nicht auspolymerisiert, sondern teilweise als Monomer in der Polymermatrix verbleibt), ein geringer Restmonomergehalt, etc..

Die unter Verwendung der erfindungsgemäßen Monomeren erhältlichen Polymere weisen eine ausgeprägte Hydrophobie auf, die sich u.a. in einer sehr geringen Wasseraufnahme der Polymere zeigt. Daneben zeichnen sich die unter Verwendung der erfindungsgemäßen Monomeren erhältlichen Polymere durch eine hohe mechanische Stabilität aus, die sich u.a. in einer hohen Biegefestigkeit der Polymere zeigt. Die erfindungsgemäßen Monomeren, insbesondere gemäß den besonders bevorzugten Ausgestaltungen und Ausführungsformen, lassen sich zu Polymeren verarbeiten, die sowohl eine geringe Wasseraufnahme als auch eine hohe Biegefestigkeit aufweisen. Das polyalicyclische Strukturelement Q, insbesondere in den bevorzugten und besonders bevorzugten Ausgestaltungen, trägt zu der hohen Hydrophobie bei, die sich u.a. in einer sehr geringen Wasseraufnahme der Polymere zeigt.

Es wurde gefunden, dass die erfindungsgemäßen Monomere gut verarbeitbar sind. Die erfindungsgemäßen Monomere sind homo- oder copolymerisierbar, wobei die ausgehärteten Polymere bzw. Formstoffe einen geringen Schrumpf, eine gute Haftung auf verschiedenen Untergründen, eine hohe Hydrolysebeständigkeit, eine geringe Wasseraufnahme, eine hohe mechanische Festigkeit und einen hohen Glanz aufweisen. Die genannten Eigenschaften sind insbesondere im Bereich der Dentaltechnik wichtig.

Insbesondere die bevorzugten und besonders bevorzugten erfindungsgemäßen Verbindungen ermöglichen einen hohen Vernetzungsgrad und sind ferner vorzugsweise radikalisch vernetzbar. Wegen ihrer hochfunktionalisierten Struktur weisen sie eine hohe Vernetzungs- und Polymerisationswahrscheinlichkeit auf.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Ein erfindungsgemäßes Monomer umfasst mindestens ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten YZₑ ersetzt sind (wie oben beschrieben), und optional ein, zwei oder mehr der nicht durch Substituenten YZₑ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Die Struktur unsubstituierter Bicyclen ist wie folgt: 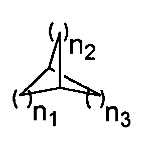 wobei n₁, n₂ und n₃ jeweils unabhängig voneinander eine natürliche Zahl von 1 bis 8 bedeuten können, vorzugsweise eine natürliche Zahl von 1 bis 4.

Exemplarisch genannt seien hier:
- Für n₁ = n₂ = 1; n₃= 2: Bicyclo[2.1.1]hexan
- für n₁ =1; n₂ = n₃= 2: Bicyclo[2.2.1]heptan
- für n₁ = n₂ = 1; n₃= 3: Bicyclo[3.1.1]heptan
- für n₁ = n₂ = n₃= 2: Bicyclo[2.2.2]octan
- für n₁ = n₂ = 1; n₃ = 4: Bicyclo[4.1.1]octan
- für n₁ =1; n₂ = 2; n₃= 3: Bicyclo[3.2.1]octan
- für n₁ =1; n₂ = 2; n₃= 4: Bicyclo[4.2.1]nonan
- für n₁ = n₂ = 2; n₃= 4: Bicyclo[4.2.2]decan

Nachfolgend werden exemplarisch einige disubstituierte Bicyclen gezeigt: 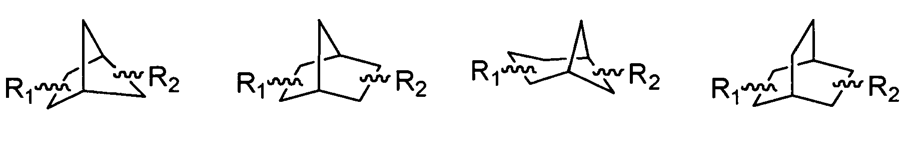 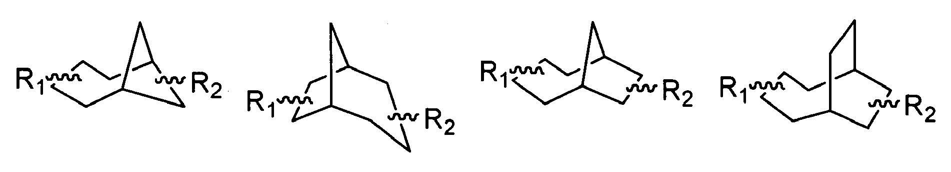 wobei R1 und R2 jeweils die sonstigen Reste der Verbindung bedeuten.

Beispiele für bicyclische Strukturelemente Q sind das Bicyclo[1.1.1]pentan-, das Bicyclo[2.1.1]hexan-, das Bicyclo[2.2.1]heptan-, das Bicyclo[3.1.1]heptan-, das Bicyclo[2.2.2]octan-, das Bicyclo[4.1.1]octan-, das Bicyclo[3.2.1]octan-, das Bicyclo[4.2.1]nonan-, das Bicyclo[3.3.1]nonan-, das Bicyclo[5.1.1]nonan-, das Bicyclo[3.2.2]nonan-, das Bicyclo[6.1.1]decan-, das Bicyclo[5.2.1]decan-, das Bicyclo[4.2.2]decan-, das Bicyclo[3.3.2]decan-, das Bicyclo[7.1.1]undecan-, das Bicyclo[6.2.1]undecan-, das Bicyclo[5.2.2]undecan-, das Bicyclo[4.3.2]undecan-, das Bicyclo[3.3.3]undecan-, das Bicyclo[8.1.1]dodecan-, das Bicyclo[7.2.1]dodecan-, das Bicyclo[6.2.2]dodecan-, das Bicyclo[5.3.2]dodecan-, das Bicyclo[4.3.3]dodecan-, das Bicyclo[4.4.2]dodecan-, das Bicyclo[5.4.1]dodecanstrukturelement sowie noch höhere Strukturelemente wie die entsprechenden Tridecane, Tetradecane, Pentadecane, etc.

Für unsubstituierte Tricyclen sind z.B. folgende Strukturen möglich: 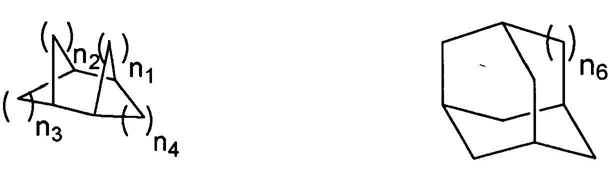 wobei n₁, n₂, n₃, n₄ bzw. n₆ jeweils unabhängig voneinander eine natürliche Zahl von 0 bis 5 bedeuten können.

Exemplarisch genannt seien:
- Für n₁ = 2; n₂ = 0; n₃= 2; n₄ = 3: Tricyclo[4.3.2.0^{2,5}]undecan
- für n₁ = 0; n₂ = 1; n₃= 2; n₄ = 3: Tricyclo[5.2.1.0^{2,6}]decan
- für n₁ = 0; n₂ = 2; n₃= 2; n₄ = 3: Tricyclo[5.2.2.0^{2,6}]undecan
- für n₁ = 2; n₂ = 0; n₃= 2; n₄ = 2: Tricyclo[4.2.2.0^{2,5}]decan
- für n₆ = 1: Thcyclo[3.3.1.1^{3,7}]decan

Nachfolgend werden exemplarisch einige di- oder trisubstituierte Tricyclen gezeigt: 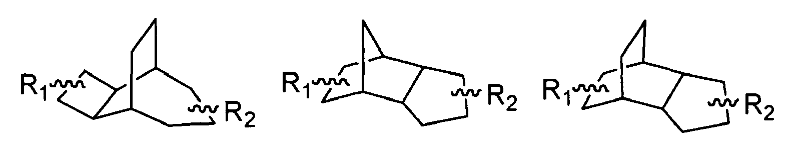 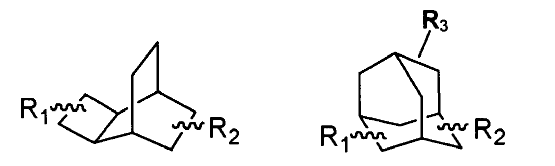 wobei R1, R2 und R3 jeweils die sonstigen Reste der Verbindung bedeuten.

Beispiele für tricyclische Strukturelemente Q sind das Tricyclo[3.2.1.0^{2,6}]octan-, das Tricyclo[4.2.1.0^{2,6}]nonan-, das Tricyclo[5.2.1.0^{2,6}]decan-, das Tricyclo[6.2.1.0^{2,6}]undecan-, das Tricyclo[7.2.1.0^{2,6}]dodecan-, oder das Tricyclo[4.2.1.1^{2,5}]decan-, das Tricyclo[4.3.1.1 ^{2,5}]decan-, das Tricyclo[4.4.1.1 ^{2,5}]decan-, das Tricyclo[2.2.1.0^{2,6}]heptan-, das Tricyclo[2.2.2.0^{2,6}]octan-, das Tricyclo[3.2.2.0^{2,6}]nonan-, das Tricyclo[3.3.1.1^{3,7}]decan-, das Tricyclo[3.2.1.1^{3,7}]nonan-, das Tricyclo[4.2.2.2^{2,5}]dodecan-, das Tricyclo[4.3.2.2^{2,5}]tridecan-, das Tricyclo[4.4.2.2^{2,5}]tetradecan-, das Tricyclo[4.2.1.0 ^{3,7}]nonan-, das Tricyclo[4.4.1.1^{1,5}]dodecan-, das Tricyclo[6.2.1.0^{2,7}]undecan-, das Tricyclo[5.2.2.0^{2,6}]undecan, das Tricyclo[6.2.2.0^{2,7}]dodecan-, Tricyclo[4.3.2.0^{2,5}]undecan-, das Tricyclo[4.2.2.0^{2,5}]decan- oder das Tricyclo[5.5.1.0^{3,11}]tridecanstrukturelement.

Für unsubstituierte Tetracyclen ist z.B. die folgende Struktur möglich: 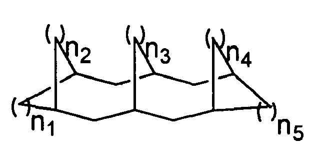 wobei n₁, n₂, n₃, n₄ und n₅ jeweils unabhängig voneinander eine natürliche Zahl von 1 bis 5 bedeuten können.

Exemplarisch genannt seien hier:
- Für n₁ = n₂ = n₃ = n₄ = 2; n₅ = 5: Tetracyclo[9.6.2^{3,9}.2^{13,16}]tricosan
- für n₁ = n₅ = 2; n₂ = n₃ = n₄ = 1: Tetracyclo[6.6.1^{3,6}.1^{10,13}]heptadecan
- für n₁ = n₂ = n₃= n₄ = n₅ = 2: Tetracyclo[6.6.2^{3,6}.2^{10,13}]eicosan

Nachfolgend werden exemplarisch einige disubstituierte Tetracyclen gezeigt 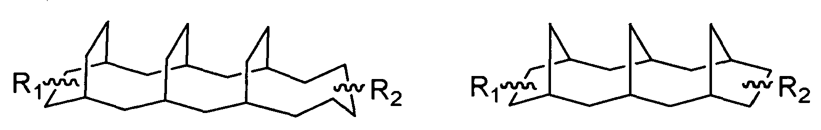 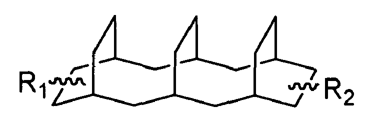 wobei R1 und R2 jeweils die sonstigen Reste der Verbindung bedeuten.

Beispiele für tetracyclische Strukturelemente Q sind das Tetracyclo[4.4.2.2^{2,5}.1^{7,10}]pentadecan-, das Tetracyclo[5.5.2.2^{2,6}.1^{8,12}]heptadecan-, das Tetracyclo[6.6.2.2^{2,7}.1^{9,14}]nonadecan-, das Tetracyclo[4.4.2.2^{2,5}.2^{7,10}]hexadecan-, das Tetracyclo[5.4.2.2²,⁶.1⁸,¹¹] hexadecan-, das Tetracyclo[6.2.1.1^{3,6}.0^{2,7}]dodecan, das Tetracyclo[9.6.2^{3,9}.2^{13,6}]tricosan-, das Tetracyclo[9.6.2^{3,9}.2^{13,16}]tricosan-, das Tetracyclo[6.6.1^{3,6}.1^{10,13}]heptadecan-, das Tetracyclo[6.6.2^{3,6}.2^{10,13}]cosan-, oder das Tetracyclo[5.3.2.1^{2,4}.0^{3,6}]tridecanstrukturelement.

Beispiele für pentacyclische Strukturelemente Q sind das Pentacyclo[4.2.0.0^{2,5}.0^{3,8}.0^{4,7}]octan-, das Pentacyclo[13.7.4.^{3,8}.0^{18,20}.1^{13,28}]triacontan-, das Pentacyclo[8.6.6.5^{2,9}.1^{23,26}]octacosan oder das Pentacyclo[3.3.0.0^{2,4}.0^{3,7}.0^{6,8}]octan-Strukturelement.

Ein Beispiel eines hexacyclischen Strukturelementes Q ist das Hexacyclo[15.3.2.2^{3,7}.1^{2,12}.0^{13,21}.0^{11,25}]pentacosan.

Bevorzugte Verbindungen gemäß A) zur Herstellung der erfindungsgemäßen Monomere sind beispielsweise:
Carbonsäuresubstituierte polyalicyclische Kohlenwasserstoffe:
   Bicyclo[3.2.2]nonan-6,7-dicarbonsäure,
   Bicyclo[3.3.1]nonan-1,5-dicarbonsäure,
   Tricyclo[3.2.2.0^{2,4}]non-8-en-6,7-dicarbonsäure,
   Bicyclo(2.2.1)-2,5-heptadien-2,3-dicarbonsäure,
   Bicyclo[2.2.1]heptan-5,6-dicarbonsäure,
   Norbornan-2,3-dicarbonsäure,
   Bicyclo[2.2.1]hept-5-en-2,3-dicarbonsäure,
   Pentacyclo[4.4.0.0^{2,5}.0^{3,8}.0^{4,7}]decan-2,4-dicarbonsäure,
   Bicyclo[2.2.2]octan-1,4-dicarbonsäure, etc..
Alkoholsubstituierte polyalicyclische Kohlenwasserstoffe:
   Bicyclo(2.2.1)heptan-2,7-diol,
   [5-(Hydroxymethyl)-6-bicyclo[2.2.1]hept-2-enyl] -methanol,
   Tricyclo[3.3.1.1^{3,7}]decan-1,3-diethanol,
   Tetracyclo[6.3.0.0^{2,6}.0^{5,9}]undecan-3,11-diol,
   [6-(Hydroxymethyl)-6-bicyclo[2.2.1]hept-2-enyl]methanol,
   Tricyclo[3.3.^{13,7}]decan-1,3-diol,
Bis(hydroxymethyl)tricyclo[5.2.1.0^{2,6}]decan, etc..
   Isocyanatsubstituierte polyalicyclische Kohlenwasserstoffe:
   Bis(2-isocyanatoethyl)-5-norbornen-2,3-dicarboxylate,
   2,5 (2,6)-Bis(isocyanatomethyl)bicyclo[2.2.1]heptan,
   Bis(isocyanatomethyl)tricyclo[5.2.1.0^{2,6}]decan, etc..
Gemischt- oder Aminosubstituierte polyalicyclische Kohlenwasserstoffe:
   Tricyclo[2.2.1.0^{2,6}]heptan-1,3-dicarbonsäure-3-amino,
   Tricyclo[3.3.1.1^{3,7}]decan-1-ol-3-amino,
   Pentacyclo[4.3.0.0^{2,5}.0^{3,8}.0^{4,7}]nonan-2,4-diamin,
   Tricyclo[3.3.1.1^{3,7}]decan-1-ol-3-amino,
   Bis(aminomethyl)tricyclo[5.2.1.0^{2,6}]decan, etc..

In einer bevorzugten Ausführungsform leitet sich die Struktur des polyalicyclischen Strukturelements Q von einem bicyclischen [a.c.d] Kohlenwasserstoff ab. Die Buchstaben a, c und d sind natürliche Zahlen und haben die Bedeutung der IUPAC-Nomenklatur. Die Summe von a, c und d liegt vorzugsweise im Bereich von 3 bis 13, bevorzugt im Bereich von 4 bis 7.

In einer weiteren bevorzugten Ausführungsform leitet sich die Struktur des polyalicyclischen Strukturelements von einem tricyclischen [a.c.d.f]-Kohlenwasserstoff ab. Die Summe von a, c, d und f liegt vorzugsweise im Bereich von 6 bis 12, bevorzugt im Bereich von 7 bis 9.

In einer bevorzugten Ausführungsform leitet sich die Struktur des polyalicyclischen Strukturelements von einem tricyclischen [a.2.1.0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricydischen[a.2.2.0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen[a.3.1.1] Kohlenwasserstoff ab, wobei a jeweils die Zahl 3, 4, 5, 6 oder 7 bedeuten kann.

Sofern ein erfindungsgemäßes Monomer zwei oder mehrere polyalicyclische Strukturelemente Q umfasst, können diese identisch oder verschieden sein.

Bevorzugt sind erfindungsgemäße Verbindungen, in denen
(i) das Strukturelement Z = -O-(C=O)-C(CH₃)=CH₂ bedeutet, wobei die funktionellen Gruppen Allophanat-, Biuret- oder Acylharnstoff-Gruppen sind, da mit diesen Verbindungen besonders gute Ergebnisse erzielt wurden (siehe hierzu auch die Beispiele),
   und/oder
(ii) das Strukturelement Q einen Tricyclo[5.2.1.0^{2,6}]decan-Rest bedeutet.

Weiter bevorzugt sind erfindungsgemäße Verbindungen, in denen das Strukturelement Z = -O-(C=O)-C(CH₃)=CH₂ bedeutet, wobei die funktionellen Gruppen Allophanat-, Biuret- oder Acylharnstoff-Gruppen sind und das Strukturelement Q einen Tricyclo[5.2.1.0^{2,6}]decan-Rest bedeutet.

Bevorzugt sind erfindungsgemäße Verbindungen, in denen sämtliche vorhandenen lichthärtbaren Gruppen dem Strukturelement Z entsprechen.

Bevorzugt sind erfindungsgemäße Verbindungen, in denen sämtliche vorhandenen endständigen polymerisierbaren Gruppen dem Strukturelement Z entsprechen.

Eine erfindungsgemäße Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Bevorzugte erfindungsgemäße Verbindungen umfassen zwei, drei, vier oder mehr funktionelle Gruppen ausgewählt aus der Gruppe bestehend aus N-Acylharnstoff, Allophanat, und Biuret.

Erfindungsgemäß bevorzugt sind Verbindungen mit ein, zwei, drei, vier oder mehr funktionellen Gruppen ausgewählt aus der Gruppe bestehend N-Acylharnstoff, Allophanat, und Biuret.

Die vorliegende Erfindung betrifft auch eine Verbindung der Struktur Q(YZₑ)_{b} mit ein, zwei, drei, vier oder mehr funktionellen Gruppen, welche ausgewählt sind aus der Gruppe bestehend aus N-Acylharnstoff, Allophanat und Biuret,
wobei gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten YZₑ substituierten Wasserstoffatome dieses polyalicyclischen Strukturelements Q durch Alkylgruppen, Alkoxygruppen, Halogenatome oder Trifluormethylgruppen substituiert ist bzw. sind;
- b ist eine natürliche Zahl ausgewählt aus der Gruppe der natürlichen Zahlen 2, 3, 4;
- jedes Z bedeutet ein Strukturelement, das unabhängig von etwaigen weiteren Strukturelementen Z ausgewählt ist aus der Gruppe bestehend aus

   -O-(C=O)-CH=CH₂, -O-(C=O)-C(CH₃)=CH₂,

   -(C=O)-CH=CH₂, -(C=O)-C(CH₃)=CH₂;

   -CH=CH₂ und -C(CH₃)=CH₂;
- jeder Index e ist eine natürliche Zahl, die unabhängig von etwaigen weiteren Indizes e ausgewählt ist aus der Gruppe der natürlichen Zahlen 1, 2, 3 und 4;
- jedes Y bedeutet ein Strukturelement, welches in der Struktur Q(YZₑ)_{b} das polyalicyclische Strukturelement Q mit e Strukturelementen Z verknüpft und ein Strukturelement enthält oder aus diesem besteht, ausgewählt aus der Gruppe bestehend aus 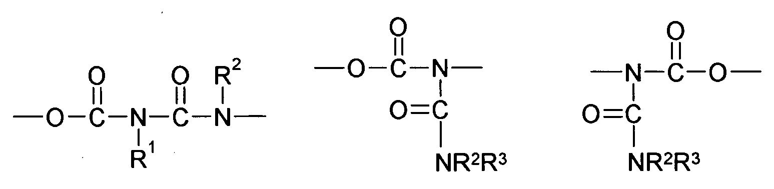 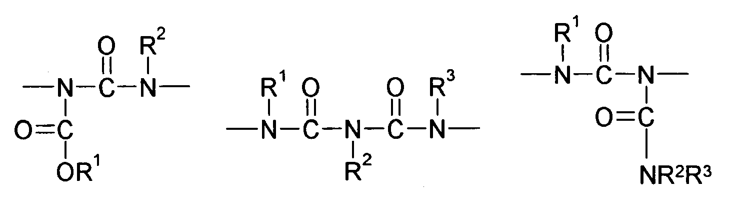 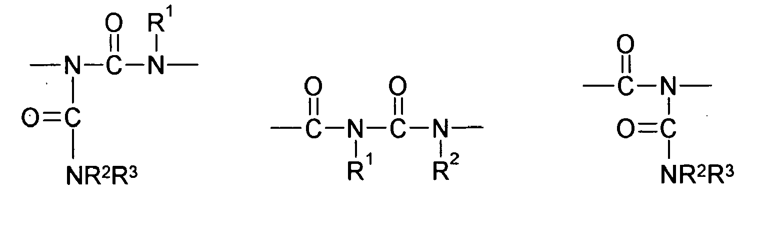 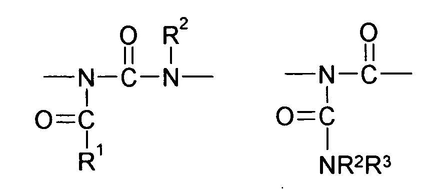
wobei R¹, R² und R³ sonstige Reste der Verbindung bedeuten, wobei die jeweils im Formelbild links angeordnete Bindung dem Strukturelement Q und die rechts angeordnete Bindung dem Strukturelement Z näher ist.

Vorzugsweise umfasst eine solche erfindungsgemäße Verbindung der Struktur Q(YZₑ)_{b} zwei, drei, vier oder mehr funktionelle Gruppen ausgewählt aus der Gruppe bestehend aus N-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste R¹, R² bzw. R³ einer erfindungsgemäßen Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste R¹, R² bzw. R³ einer erfindungsgemäßen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste R¹, R² bzw. R³ einer erfindungsgemäßen Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine erfindungsgemäße Verbindung ist vorzugsweise herstellbar durch Umsetzung eines ersten Umsetzungsproduktes, welches das Umsetzungsprodukt ist aus einer ersten Reaktion von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH
   mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH,
   wobei gilt:
   - R bedeutet jeweils unabhängig von etwaigen weiteren R ein Wasserstoffatom oder einen Alkylrest;
   - m ist eine natürliche Zahl ausgewählt aus der Gruppe der natürlichen Zahlen von 0 bis 10,
   - jeder Index n ist eine natürliche Zahl, die unabhängig von etwaigen weiteren Indizes n ausgewählt ist aus der Gruppe bestehend aus 0 und 1,
      wobei die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
   oder
   die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Eine erfindungsgemäße Verbindung gemäß einer bevorzugten Ausgestaltung ist ein zweites Umsetzungsprodukt aus einer Reaktion von dem obigen ersten Umsetzungsprodukt mit
C) einer weiteren Verbindung gemäß A) oder B), wobei jede weitere Verbindung gemäß A) bzw. B) dieselbe Bedeutung hat wie in der ersten Reaktion,
   und/oder
   wobei die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0. Dies gilt für sämtliche Aspekte der vorliegenden Erfindung.

In einer bevorzugten Ausgestaltung umfasst eine erfindungsgemäße Verbindung (wie oben definiert) ein oder mehrere Strukturelemente ausgewählt aus der Gruppe bestehend aus 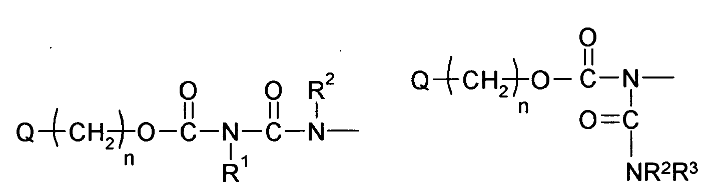 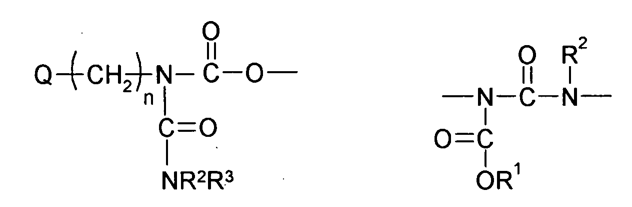 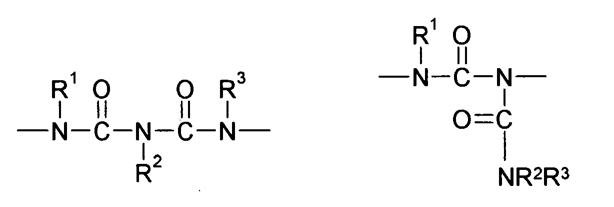 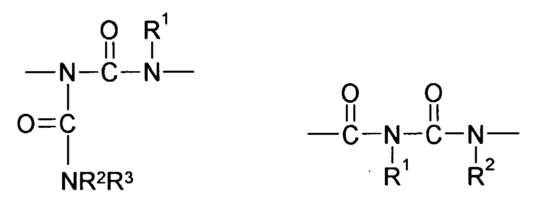 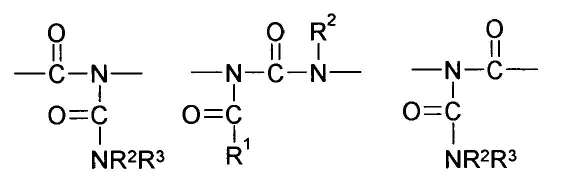 wobei R¹, R² und R³ sonstige Reste der Verbindung bedeuten (und vorzugsweise die oben genannten bevorzugte Bedeutung haben) und Q die oben genannte Bedeutung hat und der Index n ausgewählt ist aus der Gruppe bestehend aus 0 und 1.

Die jeweils im Formelbild rechts angeordnete Bindung ist dem Strukturelement Z näher.

Bevorzugte erfindungsgemäße Verbindungen sind solche, wobei Q ein polyalicyclisches Strukturelement bedeutet, vorzugsweise ein gesättigtes polyalicyclisches Strukturelement, das ausgewählt ist aus der Gruppe bestehend aus bicyclischen und tricyclischen Kohlenwasserstoffresten, wobei vorzugsweise keines der nicht durch Substituenten YZₑ substituierten Wasserstoffatome dieses polyalicyclischen Strukturelements Q substituiert ist.

Ein bevorzugter bicyclischer Kohlenwasserstoffrest ist ein Bicyclo[2.2.1]heptan-Rest, d.h. bevorzugt sind erfindungsgemäße Verbindungen, die ein Bicyclo[2.2.1]heptan (Norbornan) - Gerüst aufweisen.

In einer weiteren bevorzugten Ausführungsform leitet sich die Struktur des polyalicyclischen Strukturelements Q von einem Tricyclodecan- bzw. Tricyclodecen-Kohlenwasserstoffrest ab.

Besonders bevorzugt sind erfindungsgemäße Monomere Q(YZₑ)_{b}, deren polyalicyclisches Strukturelement Q sich von einem der folgenden tricyclischen Kohlenwasserstoffe ableitet: Tricycio[5.2.1.0^{2,6}]decan (TCD), Tricyclo[5.2.1.0^{2,6}]dec-3-en oder Tricyclo[3.3.1.1^{3,7}]decan (Adamantan), d.h. bevorzugt sind erfindungsgemäße Verbindungen, die ein TCD - Gerüst, ein Tricyclo[5.2.1.0^{2,6}]dec-3-en - Gerüst oder ein Adamantan-Gerüst aufweisen.

Besonders bevorzugte erfindungsgemäße Verbindungen sind solche, wobei das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet.

Bei den genannten besonders bevorzugten erfindungsgemäßen Verbindungen, in denen das Strukturelement Q einen Tricyclo[5.2.1.0^{2,6}]decan-Rest, einen Tricyclo[5.2.1.0^{2,6}]dec-3-en-Rest, einen Tricyclo[3.3.1.1^{3,7}]decan-Rest oder einen Bicyclo[2.2.1]heptan-Rest bedeutet, handelt es sich vorzugsweise um solche mit einem Tricyclo[5.2.1.0^{2,6}]decan-Gerüst, einem Tricyclo[5.2.1.0^{2,6}]dec-3-en-Gerüst, einem Tricyclo[3.3.1.1^{3,7}]decan-Gerüst bzw. einem Bicyclo[2.2.1]heptan-Gerüst, bei dem jeweils keines der nicht durch Substituenten YZₑ substituierten Wasserstoffatome dieses polyalicyclischen Strukturelements Q substituiert ist.

Besonders bevorzugte erfindungsgemäße Verbindungen sind solche, wobei
A) eine Verbindung der Struktur QG_{b} ist, worin jedes G eine reaktive Gruppe bedeutet, die unabhängig von weiteren Gruppen G ausgewählt ist aus der Gruppe bestehend aus -NH₂, -CH₂NH₂, -OH, -CH₂OH, -NCO, -CH₂NCO, und -COOH.

Weiter bevorzugte Verbindungen sind solche, in denen mindestens ein Strukturelement YZₑ unabhängig von dem oder den weiteren Strukturelementen YZₑ ausgewählt ist, und vorzugsweise sämtliche Strukturelemente YZₑ ausgewählt sind, aus der Gruppe bestehend aus 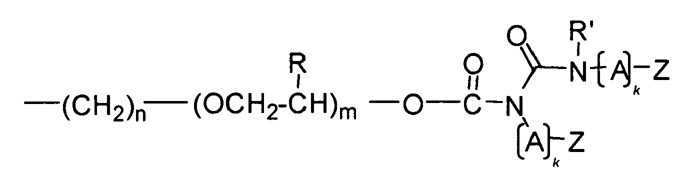 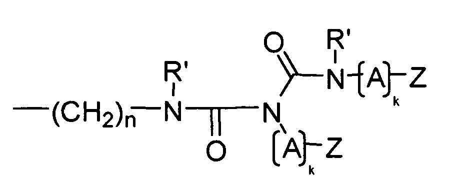 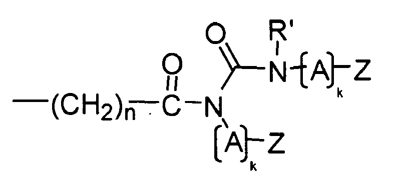 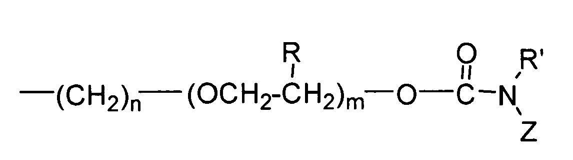 wobei Z, R, m sowie n die obige Bedeutung haben und worin ferner gilt:
- jedes A bedeutet ein zweibindiges organisches Brückenglied,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

Weiter bevorzugte Verbindungen sind solche, bei denen mindestens ein Strukturelement YZₑ unabhängig von dem oder den weiteren Strukturelementen YZₑ ausgewählt ist, und vorzugsweise sämtliche Strukturelemente YZₑ ausgewählt sind, aus der Gruppe bestehend aus 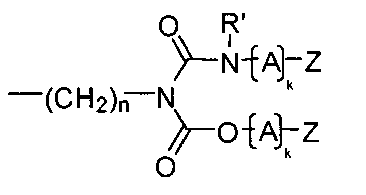 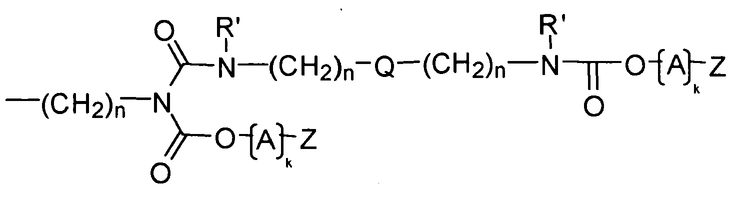 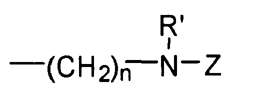 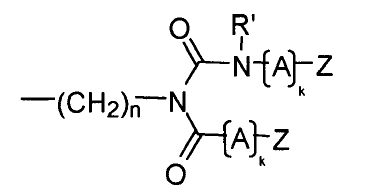 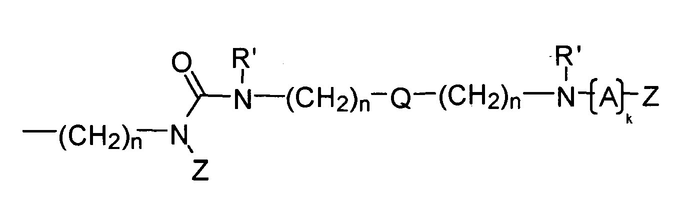 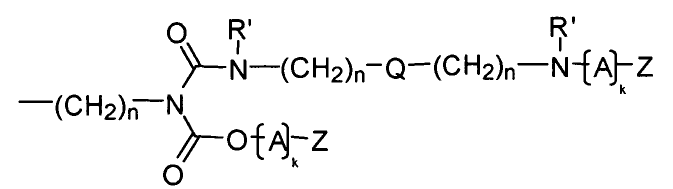 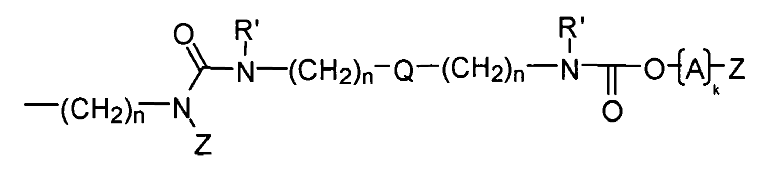 wobei jedes Q unabhängig von etwaigen weiteren Strukturelementen Q die obige Bedeutung hat und wobei Z, A, k und R' sowie n die oben genannte Bedeutung haben.

Dabei wiederum bevorzugt sind Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.

Dabei wiederum bevorzugt ist eine erfindungsgemäße Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Weiter bevorzugt sind Verbindungen, in denen jedes Strukturelement A unabhängig von etwaigen weiteren Strukturelementen A ausgewählt ist aus der Gruppe bestehend aus C₁-C₂₀-Alkylen, C₁-C₂₀-Heteroalkylen, C₃-C₂₀-Cycloalkylen, C₄-C₂₀-Cycloalkylalkylen, C₂-C₂₀-Alkenylen, C₃-C₂₀-Cycloalkenylen, C₄-C₂₀-Cycloalkenylalkylen, C₄-C₂₀-Cycloalkenylenalkylen, C₃-C₂₅-Arylen, C₂-C₂₅-Heteroarylen, C₄-C₂₅-Arylalkylen, C₄-C₂₅-Arylenalkylen, C₄-C₂₅-Arylheteroalkylen, C₄-C₂₅-Arylenheteroalkylen.

In bevorzugten Ausgestaltungen umfasst Strukturelement A eine oder mehrere der folgenden Atome oder Atomgruppen:
-O-, -O-Ar¹-CR⁶R⁷-Ar²-O-, -NR⁸-, -N-(C=O)-, -NH-(C=O)-O-, -NH-C(=O)-NH-
   wobei gilt:
Ar¹ und Ar² bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Phenylring,
R⁶, R⁷ und R⁸ bedeuten unabhängig voneinander Wasserstoff oder einen C1-C8-Rest, dabei vorzugsweise einen C1-C4-Alkylrest, dabei wiederum bevorzugt Methyl oder Ethyl. Zur Herstellung der erfindungsgemäßen Verbindungen können vorzugsweise Hydroxylverbindungen von (Meth)acrylaten eingesetzt werden, wobei auch Gemische von Acrylaten und Methacrylaten verwendet werden können. Bevorzugt sind zum Einsatz als Reaktionspartner gemäß Komponente B), C) oder D):
Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritoltri(meth)acrylat, wie Dipentaerythrit, wie beispielsweise Dipentaerythritolpenta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Zur Herstellung der erfindungsgemäßen Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandiisocyanat, Methylcyclohexandiisocyanat, Ethylcyclohexandiisocyanat, Propylcyclohexandiisocyanat, Methyldiethylcyclohexandiisocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-Diisocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat, Undekandi- und -triisocyanat, Dodecandi- und -triisocyanate, Isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacern, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondiisocyanat, Dicyclohexylmethan-4,4'-diisocyanat oder Hexamethylendiisocyanat genannt.

Ein erfindungsgemäß bevorzugtes Verfahren zur Herstellung einer Verbindung Q(YZₑ)_{b}, vorzugsweise in einer der oben genannten bevorzugten bzw. besonders bevorzugten Ausgestaltungen, oder einer Mischung umfassend zumindest eine Verbindung Q(YZₑ)_{b}, ist ein Verfahren mit folgenden Schritten:
In einer ersten Reaktion Umsetzen von
   A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₙ-OH, (-CH₂)ₙ-NCO und (-CH₂)ₙ-COOH
      mit
   B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH
      zu einem ersten Umsetzungsprodukt,
      in einer zweiten Reaktion Umsetzen des ersten Umsetzungsproduktes mit
   C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
      zu einem zweiten Umsetzungsprodukt
      und gegebenenfalls in einer dritten Reaktion Umsetzen des zweiten Umsetzungsproduktes mit
   D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
      wobei Q, b, Y, Z, e, M, R, m und n jeweils die oben genannte Bedeutung haben, und
      wobei das Verhältnis der Gesamtzahl von NCO-Gruppen zu der Gesamtzahl von -NH₂, - OH und -COOH in der Gesamtzahl von Verbindungen gemäß A) und B) in der ersten, der zweiten und gegebenenfalls der dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

In einem erfindungsgemäßen Verfahren zur Herstellung einer erfindungsgemäßen Verbindung Q(YZₑ)_{b} liegt das Verhältnis der Gesamtzahl von umgesetzten NCO-Gruppen zu der Gesamtzahl von umgesetzten -NH₂, -OH und -COOH in der Gesamtzahl von Verbindungen gemäß A) und B) in der ersten, (gegebenenfalls) zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.

Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.

Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.

Die Menge des Katalysators liegt dabei vorzugsweise im Bereich von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D).

Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevorzugt im Bereich von 60 bis 120°C. Die Umsetzung wird vorzugsweise bei Normaldruck (1013 mbar) ausgeführt.

Die vorstehenden bzw. nachfolgenden Ausführungen zu den als bevorzugt und besonders bevorzugt gekennzeichneten erfindungsgemäßen Verbindungen gelten für die bevorzugten und besonders bevorzugten Ausgestaltungen der erfindungsgemäßen Verfahren, Mischungen, Gemische, Erzeugnisse und Verwendungen jeweils entsprechend.

In einem weiteren Aspekt betrifft die vorliegende Erfindung eine Mischung umfassend ein, zwei oder mehr unterschiedliche erfindungsgemäße Verbindungen (vorzugsweise in einer der als bevorzugt bzw. besonders bevorzugt gekennzeichneten Ausgestaltungen), herstellbar nach einem erfindungsgemäßen Verfahren (vorzugsweise in einer der als bevorzugt bzw. besonders bevorzugt gekennzeichneten Ausgestaltungen).

In einem weiteren Aspekt betrifft die vorliegende Erfindung ein härtbares Gemisch, umfassend
(a) eine oder mehrere erfindungsgemäße Verbindungen (vorzugsweise in einer der als bevorzugt bzw. besonders bevorzugt gekennzeichneten Ausgestaltungen) oder eine erfindungsgemäße Mischung (vorzugsweise in einer der als bevorzugt bzw. besonders bevorzugt gekennzeichneten Ausgestaltungen),
   und
(b) einen oder mehrere weitere Bestandteile gewählt aus der Gruppe bestehend aus
   (b-1) von Bestandteil (a) verschiedene Monomere, welche mit Bestandteil (a) co-polymerisierbar sind, vorzugsweise lichtpolymerisierbare Monomere, bevorzugt gewählt aus der Gruppe bestehend aus Acrylaten und Methacrylaten,
   (b-2) einen oder mehrere Füllstoffe, vorzugsweise einen oder mehrere nanoskalige Füllstoffe,
   (b-3) Photoinitiatoren und Initiatoren für die chemische Aushärtung,
   (b-4) Polymerisationsinhibitoren,
      und
   (b-5) Lösungsmittel.

Bei einem bevorzugten erfindungsgemäßen Gemisch handelt es sich um eine chemisch und/oder durch Licht induziert und/oder thermisch induziert härtbare dentale Zusammensetzung.

### Bestandteil (b-1) - polymerisierbare Monomere

Die polymerisierbaren Monomere sind vorzugsweise radikalisch lichtpolymerisierbare Monomere, die bevorzugt eine, zwei oder mehr ethylenische Gruppen aufweisende Substanzen sind wie beispielsweise, ohne darauf beschränkt zu sein, die in der Dentalchemie üblicherweise eingesetzten (Meth)acrylatmonomere.

In der Patentliteratur ist eine Vielzahl weiterer Verbindungen genannt (beispielsweise auch in der DE 39 41 629 A1, welche im Wege der Verweisung Bestandteil der vorliegenden Anmeldung ist), die allesamt Diester der Acryl- oder Methacrylsäure sind und sich zum Einsatz in einem erfindungsgemäßen härtbaren Gemisch eignen.

In einem bevorzugten erfindungsgemäßen härtbaren Gemisch enthält Bestandteil (b-1) ein oder mehrere Dimethacrylat-Monomere gewählt aus der Gruppe bestehend aus Ethylenglykoldimethacrylat, 1,6-Hexandioldimethacrylat (HEDMA), Triethylenglykoldimethacrylat (TEDMA), 1,12-Dodecandioldimethacrylat, ethoxyliertes Bisphenol-A-dimethacrylat, Polyethylenglykoldimethacrylat, 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans.

Die radikalisch lichtpolymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Weiterhin können auch lichthärtbare Monomere mit ethylenischen Doppelbindungen auf Basis von Polysiloxanen, wie sie beispielsweise in der DE 199 03 177 oder in der DE 44 16 857 beschrieben sind, verwendet werden, welche im Wege der Verweisung Bestandteil der vorliegenden Anmeldung sind.

Ferner sind die sogenannten Ormocere, die den Fachleuten bekannt sind und die beispielsweise in der DE 199 03 177 oder in der DE 44 16 857 beschrieben sind, verwendbar, welche im Wege der Verweisung Bestandteil der vorliegenden Anmeldung sind.

### Bestandteil (b-2) - Füllstoffe

Als Bestandteil (b-2) können organische und/oder anorganische Füllstoffe eingesetzt werden.

Anorganische Füllstoffe können allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Die mittlere Partikelgröße d₅₀ der erfindungsgemäß einzusetzenden Füllstoffpartikel der Füllstoffkomponente (b-2) eines erfindungsgemäßen Gemisches wird mittels Lichtstreuung (Laserbeugung) bestimmt, vorzugsweise mit einem Partikelgrößenmessgerät Beckman Coulter LS 13320.

Als anorganische Füllstoffe kommen kompakte Gläser und unterschiedliche Kieselsäuren in verschiedenen Größen und Zuständen (monodispers, polydispers) zum Einsatz.

Geeignete anorganische Bestandteile sind beispielsweise amorphe Materialien auf der Basis von Mischoxiden aus SiO₂, ZrO₂ und/oder TiO₂, mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können erfindungsgemäße härtbare Gemische und Erzeugnisse unterschiedliche Kieselsäuren, bevorzugt pyrogene Kieselsäuren, enthalten.

Daneben können verstärkend wirkende Materialien, wie Glasfasern, Polyamid- oder Kohlenstofffasern eingesetzt werden. Die erfindungsgemäßen härtbaren Gemische und Erzeugnisse kann zusätzlich feinteilige Splitter oder Perlpolymerisate enthalten, wobei die Perlpolymerisate Homo- oder Copolymere organischer härtbarer Monomerer sein können.

Bevorzugt enthalten die erfindungsgemäßen härtbaren Gemische und Erzeugnisse, insbesondere zum Einsatz im Dentalbereich, nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Sulfiden, Seleniden und Telluriden von Metallen und deren Mischungen. Besonders bevorzugt sind nanoskalige Teilchen von SiO₂, TiO₂, ZrO₂, ZnO, SnO₂ und Al₂O₃ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc. Bevorzugt werden nanoskalige anorganische Feststoffteilchen, die organisch oberflächenmodifiziert sind. Die organische Oberflächenmodifizierung verbessert die Einbindung der nanoskaligen Partikel in die Polymermatrix.

In einer bevorzugten Ausgestaltung liegen die nanoskaligen Teilchen in nicht agglomerierter Form vor, beispielsweise dispergiert in einem Medium, vorzugsweise in monodisperser Form.

Um eine gute Einbindung der Nanopartikel in die Polymermatrix eines erfindungsgemäßen härtbaren Gemisches oder Erzeugnisses zu erreichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan, wodurch silanisierte Nanopartikel gebildet werden. Als Haftvermittler eignet sich besonders das Methacryloxypropyltrimethoxysilan.

In einer weiteren bevorzugten Ausgestaltung sind die nanoskaligen Teilchen nicht agglomerierte, organisch oberflächenmodifizierte Nanopartikel mit einer mittleren Partikelgröße kleiner 200 nm, vorzugsweise kleiner 100 nm, besonders bevorzugt kleiner 60 nm), die wiederum bevorzugt silanisiert sind.

### Bestandteil (b-3) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen härtbaren Gemisches bewirken können.

Das Absorptionsmaximum von Campherchinon (CC) liegt bei ca. 470 nm und somit im Bereich des blauen Lichts. Campherchinon (CC) zählt zu den PI₂-Initiatoren und wird regelmäßig zusammen mit einem Coinitiator eingesetzt.

Vorzugsweise enthält eine erfindungsgemäßes härtbares Gemsich die Kombination eines alpha-Diketons und eines aromatischen tertiären Amins, bevorzugt ist die Kombination von Campherchinon (CC) und Ethyl-p-N,N-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in erfindungsgemäßen härtbaren Gemischen wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Gemischen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der Verweisung Bestandteil der vorliegenden Anmeldung sind.

### Bestandteil (b-3) - Initiatoren für die chemische Aushärtung

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023, welche im Wege der Verweisung Bestandteil der vorliegenden Anmeldung sind.

Geeignete Malonylsulfamide sind in der EP 0 059 451 beschrieben welche im Wege der Verweisung Bestandteil der vorliegenden Anmeldung ist. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil (b-4) - Polymerisationsinhibitoren

Die erfindungsgemäßen härtbaren Gemische enthalten vorzugsweise einen oder mehrere Inhibitoren, auch Stabilisatoren genannt. Diese werden einem härtbaren Gemisch zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität des härtbaren, insbesondere lichthärtbaren, dentalen Gemisches. Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

### Bestandteil (b-5) - Lösungsmittel

Geeignet sind die in der Lackindustrie üblicherweise verwendeten Lösungsmittel, wie beispielsweise Kohlenwasserstoffe, Ketone und Ester wie beispielsweise Toluol, Xylol, Isooctan, Aceton, Butanon, Methylisobutylketon, Ethylacetat, Butylacetat, Tetrahydrofuran, N-Methylpyrrolidon, Dimethylacetamid und Dimethylformamid. Man kann auch Alkohole wie Ethanol, Propanole, Butanole, Pentanole, Hexanole, Cyclohexanol, Heptanole, Octanole, Nonanole, Decanole, etc. einsetzen. Ebenfalls geeignet sind cycloaliphatische oder arylaliphatische Alkohole.

Im Folgenden wird die Erfindung zunächst für Monomere umfassend tricyclische Strukturelemente Q am Beispiel von Tricyclo[5.2.1.0^{2,6}]decan (TCD) - Derivaten im Detail erläutert.

### 1.) .) Ausgehend vom Bis(hydroxymethyl)tricyclo[5.2.1.0^{2,6}]decan (TCD-Diol)

Bis(hydroxymethyl)tricyclo[5.2.1.0^{2,6}]decan ist kommerziell erhältlich, beispielsweise als Dicidolgemisch der isomeren Verbindungen 3,8-Bis(hydroxymethyl)tricyclo[5.2.1.0^{2,6}]decan und 4,8-Bis(hydroxymethyl)tricyclo[5.2.1.0^{2,6}]decan sowie 3,9-Bis(hydroxymethyl)tricyclo[5.2.1.0^{2,6}]decan und 4,9-Bis(hydroxymethyl)tricyclo[5.2.1.0^{2,6}]decan.

Die Bis(hydroxymethyl)tricyclo[5.2.1.0^{2,6}]decane können, ausgehend von Dicyclopentadien (Tricyclo[5.2.1.0^{2,6}]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0^{2,6}]decan. Je nach Synthesepfad können gezielt an unterschiedlichen Positionen substituierte Bis(hydroxymethyl)tricyclo[5.2.1.0^{2,6}]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0^{2,6}]decan herstellbar ist. Die DE 103 52 260 B3 beschreibt dagegen Verfahren zur Herstellung von 3(4), 8(9)-Bis(hydroxymethyl)tricyclo[5.2.1.0^{2,6}]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäßer Monomere einsetzbare Bis(hydroxymethyl)tricyclo[5.2.1.0^{2,6}]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.0^{2,6}]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0^{2,6}]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.0^{2,6}]decan weist in einer Formulierung nach Aushärtung einen niedrigen Reaktionsschrumpf und eine hohe mechanische Festigkeit auf.

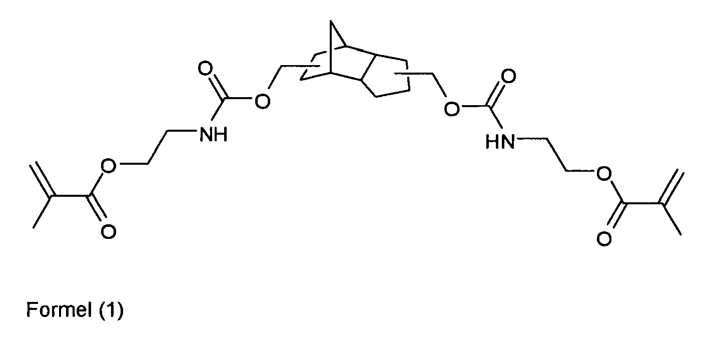

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer erfindungsgemäßen Verbindung. Es bildet sich hierbei zunächst das Allophanat der Formel (2) als tetrafunktionalisierte, radikalisch vernetzbare Verbindung. Auch dieses Monomer weist wiederum noch umsetzungsfähige Wasserstoffatome am Stickstoff auf, die erfindungsgemäß bei Reaktion mit weiterem Isocyanat das hexafunktionalisierte, radikalisch härtbare Allophanat der Formel (3) bilden.

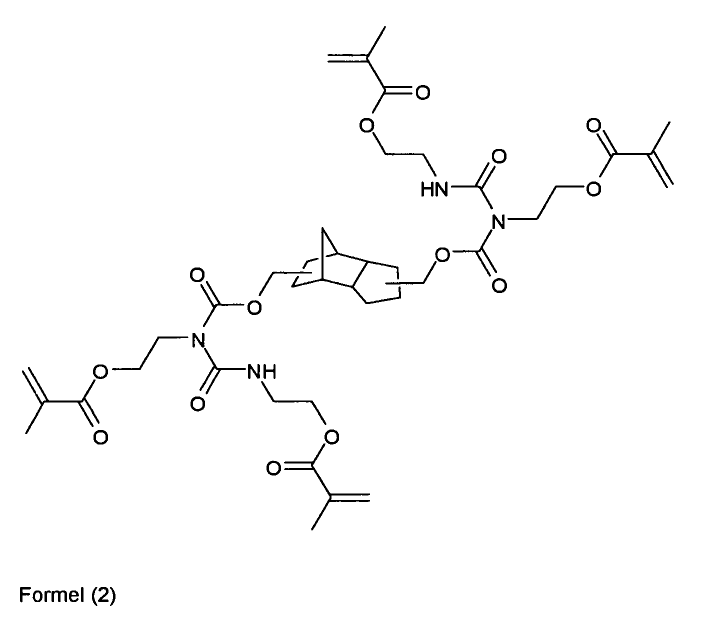

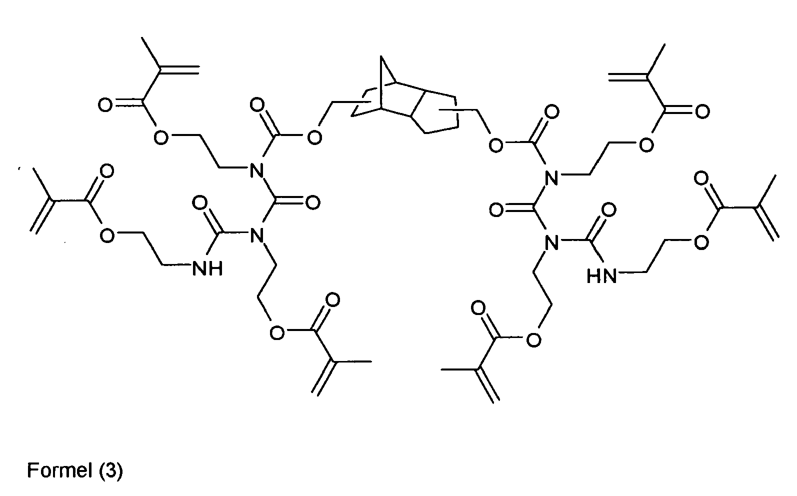

Alternativ kann das 3(4), 8(9)-Bis(hydroxymethyl)tricyclo[5.2.1.0^{2,6}]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Bei der Reaktion wird zunächst das 2-Isopropenyloxazolin-4,5-dionhydrochlorid gebildet, welches dann unter Zersetzung zum Methacryloylisocyanat führt. Experimentelle Angaben hierzu sind der EP 0 202 840 entnehmbar. Durch Umsetzung von 3(4), 8(9)-Bis(hydroxymethyl)tricyclo[5.2.1.0^{2,6}]decan auch mit Methacryloylisocyanat wird eine Verbindung der Formel (4) erhalten:

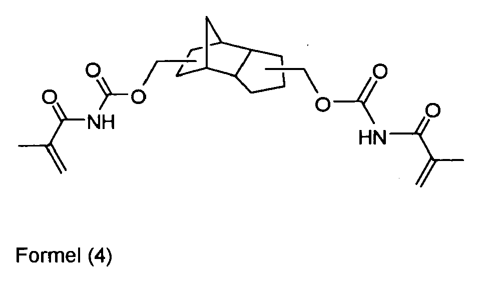

Die verbleibenden reaktiven Wasserstoffatome am Stickstoff der Verbindung der Formel (4) können anschließend wiederum in Isocyanatreaktionen zu den Allophanaten umgesetzt werden. Beispielhaft seien hier die Reaktionsprodukte mit 2-Isocyanatoethylmethacrylat (Formel (5)) und Methacryloylisocyanat (Formel (6)) gezeigt.

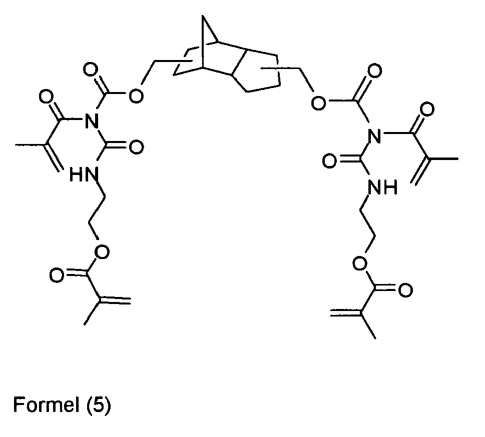

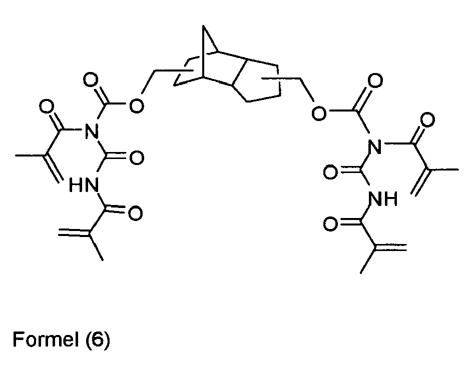

Wird 1,6-Hexandiisocyanat mit einem Äquivalent 2-Hydroxyethylmethacrylat umgesetzt, erhält man ein Urethan.

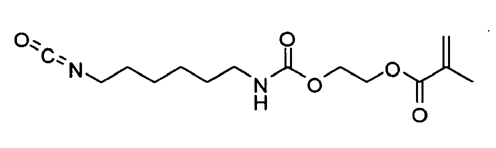

Dieses kann aufgrund der verbleibenden Isocyanatfunktionalität mit 3(4), 8(9)-Bis(hydroxymethyl)tricyclo[5.2.1.0^{2,6}]decan zu dem nachfolgend dargestellten Allophanat (Formel (7)) umgesetzt werden.

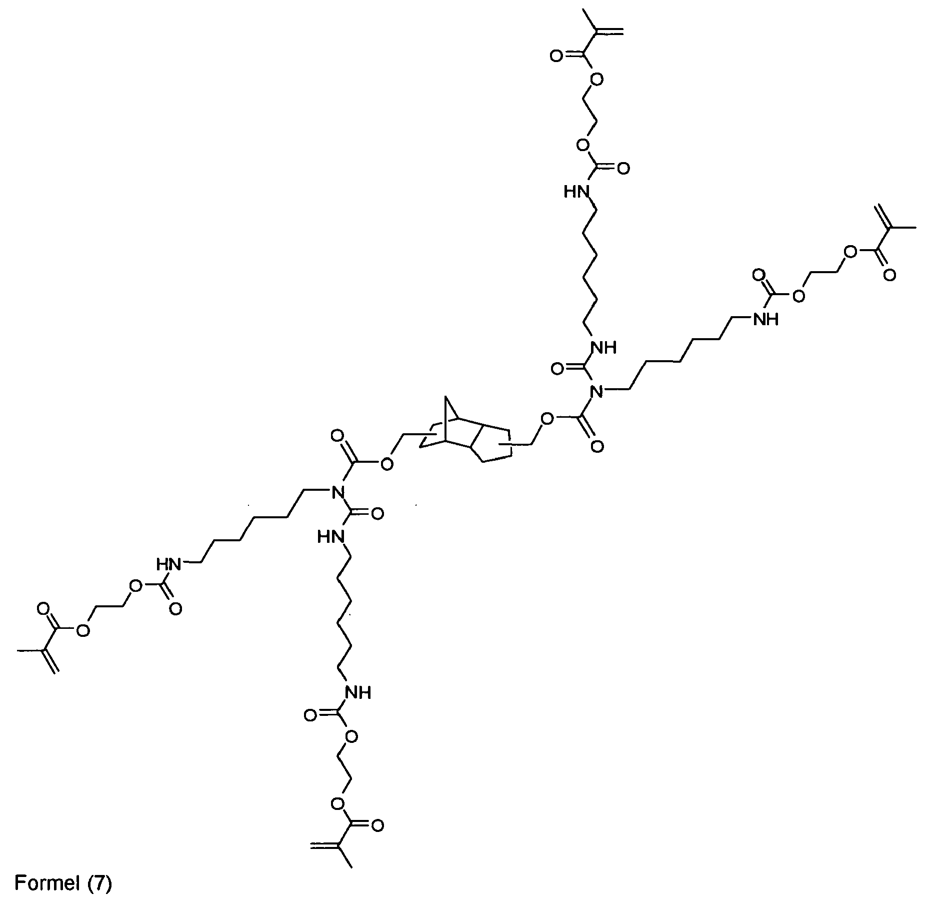

Anstelle von 1,6-Hexandiisocyanat könnte auch Methandiphenyldiisocyanat oder Isophorondiisocyanat eingesetzt werden

Die noch reaktiven Wasserstoffatome am Stickstoff können in weiteren Reaktionen mit Isocyanatgruppen umgesetzt werden.

### 2.) Ausgehend von 3(4), 8(9)-Bis(Carbonsäure)tricyclo[5.2.1.0^{2,6}]decan

Das 3(4), 8(9)-Bis(Carbonsäure)tricyclo[5.2.1.0^{2,6}]decan ist durch einfache Oxidation des kommerziell erhältlichen 3(4), 8(9)-Bis(formyl)tricyclo[5.2.1.0^{2,6}]decans herstellbar. Umsetzung der Dicarbonsäure mit 2-Isocyanatoethylmethacrylat ergibt das Amid der Formel (8):

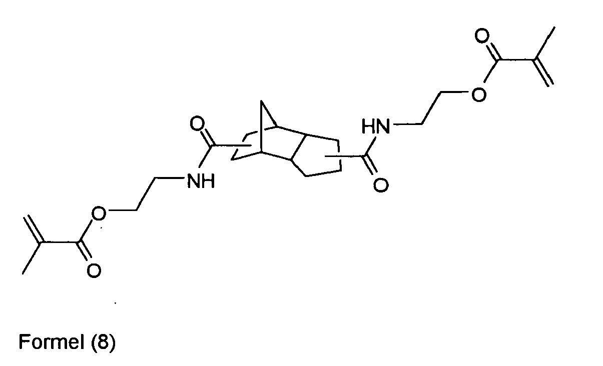

Weitere Umsetzung der beiden reaktionsfähigen Amid-Wasserstoffatome des Amids der Formel (8) mit 2-Isocyanatoethylmethacrylat ergibt den Acylharnstoff der Formel (9).

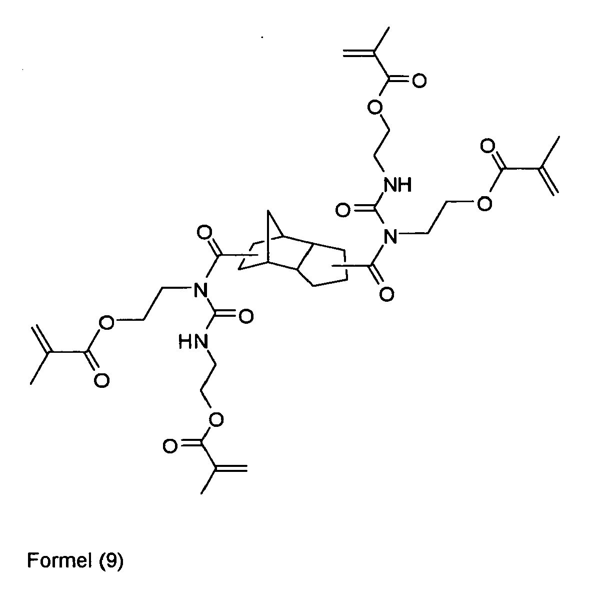

Wird 3(4), 8(9)-Bis(Carbonsäure)tricyclo[5.2.1.0^{2,6}]decan mit Methacryloylisocyanat umgesetzt, ergibt sich das Imid der Formel (10). Die reaktiven Wasserstoffatome am Stickstoff können auch hierweiter in Isocyanatreaktionen umgesetzt werden.

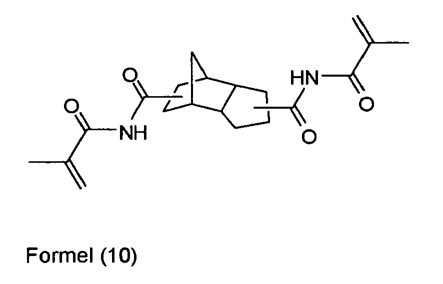

### 3.) Ausgehend von 3(4), 8(9)-Bis(isocyanatomethyl)tricyclo[5.2.1.0^{2,6}]decan

Das 3(4), 8(9)-Bis(isocyanatomethyl)tricyclo[5.2.1.0^{2,6}]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die erfindungsgemäße Durchführung der zweiten Umsetzungsstufe der Isocyanat-Alkoholreaktion kann nicht nur ausgehend vom Tricyclodecandiol mit dem Isocyanatoethylmethacrylat, sondern auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

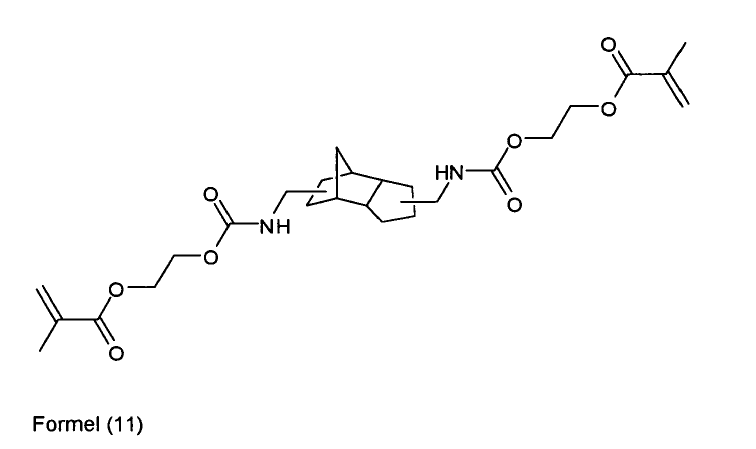

Auch dieses Carbamat (Formel (11)) weist zwei reaktionsfähige Wasserstoffatome am Stickstoff auf, die mit einem Überschuss an Bis(isocyanatomethyl)tricyclo[5.2.1.0^{2,6}]decan zu dem Diisocyanat der Formel (12) weiter umgesetzt werden kann.

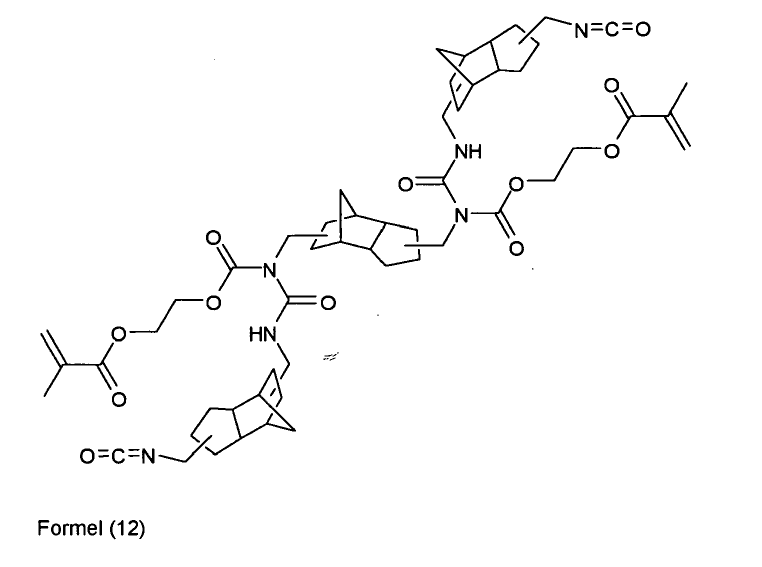

Umsetzung des Allophanat-Diisocyanats (Formel (12)) mit Methacrylsäure liefert die Verbindung der Formel (13).

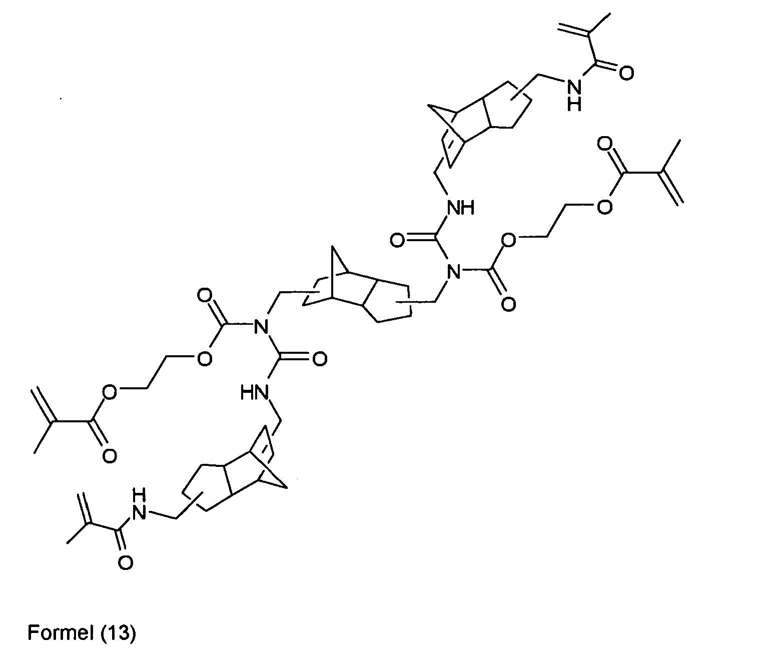

Alternativ kann das Urethan der Formel (11) in der zweiten Umsetzungsstufe mit 2-Isocyanatoethylmethacrylat auch zum Allophanat der Formel (14) umgesetzt werden.

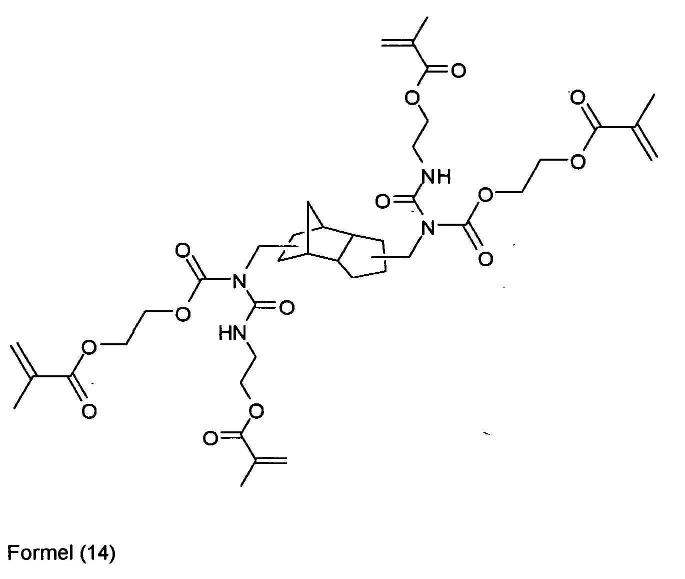

Die Diisocyanatgruppen der Verbindung der Formel (12) können weiter mit stöchiometrischen Mengen beispielsweise an Hydroxyethylmethacrylat zu der Verbindung der Formel (15) umgesetzt werden.

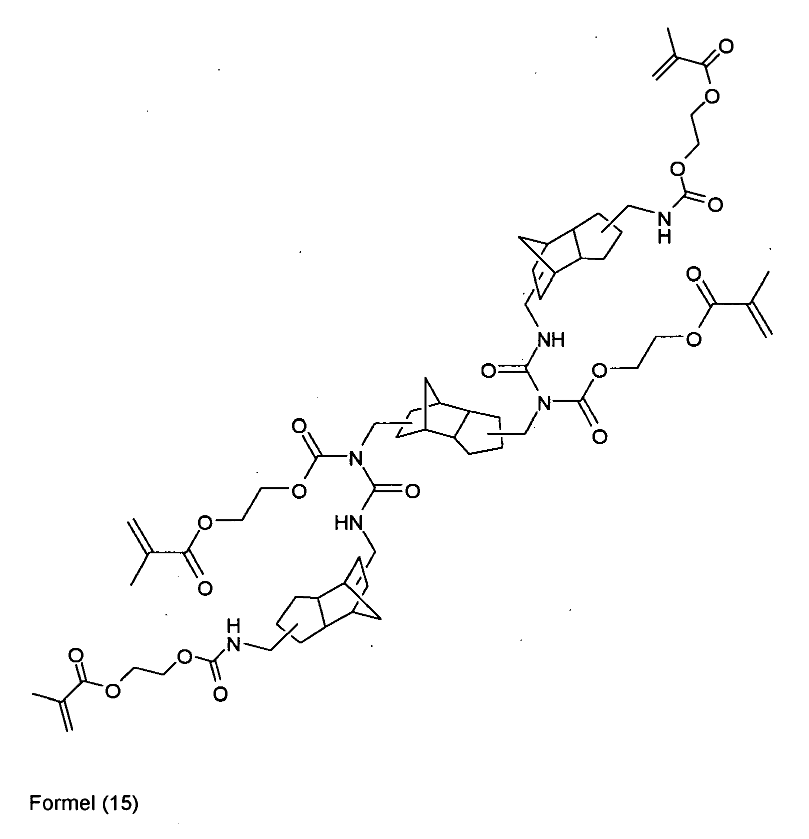

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.0^{2,6}]decan umgesetzt werden. Dabei bevorzugte Hydroxylverbindungen von (Meth)acrylaten sind

Alkylenoxidmono(meth)acrylate wie beispielsweise Ethylenglykolmono(meth)acrylat, Diethylenglykolmono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritoltri(meth)acrylat, wie Dipentaerythrit, wie beispielsweise Dipentaerythritolpenta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan, etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Alle diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.0^{2,6}]decan beschriebenen Weise reagieren. So lässt sich in einem einzigen Reaktionsschritt ein hoher Funktionalisierungsgrad erreichen.

3(4), 8(9)-Bis(isocyanatomethyl)tricyclo[5.2.1.0^{2,6}]decan kann mit 2-Carbonsäureethylmethacrylat zum entsprechenden Amid der Formel (16) umgesetzt werden.

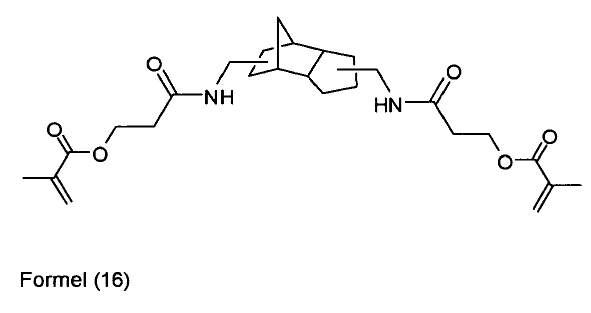

Umsetzung des Amids der Formel (16) mit 2-Isocyanatoethylmethacrylat liefert den Acylharnstoff der Formel (17).

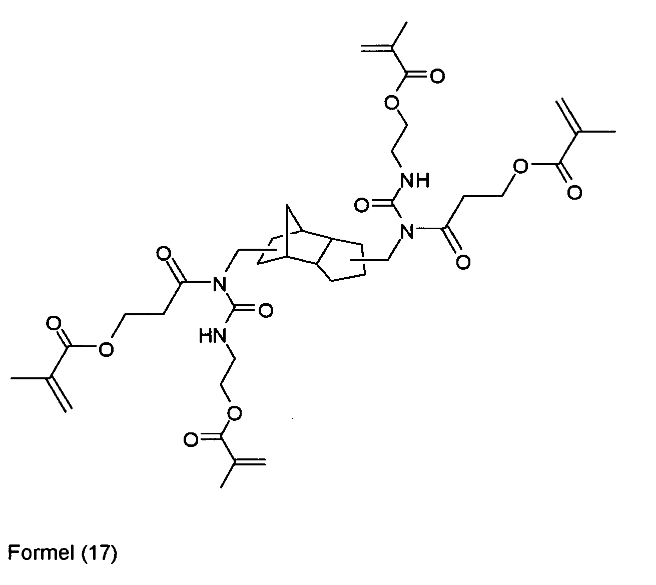

Das Amid der Formel (16) kann auch mit einem Überschuss an 3(4), 8(9)-Bis(isocyanatomethyl)tricyclo[5.2.1.0^{2,6}]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

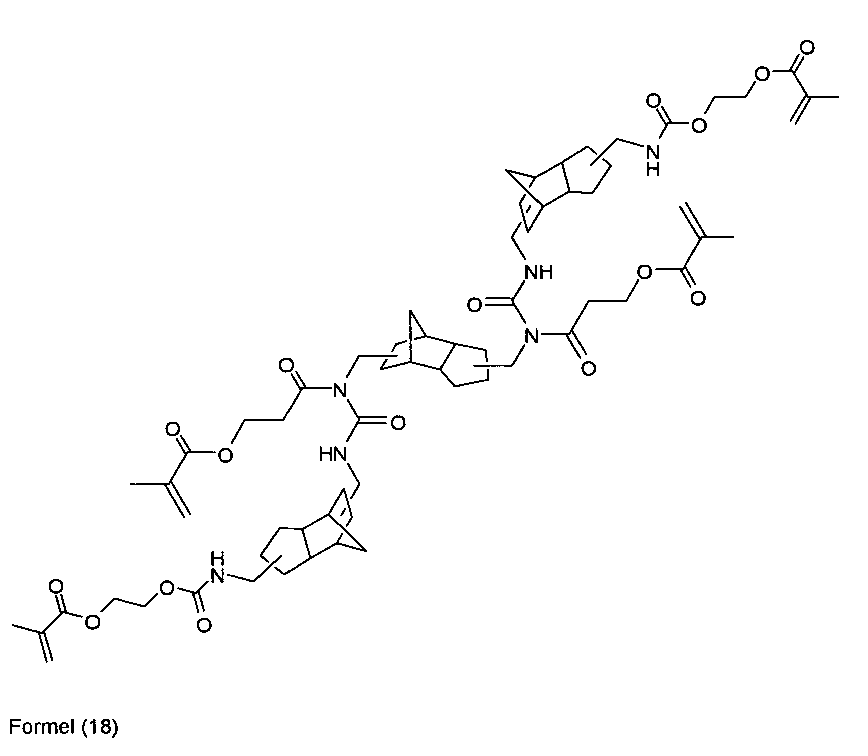

Wird 3(4), 8(9)-Bis(isocyanatomethyl)tricyclo[5.2.1.0^{2,6}]decan mit 2-Methacryloyloxyethylhydrogensuccinat umgesetzt, so erhält man das Amid der Formel (19), das weiter mit 2-Isocyanatoethylmethacrylat zum Acylharnstoff der Formel (20) abreagiert wird.

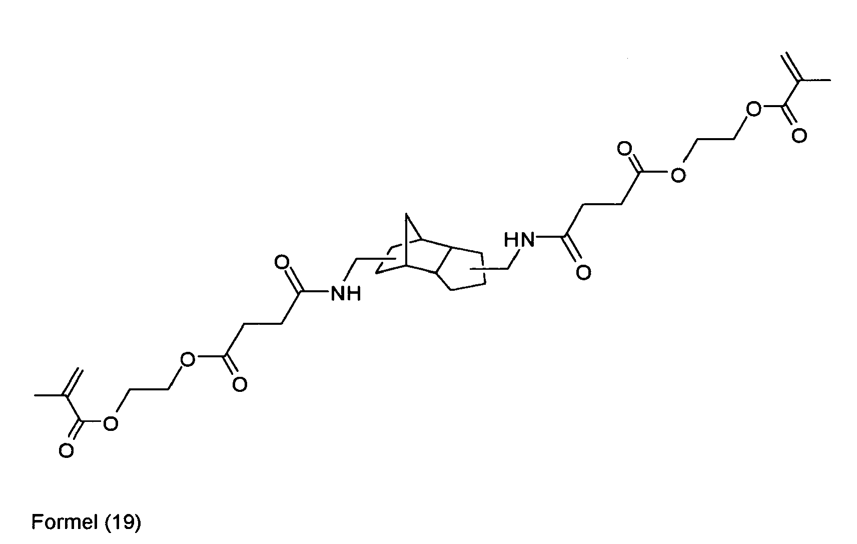

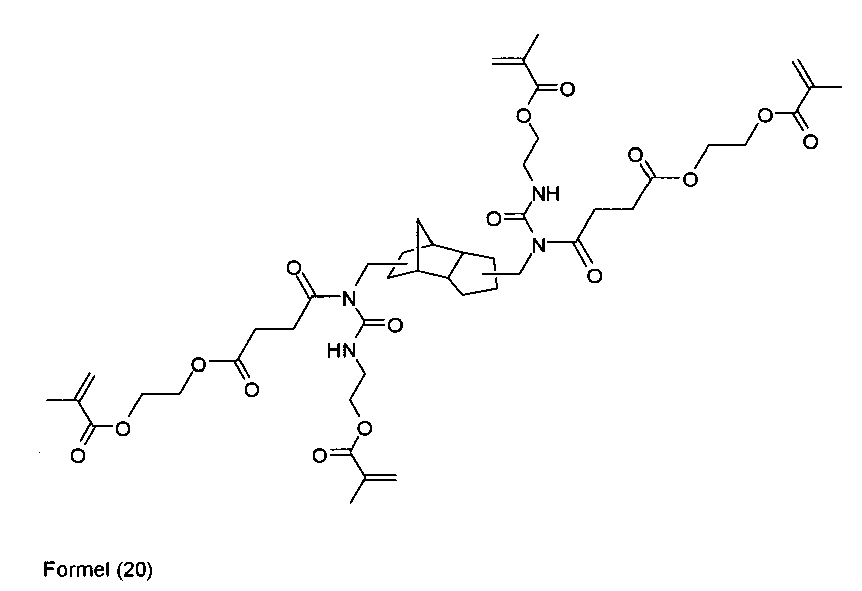

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

Aus der Umsetzung des 3(4), 8(9)-Bis(isocyanatomethyl)tricyclo[5.2.1.0^{2,6}]decan mit Methacrylsäure ergibt sich nach anschließender Decarboxylierung das Amid der Formel (21).

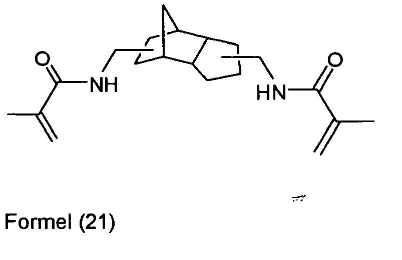

Aus dem Amid der Formel (21) ergibt sich durch weitere Umsetzung der NH-Funktionalität mit 2-Isocyanatoethylmethacrylat das tetrafunktionalisierte Monomer der Formel (22), welches ein Vernetzer ist.

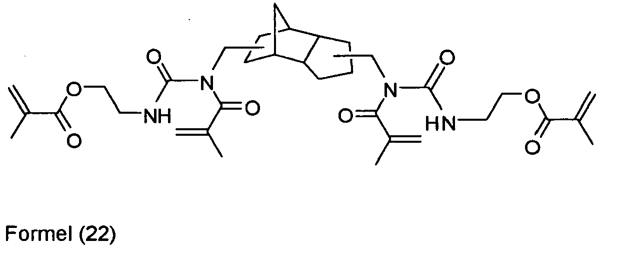

Wird das Amid der Formel (21) mit weiterem 3(4), 8(9)-Bis(isocyanatomethyl)tricyclo[5.2.1.0^{2,6}]decan umgesetzt, ergibt sich das Diisocyanat der Formel (23).

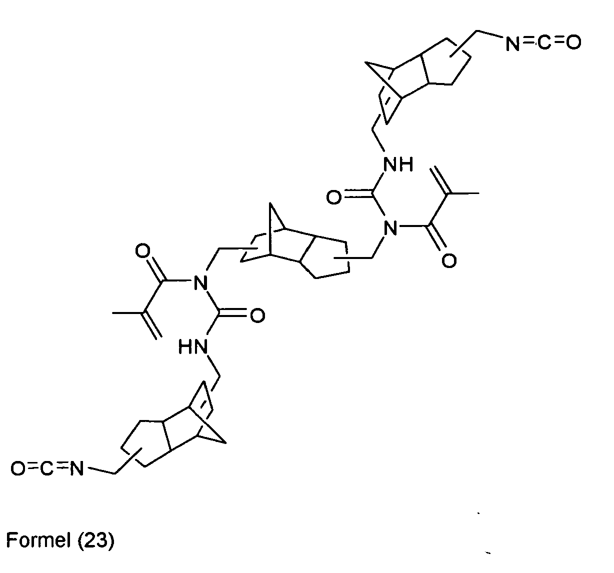

Das Diisocyanat der Formel (23) kann mit zwei weiteren Äquivalenten Methacrylsäure zu der Verbindung der Formel (24) umgesetzt werden.

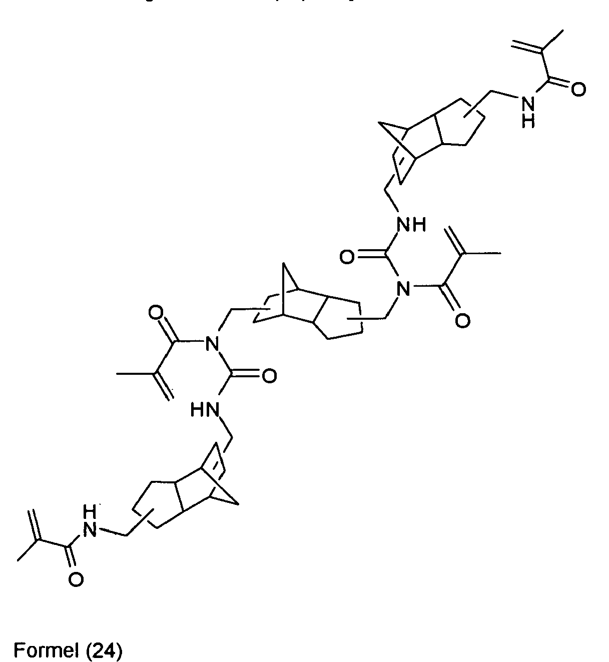

Die Umsetzung des Diisocyanats der Formel (23) mit 2-Hydroxyethylmethacrylat ergibt die Verbindung der Formel (25).

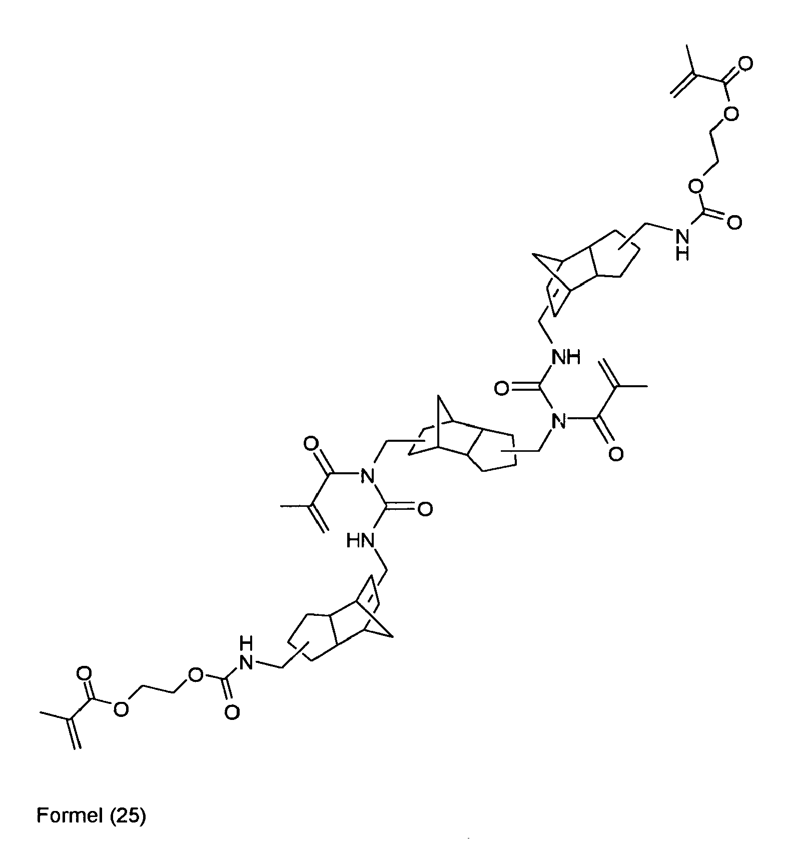

### 4.) Ausgehend von 3(4), 8(9)-Bis(Aminomethyl)tricyclo[5.2.1.0^{2,6}]decan

Das 3(4), 8(9)-Bis(Aminomethyl)tricyclo[5.2.1.0^{2,6}]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.0^{2,6}]decans mit 2-Isocyanatoethylmethacrylat ergibt die aus der EP 0 209 700 A2 bekannte Harnstoffverbindung der Formel (26).

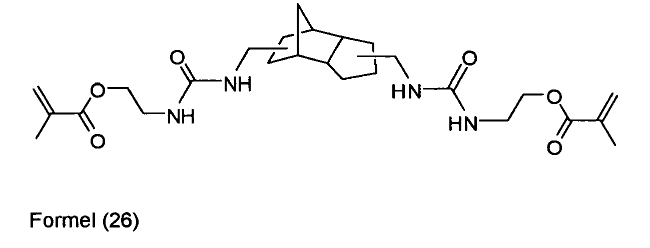

Auch hier befinden sich noch aktive, reaktionsfähige Wasserstoffatome am Stickstoff, die mit einem Überschuss an Isocyanat beispielsweise zum Biuret der Formel (27) reagieren.

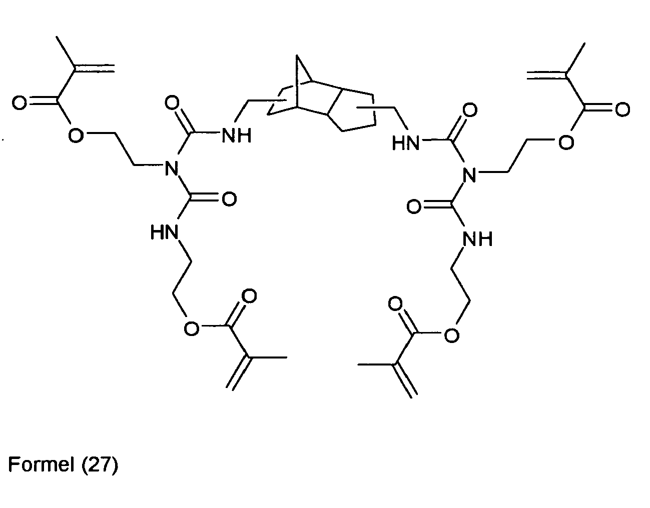

Das 3(4), 8(9)-Bis(Aminomethyl)tricyclo[5.2.1.0^{2,6}]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

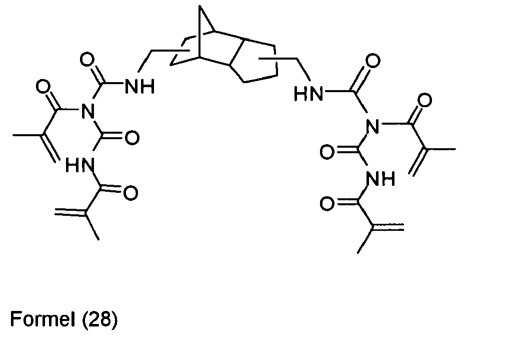

Analog zu den vorstehend beschriebenen Monomeren, welche ein vom Tricyclo[5.2.1.0^{2,6}]decan abgeleitetes polyalicyclisches Strukturelement Q umfassen, können auch Monomere hergestellt werden, welche ein vom Tricyclo[3.3.1.1^{3,7}]decan (Adamantan) abgeleitetes polyalicyclisches Strukturelement Q umfassen. Als Beispiele seien folgende Reaktionsprodukte gezeigt:

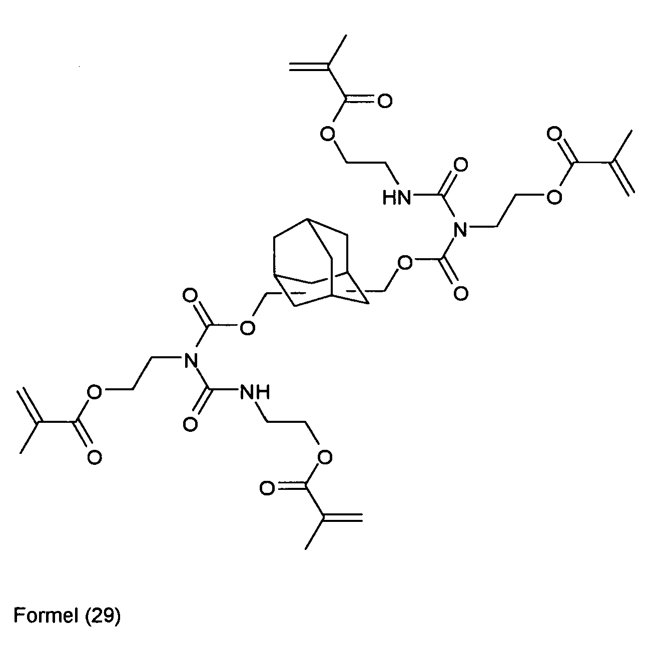

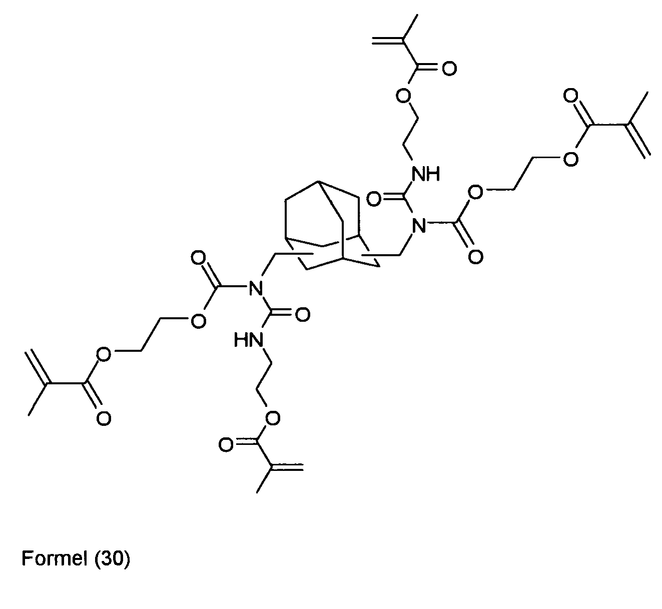

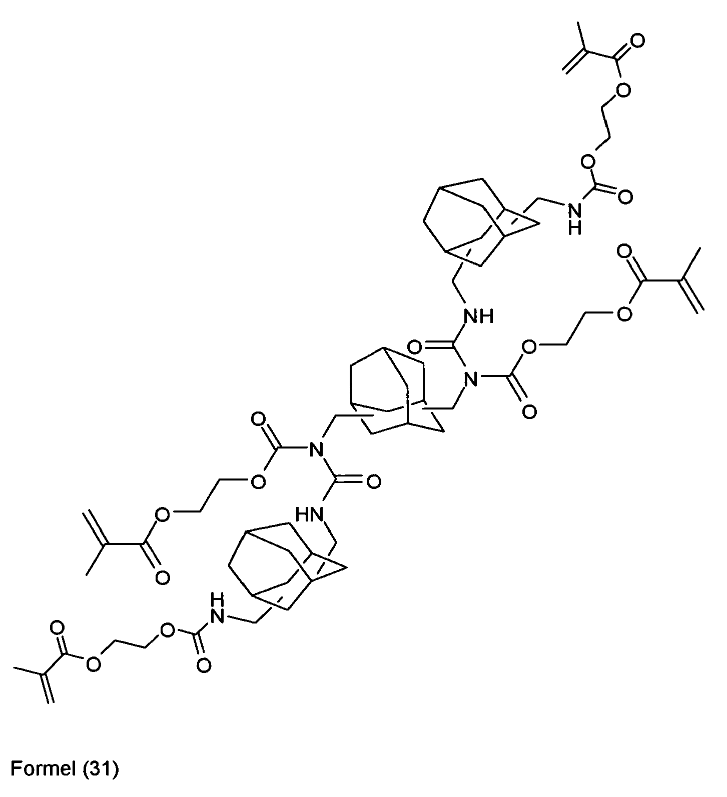

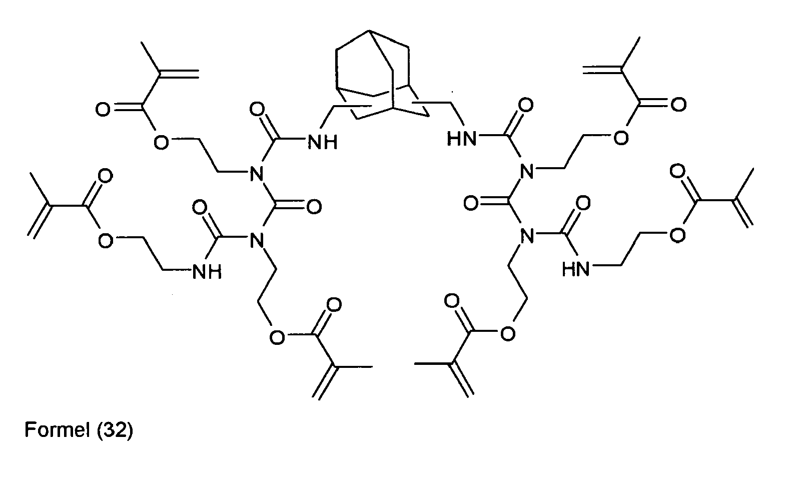

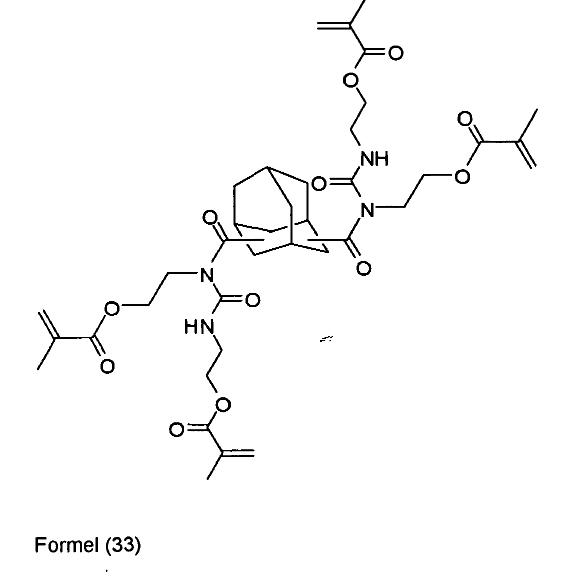

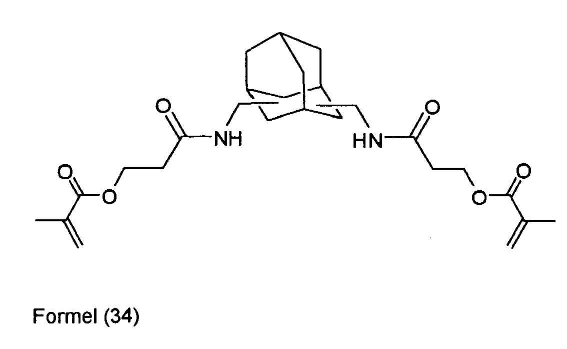

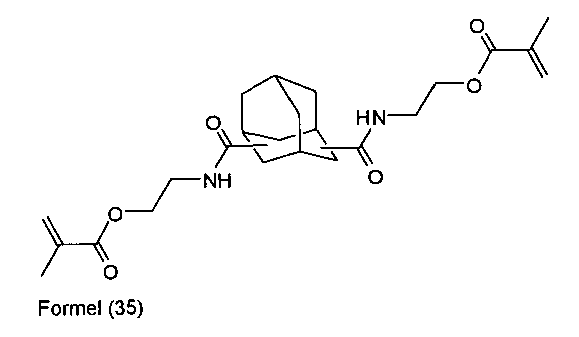

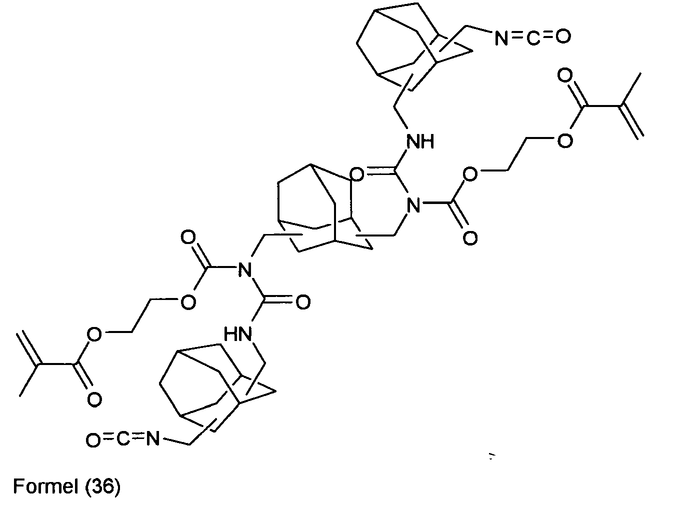

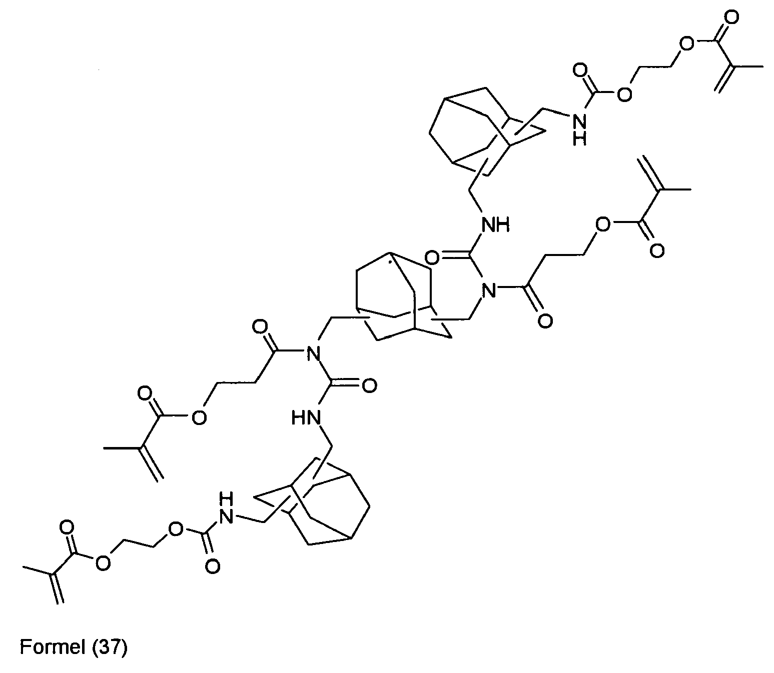

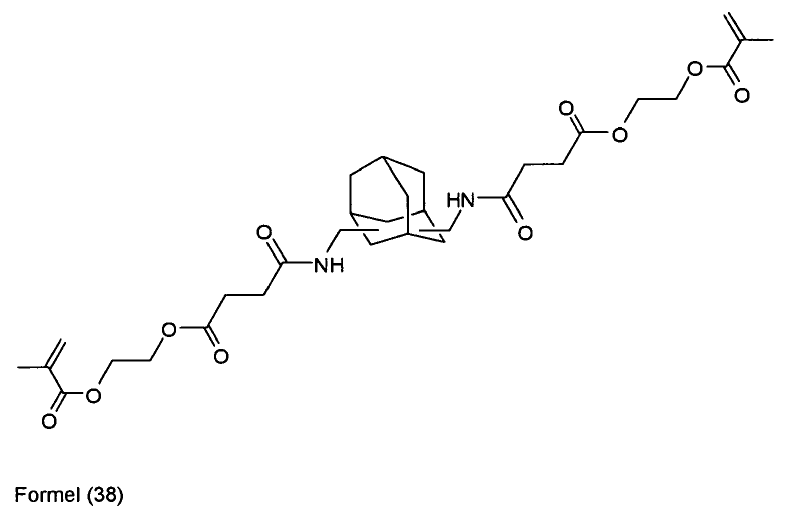

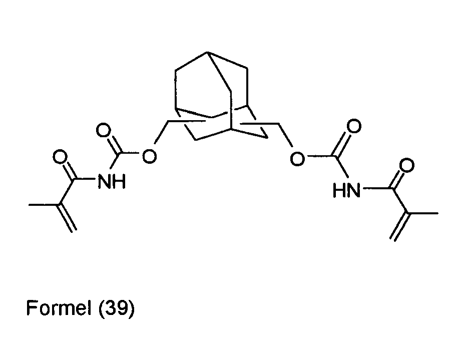

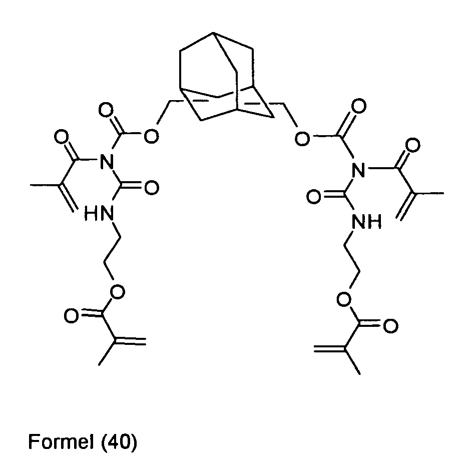

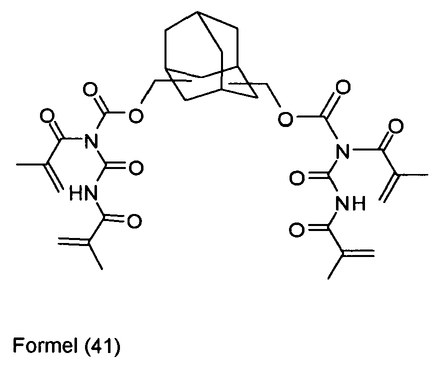

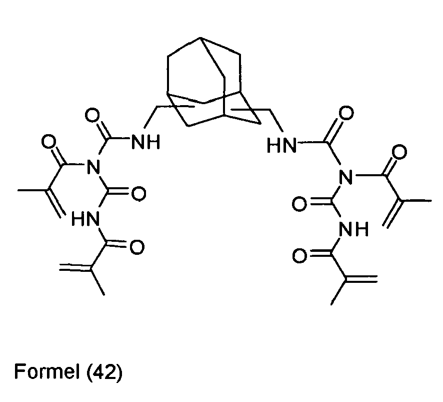

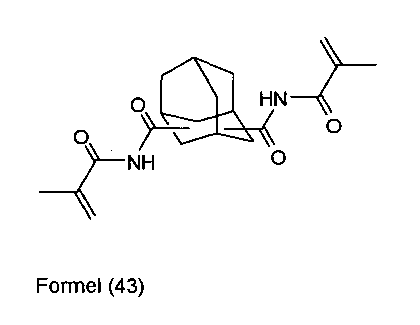

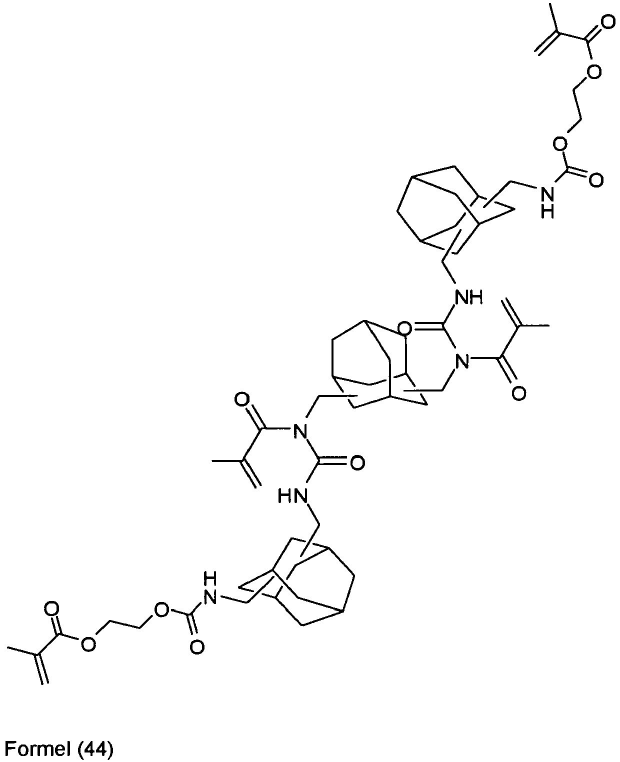

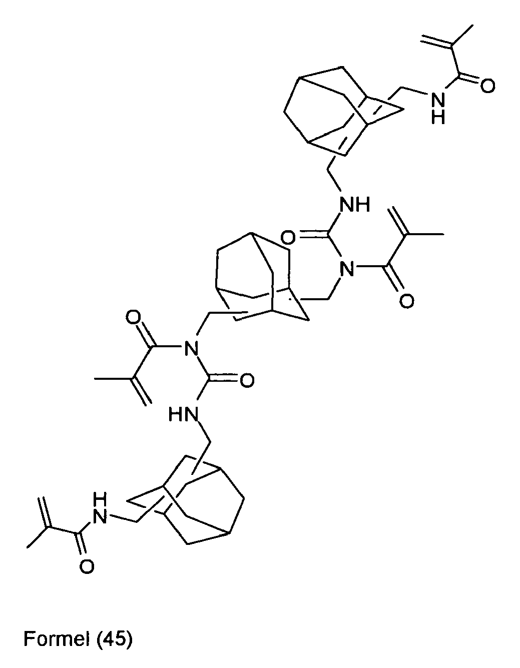

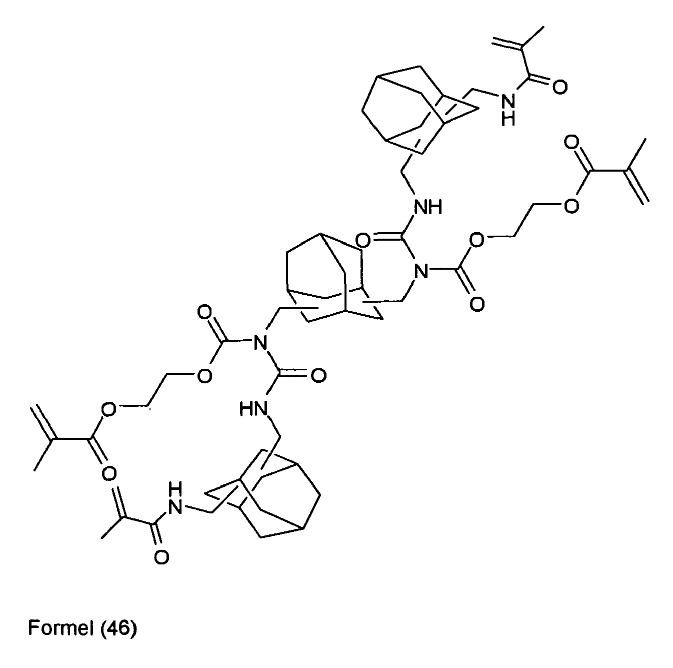

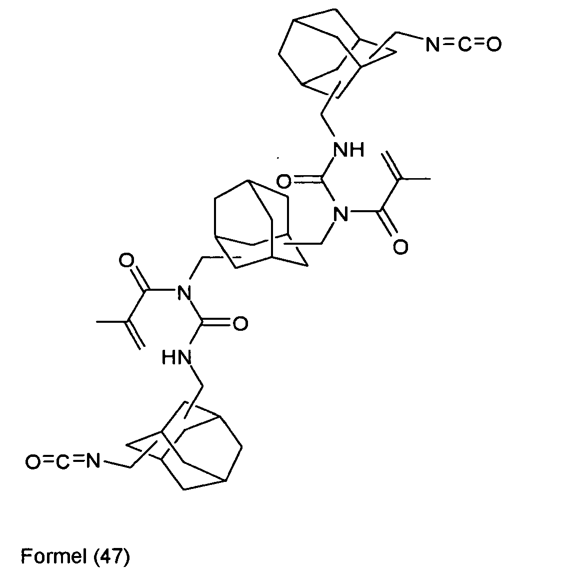

Im Folgenden wird die Erfindung für Monomere umfassend bicyclische Strukturelemente Q im Detail erläutert:
Eine geeignete Ausgangsverbindung ist 2,5 (2,6) - Bis(hydroxymethyl)bicyclo(2.2.1)heptan. 2,5 (2,6) - Bis(hydroxymethyl)bicyclo(2.2.1)heptan ist an sich bekannt und kommerziell oder aus Norbornadien (herstellbar aus Cyclopentadien und Ethin) durch Hydroformylierung und anschließende Reduktion des Diformylnorbornans zum Norbornandiol erhältlich. Die Hydroformylierung des Norbornadiens kann sowohl klassisch als auch nicht-klassisch erfolgen.

Im klassischen Verfahren wird Norbornadien in einem organischen Lösungsmittel, beispielsweise Toluol, in einem Autoklaven unter Druck (100 atm) und hohen Temperaturen (100°C) in Gegenwart eines Katalysators mit dem Synthesegas CO/H₂ (1:1) umgesetzt. Nach einer Reaktionszeit von 90 Minuten kann kein Substrat mehr nachgewiesen werden und die Umsetzung ist mit Bildung der Dialdehyde mit hoher Selektivität abgeschlossen. Als Hauptkomponenten werden die zwei Isomere des exo-exo-Dialdehyds gebildet. Als Katalysatoren werden [Pt(C₂H₄)(dppb)]/CH₃SO₃H eingesetzt. Die Abkürzung "dppb" bedeutet 1,4-Bis(diphenylphosphino)butan. Eine genaue Herstellvorschrift ist im Journal of Organometallic Chemistry, 447, 153-157, 1993 in einer Arbeit von C. Botteghi, S. Paganelli, A. Perosa, R. Lazzaroni, G. Uccello-Baretta mit dem Titel "Hydroformylation of norbornene and 2,5-norbornadiene catalysed by platinum-(0)-alkene complexes in the presence of methanesulfonic acid: determination of the stereochemnistry of the reaction" zu finden. Als Katalysatormetalle können auch Cobalt oder Rhodium in Form ihrer Hydridocarbonylspezies (HM(CO)₄), wie beispielsweise auf Basis von Hydridocobalttetracarbonyl (HCo(CO)₄) eingesetzt werden.

Im nicht-klassischen Verfahren, d.h. in überkritischem Kohlendioxid, reagieren Wasserstoff und Kohlenmonoxid bei der katalytischen Umwandlung der Olefine zu den Aldehyden unter weit weniger drastischen Bedingungen im Vergleich zum klassischen Verfahren. Bei nur 20 bar und 100°C in Gegenwart von Rh/4-H²F⁶-TPP verläuft die Reaktion innerhalb von 30 Minuten fast quantitativ mit einem Anteil von 95% an Dialdehyden. Bei der Rhodium katalysierten Hydroformylierung in überkritischem Kohlendioxid wird zur Erhöhung der Löslichkeit des Rh-Katalysators in CO₂ der Triphenylphosphan Ligand mit Perfluoralkylgruppen derivatisiert, wobei der elektronische Einfluss auf das Metallzentrum mittels zweier CH₂-Gruppen, sogenannter spacer, auf ein Minimum reduziert ist. Das Akronym 4-H²F⁶-TPP bedeutet somit, dass in Position 4, also in para-Stellung des aromatischen Rings (vom P-Atom aus gesehen) zunächst 2 CH₂-Gruppen anschließen, gefolgt von 6 CF₂-Gruppen. Genaue Herstellvorschriften befinden sich in der Dissertation von H. Stemmer, 2001, Friedrich-Schiller-Universität Jena, mit dem Titel "Homogene Katayse in überkritischem Kohlendioxid: Analogien und Unterschiede zu konventionellen Lösungsmitteln".

Eine weitere geeignete Ausgangsverbindung ist 2,5 (2,6)-Bis(isocyanatomethyl)bicyclo[2.2.1]heptan) entsprechend der folgenden Strukturformeln:

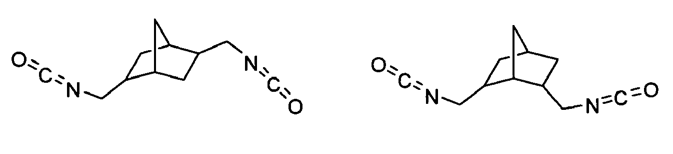

Nachfolgend werden beispielhaft einige Monomere umfassend ein von Bicyclo[2.2.1]heptan abgeleitetes bicyclisches Strukturelement Q dargestellt.

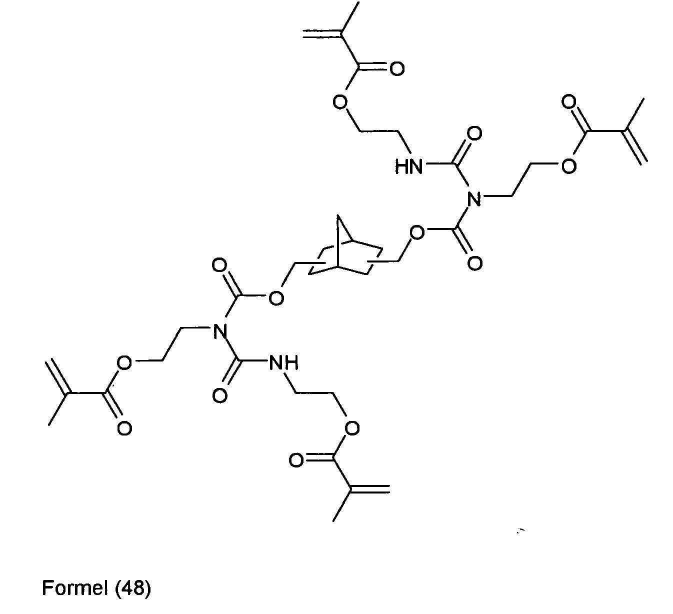

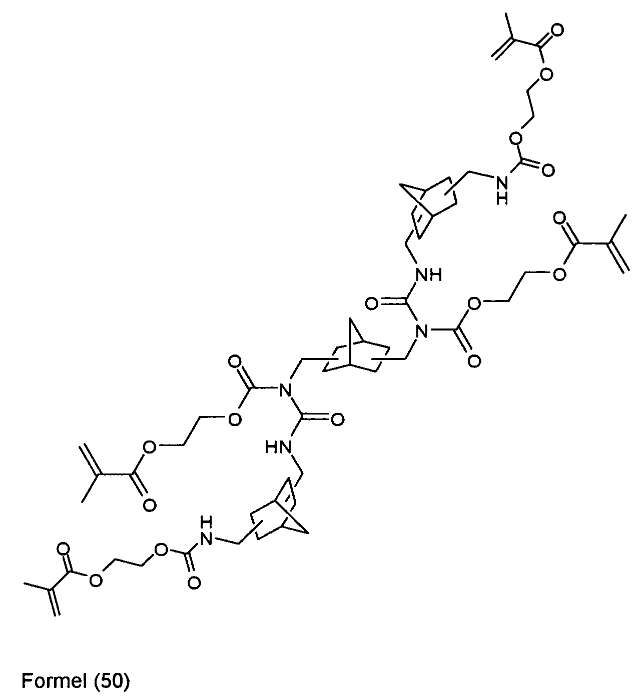

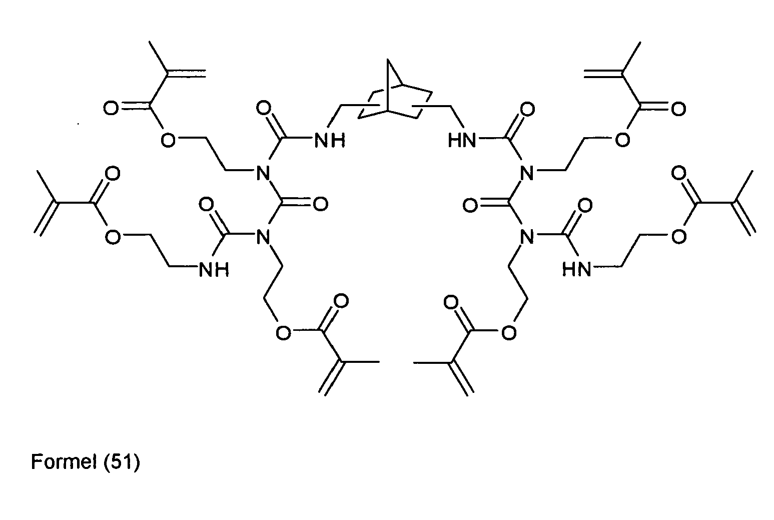

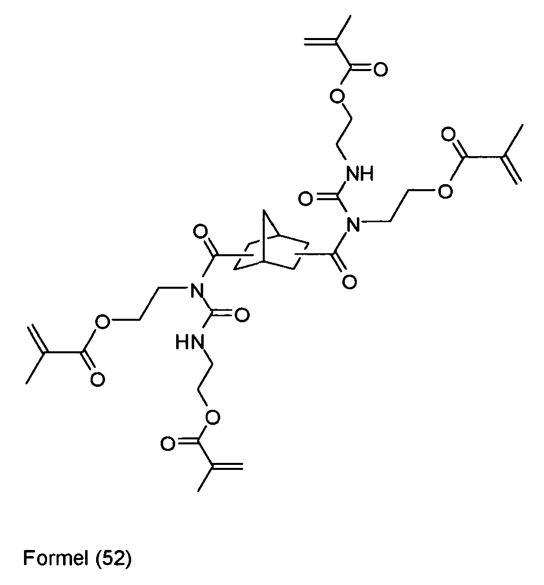

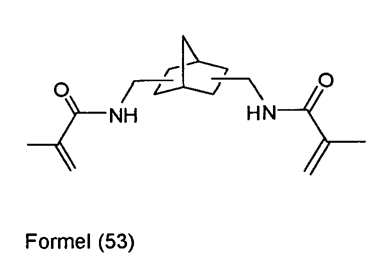

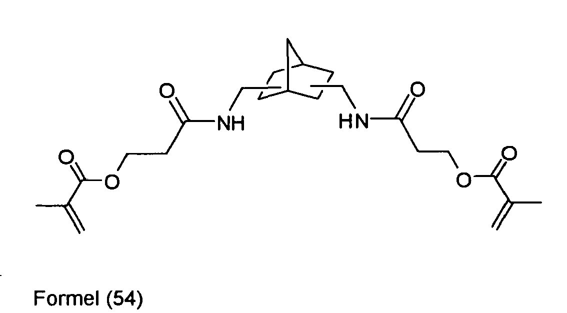

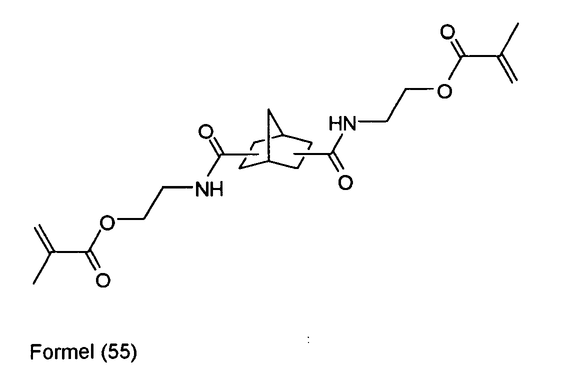

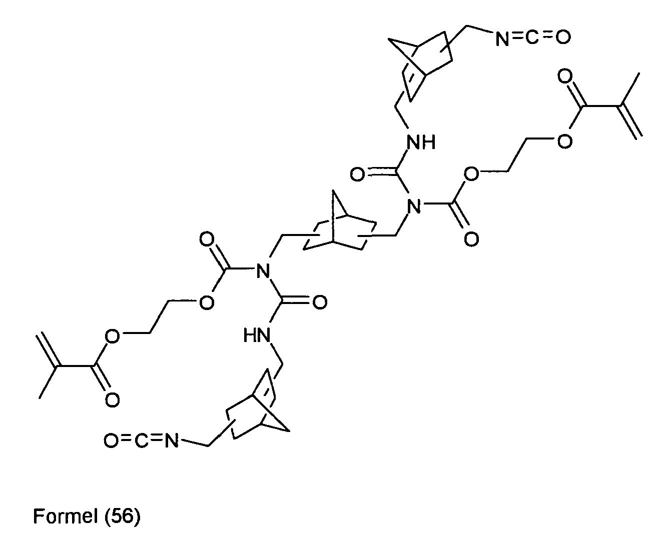

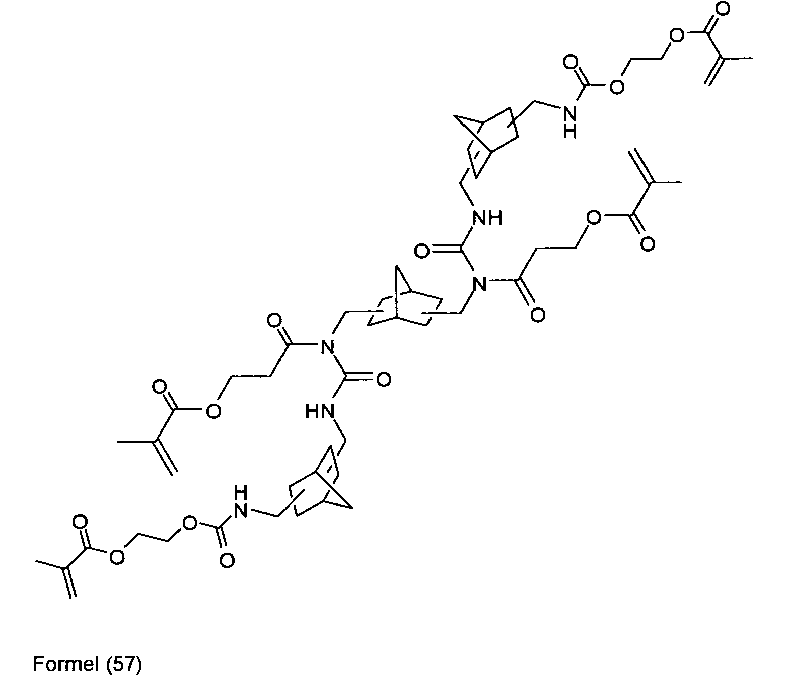

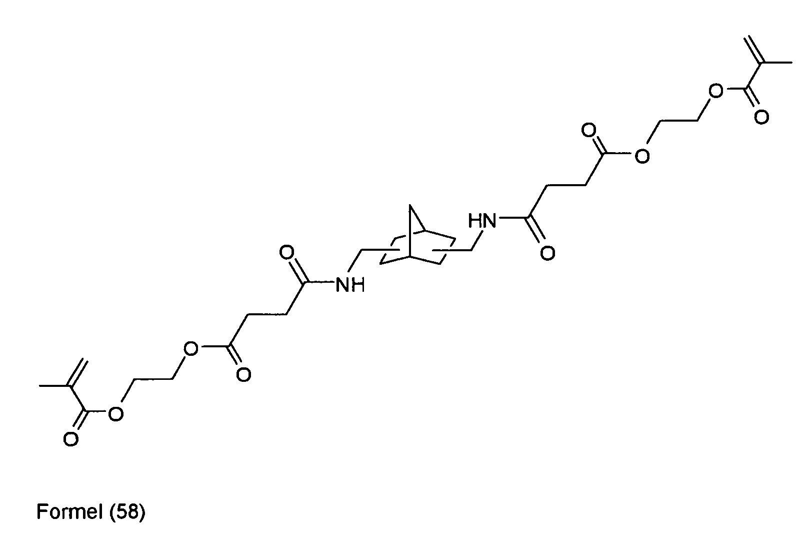

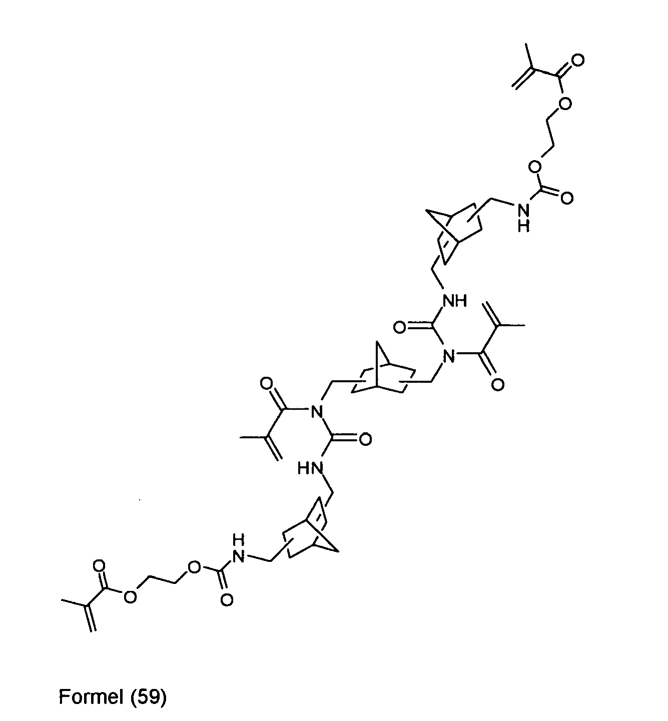

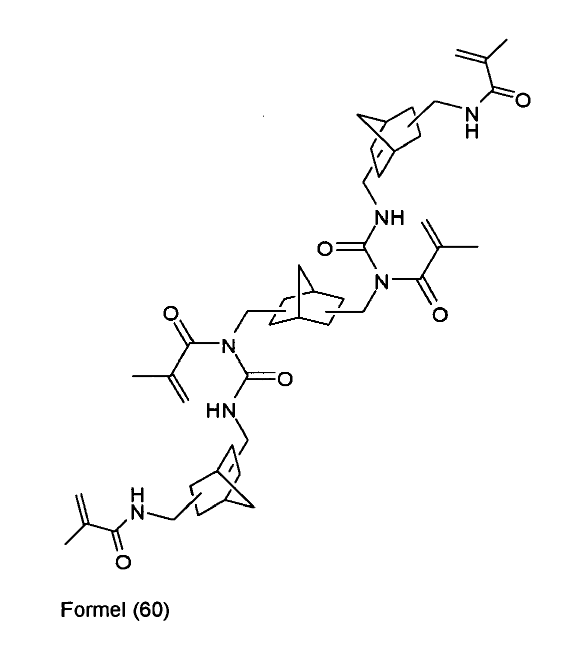

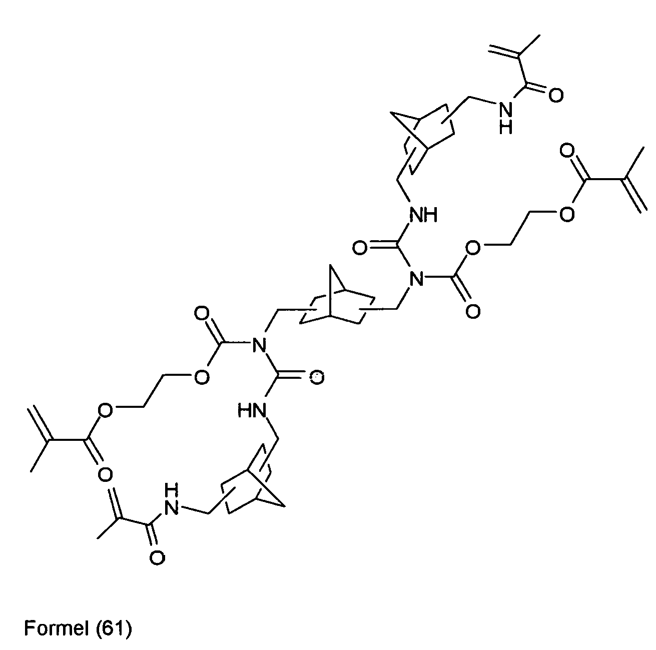

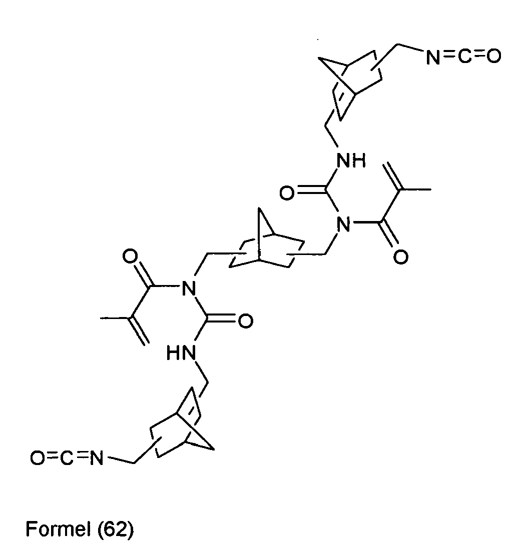

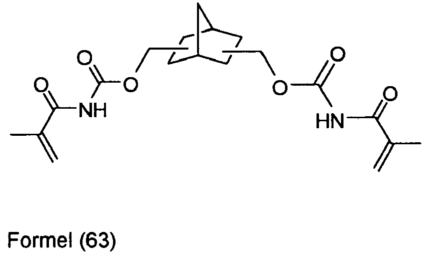

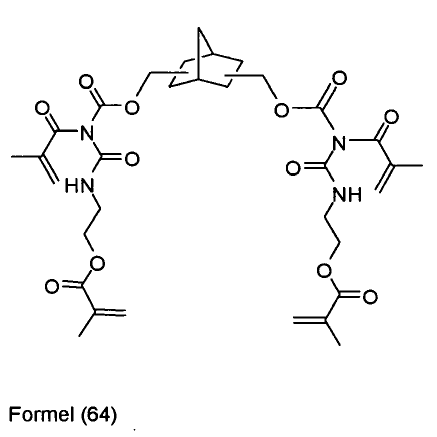

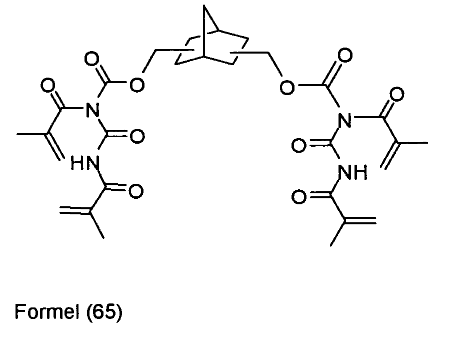

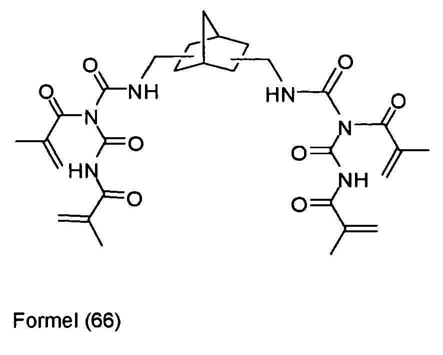

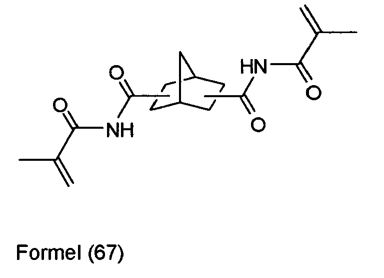

Ein erfindungsgemäßes Monomer kann zwei oder mehr polyalicyclische Strukturelemente umfassen, wobei diese identisch oder verschieden sein können.

Ein erfindungsgemäßes Monomer, dessen Molekül zwei voneinander verschiedene polyalicyclische Strukturelemente umfasst, ist zum Beispiel erhältlich, indem das TCD-Urethan der Formel (11) zunächst mit Diisocyanatonorbornan [Bis(isocyanatomethyl)bicyclo[2.2.1]heptan] und anschließend mit 2-Hydroxyethylmethacrylat umgesetzt wird, was zu der Verbindung der Formel (68) führt.

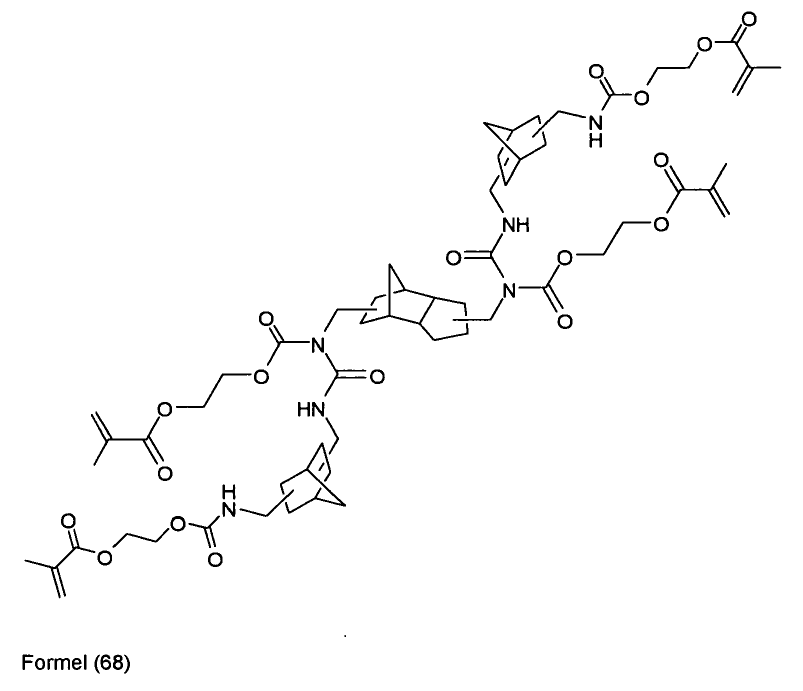

Die entsprechende Umsetzung der Verbindung der Formel (11) mit Diisocyanatoadamantan [(Bis(isocyanatomethyl)Tricyclo[3.3.1.1^{3,7}]decan] liefert ebenfalls ein erfindungsgemäßes Monomer, dessen Molekül zwei voneinander verschiedene polyalicyclische Strukturelemente Q umfasst, wie die nachfolgende Strukturformel der Verbindung der Formel (69) zeigt.

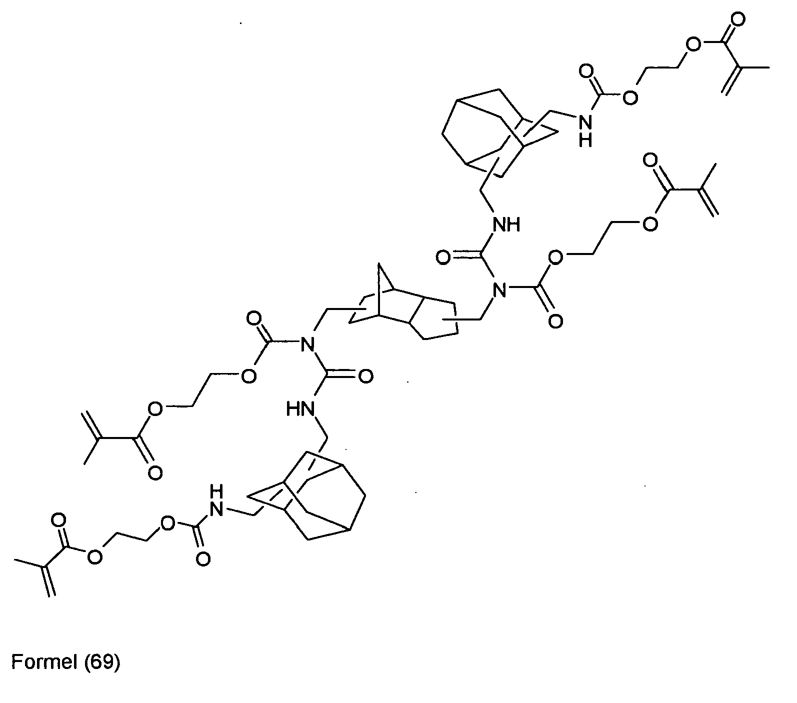

Die Herstellung der erfindungsgemäßen Monomere basiert auf dem an sich bekannten "Diisocyanat-Polyadditionsverfahren". Bei diesem Verfahren lagern sich die Isocyanatgruppen mit z.B. alkoholischen Hydroxylgruppen schnell und weitgehend quantitativ durch eine Additionsreaktion unter Wärmeentwicklung zusammen. Dabei reagieren die Isocyanate, von sterischen Einflüssen abgesehen, mit Additionspartnern umso rascher, je nucleophiler der Addend und je elektrophiler das Isocyanat ist.

Die Reaktion wird im Allgemeinen in einem inerten Lösungsmittel wie THF (Tetrahydrofuran), Toluol, Xylol, Methylenchlorid oder Acetonitril durchgeführt. Man kann die Umsetzung gegebenenfalls auch lösungsmittelfrei ausführen.

Es gibt zwei Hauptklassen geeigneter Katalysatoren für die Isocyanatadditionsreaktion: zum einen tertiäre Amine (wie Tri-N,N-dimethylaminomethylphenol oder 1,4-Diazabicyclo(2,2,2)octan auch Triethylendiamin genannt), die durch Abstraktion des Hydroxylwasserstoffatoms und Ausbildung von Alkoholatanionen die Alkohole aktivieren und so ihren nukleophilen Angriff auf das Isocyanatkohlenstoffatom beschleunigen, sowie eine Reihe von metallorganischen Verbindungen, die als Lewis Säuren die Elektrophilie des Isocyanatkohlenstoffatoms erhöhen. Bevorzugt eingesetzt werden für die erfindungsgemäßen Umsetzungen Metallsalze höherer Fettsäuren wie Dibutylzinndilaurat, Zinn(II)octoat, etc. oder Verbindungen wie das Eisen(III)acetylacetonat. Ganz bevorzugt ist der Einsatz von Dibutylzinndilaurat.

Der Katalysator wird vorzugsweise in Mengen von 0,01 bis 2 Gew.-%, bevorzugt von 0,08 bis 1 Gew.-%, bezogen auf die Gesamtmenge der Reaktanden gemäß A) und B) sowie gegebenenfalls C) und gegebenenfalls D) eingesetzt.

In einer bevorzugten Ausführungsform kann das Verfahren die Zugabe eines Polymerisationsinhibitors umfassen. Gängige und geeignete Inhibitoren sind beispielsweise Hydrochinonmonomethylether, Hydrochinon, 2,6-Di-tert.-butyl-4-methylphenol. Weitere geeignete Inhibitoren sind in der EP 0 783 880 genannt. Die Zugabe der Inhibitoren erfolgt im Allgemeinen in einer Menge von 0,001 bis 1 Gew.-%, bevorzugt von 0,01 bis 0,5 Gew.-%, bezogen auf die Gesamtmenge der Reaktanden.

Das Verfahren wird bevorzugt unter Ausschluss von Wasser durchgeführt. Bei der Synthese wird dann vorzugsweise eine Planschliffapparatur benutzt, an welcher ein Rührwerk, ein Kühler mit aufgesetztem Trockenrohr, das mit getrocknetem Kieselgur bestückt ist, ein regelbares Thermometer, das die Heizrate des Heizpilzes steuert, und ein Tropftrichter angeschlossen sind Die Apparatur wird vor der Bestückung mit den Edukten mit Hilfe einer Bunsenbrennerflamme ausgeheizt.

Die Umsetzung findet in einem Temperaturbereich von 0 bis 160°C, bevorzugt im Bereich von 30 bis 140°C und besonders bevorzugt im Bereich von 60 bis 120°C statt. Die Umsetzung wird bevorzugt bei Normaldruck ausgeführt.

Der Fortgang der Reaktion wird mit Hilfe der Änderung der Konzentration an Isocyanatgruppen verfolgt. Die Umsetzung der Isocyanatgruppen kann entweder auf nasschemischem oder spektroskopischem Wege verfolgt werden. Das nasschemische Prinzip der Analyse der Isocyanatgruppen beruht auf der quantitativen Umsetzung des Isocyanats mit einem Überschuss an Dibutylamin und Rücktitration des überschüssigen Amins mit Salzsäure gegen Bromphenolblau bis zum Umschlag von blau nach gelb. Spektroskopisch absorbieren NCO-Gruppen im Wellenlängenbereich von 2275 bis 2250 cm ⁻¹. Die Bande zeigt in diesem Bereich eine sehr hohe Intensität, deren Lage auch durch Konjugation nicht beeinflusst wird. Den charakteristischen Wellenlängenbereich der NCO-Bande erkennt man, wenn ein rein qualitatives Spektrum der Isocyanatverbindung in einem geeigneten Lösungsmittel, welches auch für die weiteren Synthesen verwandt werden soll, erstellt wird. Das Lösungsmittel darf keine Absorptionsbanden im Bereich der Wellenlänge aufweisen, der die charakteristischen Absorptionsbanden der NCO-Gruppe anzeigt. Benutzt man beispielsweise Toluol als Lösungsmittel, so kann man als "Fenster", also als den Wellenlängenbereich der charakteristischen Absorptionsbande, das Extinktionsmaximum der NCO-Bande bei 2267 cm ⁻¹ wählen.

Die Reaktion wird bis zum vollständigen Verschwinden der Isocyanatbande betrieben.

Die erfindungsgemäßen Monomere können vorteilhafterweise auch ohne spezielle Verfahren zur Aufreinigung als monomere Bausteine eingesetzt werden.

Die erfindungsgemäßen Monomere können einzeln, als Mischungen umfassend zwei oder mehr erfindungsgemäße Monomere sowie in Mischungen mit einem oder mehr herkömmlichen Monomeren sowie sogenannten Vernetzern eingesetzt werden. Durch die Mischung von zwei oder mehr verschiedenen erfindungsgemäßen Monomeren oder von einem, zwei oder mehr erfindungsgemäßen Monomeren mit einem, zwei oder mehr herkömmlichen Monomeren kann beispielsweise die Viskosität dem beabsichtigten Verwendungszweck angepasst werden. So können erfindungsgemäße Monomere beispielsweise mit Comonomeren niedriger Viskosität kombiniert werden.

Die erfindungsgemäßen Monomere können überall eingesetzt werden, wo flüssige, fließfähige Ausgangsmaterialien zu festen Endprodukten ausgehärtet werden. Der Übergang von der flüssigen zur festen Phase wird dabei chemisch, mittels Strahlung oder dual (d.h. sowohl chemisch als auch mittels Strahlung) initiiert. Der Mechanismus der Aushärtung erfolgt radikalisch und/oder ionisch. Einsetzbare Polymerisationsinitiatoren sind somit Photoinitiatoren und thermische Polymerisationskatalysatoren. Dem Fachmann sind radikalische Photoinitiatoren, radikalische Thermoinitiatoren, kationische Photoinitiatoren und kationische Thermoinitiatoren sowie deren Kombinationen bekannt.

Erfindungsgemäße Gemische umfassend die erfindungsgemäßen Monomere können verschiedene, dem Verwendungszweck angepasste Additive, Aktivatoren, Coinitiatoren, Lösungsmittel, Füllstoffe, Stabilisatoren, Pigmente, Reaktivverdünner, Comonomere, Inhibitoren, Molekulargewichtsregler, Fließmittel, Verlaufmittel, Hautverhinderungsmittel, Entschäumer, Antistatika, Weichmacher, Gleitmittel, Netz- und Dispergiermittel, Konservierungsmittel wie beispielsweise Fungizide und/oder Biozide, Modifikatoren zur Einstellung der Rheologie wie Thixotropiermittel und/oder Eindickmittel, Sensibilisatoren, grenzflächenaktiven Substanzen, Sauerstoff- und/oder Radikalfänger, Pigmente, Farbstoffe, Lichtschutzmittel, Mattierungsmittel, Brandschutzmittel, Trennmittel, usw. enthalten.

UV-Absorber, die beispielsweise durch ihre konjugierten Doppelbindungssytemen und aromatischen Ringe befähigt sind, UV Strahlung zu absorbieren, können gegebenenfalls zusätzlich Bestandteil eines erfindungsgemäßen Gemisches bzw. Erzeugnisses sein. Beispiele an UV-Absorbern sind 2-Hydroxy-4-methoxybenzophenon, Salicylsäurephenylester oder 3-(2'-Hydroxy-5'-methylphenyl)-benzotriazol.

Die Erfindung betrifft ferner ein Erzeugnis, erhältlich durch Härten einer erfindungsgemäßen Verbindung, einer erfindungsgemäßen Mischung oder eines erfindungsgemäßen Gemisches, vorzugsweise jeweils in einer der oben genannten bevorzugten bzw. besonders bevorzugten Ausgestaltungen.

Bei einem erfindungsgemäßen Erzeugnis handelt es sich vorzugsweise um ein Polymer oder einen Verbundwerkstoff, vorzugsweise ein dentales Polymer oder einen dentalen Verbundwerkstoff.

Die Erfindung betrifft ferner eine erfindungsgemäße Verbindung, eine erfindungsgemäße Mischung, ein erfindungsgemäßes Gemisch oder ein erfindungsgemäßes Erzeugnis, vorzugsweise jeweils in einer der oben genannten bevorzugten bzw. besonders bevorzugten Ausgestaltungen, zur Anwendung als Dentalmaterial, insbesondere als dentales Füllungsmaterial, dentales Unterfüllungsmaterial, als fließfähiges Kompositmaterial (Flow-Material), als Fissurenversiegler, als Wurzelkanalfüll- und -versiegelungsmaterial, als provisorisches Kronenmaterial, als provisorisches Brückenmaterial und/oder als Stumpfaufbaumaterial,

Die Erfindung betrifft ferner die Verwendung einer erfindungsgemäßen Verbindung, einer erfindungsgemäßen Mischung, eines erfindungsgemäßen Gemisches oder eines erfindungsgemäßen Erzeugnisses zur Herstellung eines Dentalmaterials.

Einsetzbar sind die erfindungsgemäßen Verbindungen, Mischungen und Gemische in Dentalmaterialien (Dentalzusammensetzungen), in oder zur Herstellung von Kleb-, Farb-, Lack-, Anstrichs- und Beschichtungszusammensetzungen, Vergussmassen, Dichtungsmassen, Spachtelmassen, Laminierharzen, Formmassen, Bindemitteln und Gießharzen, zum Einkapseln elektrischer und elektronischer Bauteile, zur Herstellung von magnetischen Aufzeichnungsmaterialien, mikromechanischen Teilen, optischen Schaltern, Glasfaserbeschichtungen, zur Herstellung von dreidimensionalen Gegenständen mittels Stereolithographie, für photographische Reproduktionsverfahren, für holographische Aufnahmematerialien, zur Herstellung von Druckplatten, als Haftmittel sowie zur Herstellung von Verbundwerkstoffen.

Die Erfindung betrifft daher auch die Verwendung einer erfindungsgemäßen Verbindung (vorzugsweise in einer der als bevorzugt bzw. besonders bevorzugt gekennzeichneten Ausgestaltungen), einer erfindungsgemäßen Mischung (vorzugsweise in einer der als bevorzugt bzw. besonders bevorzugt gekennzeichneten Ausgestaltungen) oder eines erfindungsgemäßen Gemisches (vorzugsweise in einer der als bevorzugt bzw. besonders bevorzugt gekennzeichneten Ausgestaltungen),
- in oder zur Herstellung von Kleb-, Farb-, Lack-, Anstrichs- oder Beschichtungszusammensetzungen, Vergussmassen, Dichtungsmassen, Spachtelmassen, Laminierharzen, Formmassen, Bindemitteln oder Gießharzen,
- zum Einkapseln elektrischer oder elektronischer Bauteile,
- zur Herstellung von magnetischen Aufzeichnungsmaterialien, mikromechanischen Teilen, optischen Schaltern, Glasfaserbeschichtungen, dreidimensionalen Gegenständen mittels Stereolithographie, Druckplatten oder Verbundwerkstoffen,
- für photographische Reproduktionsverfahren oder holographische Aufnahmematerialien,
- als Haftmittel.

Die Erfindung betrifft ferner ein Verfahren zur Herstellung eines Erzeugnisses, vorzugsweise eines dentalen Erzeugnisses, mit folgenden Schritten:
(i) Bereitstellen einer erfindungsgemäßen Verbindung, einer erfindungsgemäßen Mischung oder eines erfindungsgemäßen Gemisches, jeweils vorzugsweise in einer der als bevorzugt oder besonders bevorzugt gekennzeichneten Ausgestaltungen, als erste Komponente,
(ii) gegebenenfalls Herstellen einer Zubereitung durch Vermischen der bereitgestellten ersten Komponente mit einer oder mehreren weiteren Komponenten, vorzugsweise mit einem oder mehreren weiteren dentalen Materialien,
(iii) Härten des oder der Bestandteile aus Schritt (i) oder der Zubereitung gemäß Schritt (ii), wobei das Härten vorzugsweise chemisch und/oder durch Licht induziert und/oder thermisch induziert erfolgt.

In einer bevorzugten Ausgestaltung wird dazu die erste Komponente oder die Zubereitung gemäß Schritt (ii) vor dem Härten in Schritt (iii) aufgetragen auf, eingefügt in und/oder angebracht an die dafür vorgesehene Stelle, vorzugsweise einer Stelle in der Mundhöhle, wobei diese Stelle vorzugsweise zumindest einen oder mehrere Bereiche der Mundhöhle umfasst aus der Gruppe bestehend aus Zahnsubstanz (ein oder mehrere Zähne oder Teile eines Zahns (insbesondere Zahnstumpf, Schmelz, Dentin, Pulpa, Zahnhals, Zahnrand)), Zahnfleisch und/oder einem Bereich unterhalb eines Zahns (insbesondere Wurzel und Wurzelkanal).

Ferner betrifft die Erfindung eine erfindungsgemäße Verbindung, eine erfindungsgemäße Mischung, ein erfindungsgemäßes härtbares Gemisch bzw. ein erfindungsgemäßes Erzeugnis als bzw. zur Verwendung als dentales Füllungsmaterial, dentales Unterfüllungsmaterial, als fließfähiges Kompositmaterial (Flow-Material), als Fissurenversiegler, als Wurzelkanalfüll- und -versiegelungsmaterial, als provisorisches Kronenmaterial, als provisorisches Brückenmaterial und/oder als Stumpfaufbaumaterial.

Ferner betrifft die Erfindung ein Verfahren zum Behandeln einer Zahnerkrankung, wobei eine oder mehrere erfindungsgemäße Verbindungen, eine erfindungsgemäße Mischung, ein erfindungsgemäßes härtbares Gemisch bzw. ein erfindungsgemäßes dentales Erzeugnis, vorzugsweise in einer der als bevorzugt angegebenen Ausgestaltungen, als dentales Füllungsmaterial, dentales Unterfüllungsmaterial, als Flow-Material, als Fissurenversiegler, als Wurzelkanalfüll- und -versiegelungsmaterial, als provisorisches Kronenmaterial, als provisorisches Brückenmaterial und/oder als Stumpfaufbaumaterial eingesetzt wird.

Die Erfindung betrifft ferner die Verwendung einer erfindungsgemäßen Verbindung, vorzugsweise in einer der als bevorzugt bzw. besonders bevorzugt gekennzeichneten Ausgestaltungen, in einem Kompositmaterial, vorzugsweise in einem dentalen Kompositmaterial.

In einer bevorzugten Ausgestaltung betrifft die Erfindung ein Kompositmaterial, umfassend oder bestehend aus
(a) eine oder mehrere erfindungsgemäße Verbindungen oder eine erfindungsgemäße Mischung, vorzugsweise in einer der als bevorzugt bzw. besonders bevorzugt gekennzeichneten Ausgestaltungen,
(b-1) von Bestandteil (a) verschiedene Monomere, welche mit Bestandteil (a) co-polymerisierbar sind, vorzugsweise lichtpolymerisierbare Monomere, bevorzugt gewählt aus der Gruppe bestehend aus Acrylaten und Methacrylaten,
(b-2) 10 bis 75 Gew.-%, bezogen auf die Gesamtmasse des Kompositmaterials, einer Füllstoffkomponente bestehend aus
   (b-2a) 10 bis 60 Gew.-% nicht agglomerierten, organisch oberflächenmodifizierten Nanopartikeln mit einer mittleren Partikelgröße kleiner 100 nm (besonders bevorzugt kleiner 60 nm)
      und/oder
   (b-2b) 20 bis 75 Gew.-% Mikropartikeln mit einer mittleren Partikelgröße von 0,4 µm bis 10 µm
      sowie
   (b-2c) 0 bis 15 Gew.-% weiteren Füllstoffen, wobei die Füllstoffe nicht aus der Gruppe der Füllstoffe b-2a und b-2b gewählt sind,
      wobei die Gewichtsprozentangaben für die Komponenten (b-2a), (b-2b) und (b-2c) jeweils auf die Gesamtmasse des Kompositmaterials bezogen sind,
(b-3) ein oder mehrere Initiatoren und/oder Katalysatoren, sowie gegebenenfalls ein oder mehrere Additive.

Ein solches dentales Kompositmaterial ist zur Versiegelung von Fissuren, von kariösen Läsionen und zur Oberflächenversiegelung von Restaurationen geeignet, da es sich durch eine sehr geringe Wasseraufnahme, ein sehr gutes Anfließverhalten an der Zahnhartstruktur sowie ausgezeichnete mechanische Eigenschaften auszeichnet.

Die vorliegende Erfindung betrifft daher auch ein solches erfindungsgemäßes Kompositmaterial zur Verwendung als dentales Versiegelungsmaterial, insbesondere als dentales Versiegelungsmaterial zur Versiegelung von Fissuren, zur Versiegelung von Grübchen, zur Versiegelung kariöser Läsionen, zur Verblendung geschädigter Schmelzoberflächen, zur Reparatur kleiner Defekte an Kunststoff- und Amalgamfüllungen, zur Verankerung kieferorthopädischer Reguliersysteme und/oder zur Oberflächenversiegelung von Restaurationen.

In einer bevorzugten Ausgestaltung betrifft die Erfindung ein hochgefülltes Kompositmaterial bestehend aus oder umfassend
(b-2) 80 bis 95 Gew.-%, bezogen auf die Gesamtmasse des hochgefüllten Kompositmaterials, einer Mischung von Füllstoffpartikeln umfassend
   (b-2a) > 10, besonders bevorzugt > 12 bis 30 Gew.-% nicht agglomerierte, organisch oberflächenmodifizierte Nanopartikel mit einer mittleren Partikel-größe kleiner 100 nm (besonders bevorzugt kleiner 60 nm) und
   (b-2b) 45 bis < 84 Gew.-%, bevorzugt 45 bis < 83 Gew.-%, Mikropartikel mit einer mittleren Partikelgröße von 0,4 µm bis 10 µm
      sowie
   (b-2c) gegebenenfalls weitere Füllstoffe, wobei die Füllstoffe nicht aus der Gruppe der Füllstoffe b-2a und b-2b gewählt sind,
      wobei die Gewichtsprozentangaben für die Komponenten (b2-a) und (b-2b) auf die Gesamtmasse des Kompositmaterials bezogen sind,
und
3 bis 20 Gew.-%, bezogen auf die Gesamtmasse des hochgefüllten Kompositmaterials, einer Monomerenmischung umfassend
(a) eine oder mehrere erfindungsgemäße Verbindungen oder eine erfindungsgemäße Mischung, vorzugsweise in einer der als bevorzugt bzw. besonders bevorzugt gekennzeichneten Ausgestaltungen,
(b-1) ein, zwei oder mehr weitere radikalisch polymerisierbare Monomere aus der Gruppe bestehend aus Acrylaten und Methacrylaten, vorzugsweise der Gruppe der Methacrylate,
   wobei das Verhältnis der Masse der Komponente (a) zur Masse der Komponente (b-1) vorzugsweise im Bereich von 1 : 3 bis 3 : 1 liegt
(b-3) ein oder mehrere Initiatoren und/oder Katalysatoren, sowie
   gegebenenfalls ein oder mehrere Additive.

Im ausgehärteten Zustand zeichnen sich solche hochgefüllte Kompositmaterialien nicht nur aus durch eine geringe Polymerisationsschrumpfung (vorzugsweise weniger als 1,7 Vol-%, besonders bevorzugt weniger als 1,6 Vol.-%, gemessen nach der Bonded-Disc-Methode (Dental Materials 2004, 20, 88-95)), sondern auch durch eine geringe Abrasion (vorzugsweise weniger als 30 µm, bestimmt nach der ACTA-Methode) und eine hohe Vickers-Mikrohärte (vorzugsweise 140 oder mehr).

Ein erfindungsgemäßes hochgefülltes Kompositmaterial eignet sich als Dentalmaterial, insbesondere als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material.

In einer bevorzugten Ausgestaltung betrifft die Erfindung eine Lackzusammensetzung, vorzugsweise eine transparente Lackzusammensetzung, insbesondere eine dentale Lackzusammensetzung, bestehend aus oder umfassend

38 bis 96 Gew.-% einer Monomerenkomponente umfassend
(a) eine oder mehrere erfindungsgemäße Verbindungen oder eine erfindungsgemäße Mischung, vorzugsweise in einer der als bevorzugt bzw. besonders bevorzugt gekennzeichneten Ausgestaltungen,
   (b-1 a) ein, zwei oder mehr radikalisch polymerisierbare Monomere ausgewählt aus der Gruppe bestehend aus Methylmethacrylat, Ethylmethacrylat, Propylmethacrylat, Butylmethacrylat und Pentylmethacrylat,
   (b-1 b) ein, zwei oder mehr weitere radikalisch polymerisierbare Monomere mit drei oder mehr (Meth)acrylatgruppen, vorzugsweise mit drei bis sechs (Meth)acrylatgruppen, insbesondere Dipentaerythritolpentaacrylat,
      sowie
   (b-1 c) optional ein, zwei oder mehr weitere radikalisch polymerisierbare Monomere aus der Gruppe bestehend aus Acrylaten und Methacrylaten, vorzugsweise der Gruppe der Methacrylate,
   wobei das Verhältnis der Masse der Komponente (a) zur der Gesamtmasse der Komponenten (b-1 a), (b-1 b) und (b-1 c) vorzugsweise im Bereich von 4 : 1 bis 1 : 4 liegt,
(b-2) 0 bis 60 Gew.-% einer Füllstoffkomponente, umfassend einen, zwei oder mehrere Füllstoffe ausgewählt aus der Gruppe bestehend aus
   (b-2a) 0 bis 60 Gew.-% nicht agglomerierte, vorzugsweise oberflächenmodifizierte, Nanopartikel mit einer mittleren Partikelgröße kleiner 200 nm (vorzugsweise kleiner 100 nm, besonders bevorzugt kleiner 60 nm),
   (b-2b) 0 bis 10 Gew.-% Mikropartikel mit einer mittleren Partikelgröße von 0,4 µm bis 10 µm, und
   (b-2c) 0 bis 15 Gew.-% weitere Füllstoffe, vorzugsweise 0 bis 10 Gew.-%, bevorzugt 0 bis 5 Gew.-%, weiter bevorzugt 0 bis 2,5 Gew.-% weitere Füllstoffe, wobei die Füllstoffe nicht aus der Gruppe der Füllstoffe b-2a und b-2b gewählt sind,
(b-3) ein oder mehrere Initiatoren und/oder Katalysatoren,
   und
   gegebenenfalls ein oder mehrere Additive,
   wobei die Gewichtsprozentangaben jeweils auf die Gesamtmasse der Lackzusammensetzung bezogen sind.

Vorzugsweise beträgt dabei die Gesamtmenge an Komponente (a) und Komponente (b-1a) in einer erfindungsgemäßen Lackzusammensetzung zumindest 25 Gew.-%, bevorzugt 30 Gew.-% oder mehr, bevorzugt 35 Gew.-% oder mehr, jeweils bezogen auf die Gesamtmasse der Lackzusammensetzung.

Diese erfindungsgemäßen, vorzugsweise transparenten Lackzusammensetzungen, sind als Schutz- und Glanzlacke, insbesondere im Dentalbereich, geeignet. Diese Lackzusammensetzungen weisen eine sehr geringe Wasseraufnahme sowie sehr gute mechanische Eigenschaften (insbesondere Biegefestigkeit und E-Modul) auf. Darüberhinaus zeichnen sie sich durch eine sehr gute Adaptation, d.h. sehr gutes Anfließverhalten an der entsprechenden Oberfläche, insbesondere der Zahnhartstruktur (speziell am trockenen Zahnschmelz) aus.

Im nicht-ausgehärteten Zustand zeichnet sich eine erfindungsgemäße Lackzusammensetzung, insbesondere eine erfindungsgemäße dentale Lackzusammensetzung, durch einen geringen Kontaktwinkel (KW) auf trockenem Zahnschmelz aus (vorzugsweise von weniger als 50°, bevorzugt weniger als 40°, besonders bevorzugt weniger als 30°, gemessen mit einem Kontaktwinkelmessgerät der Firma Krüss (DSA 100)). Vor Durchführung der Kontaktwinkel-Messung wurde hierzu ein humaner Zahn mit einem Zellstofftuch getrocknet ("trockener Zahnschmelz").

Ferner hat sich gezeigt, dass eine erfindungsgemäße Lackzusammensetzung, insbesondere eine erfindungsgemäße dentale Lackzusammensetzung, im ausgehärteten Zustand eine sehr geringe Wasseraufnahme aufweist, vorzugsweise weniger als 50 µg/mm³, vorzugsweise weniger als 45 µg/mm³ und bevorzugt weniger als 40 µg/mm³, besonders bevorzugt weniger als 35 µg/mm³. Die Wasseraufnahme wurde dabei analog zu ISO 4049 bestimmt.

Die erfindungsgemäßen Lackzusammensetzungen sind vielseitig auf verschiedenen Substraten einsetzbar und werden vorzugsweise als Kavitäten- und Oberflächenlack im Dentalbereich verwendet. Die erfindungsgemäßen Lackzusammensetzungen werden bevorzugt auf Glasionomerfüllungen, provisorischen Kronen und Brücken aus Komposit sowie auf Kompositrestaurationen eingesetzt. Die adhäsiven "Interfaces" zwischen Komposit und Zahnhartsubstanz können durch erfindungsgemäße Lackzusammensetzungen geschützt werden.

Als Kavitätenlacke können die erfindungsgemäßen Lackzusammensetzungen als Unterfüllung für Zahnfüllmaterialien dienen und dabei die Pulpa vor schädigenden Monomeren abschirmen. Als Oberflächenlack können die erfindungsgemäßen Lackzusammensetzungen den Zahnschmelz vor äußeren Beeinträchtigungen und gegenüber den in der Mundhöhle und an der Zahnoberfläche vorhandenen Substanzen schützen und ein Auftreten von Karies verhindern.

Erfindungsgemäße dentale Lackzusammensetzungen sind vorzugsweise so ausgestaltet, dass sie als Kavitäten- und/oder Oberflächenlack eingesetzt werden können, als Unterfüllung für Zahnfüllmaterialien, zum Schutz des Zahnschmelzes, zur Verhinderung von Karies, zum Schutz einer Restauration (insbesondere gegen vorzeitige Abnutzung), zur Verbesserung der Verschleissfestigkeit eines dentalen Füllungsmaterials, zur Festigung restaurierter Oberflächen, zum Schutz vor Abrasion und/oder Verfärbung eines Zahns oder einer Restauration, zum Schließen marginaler Spalten und/oder von Mikrorissen, zur Glättung von restaurativen Oberflächen, zum Verleihen von natürlichem Glanz auf einem Zahn oder einer Restauration und zum Reduzierung von Farbstoffablagerungen auf einem Zahn oder einer Restauration. Entsprechende Verwendungen einer erfindungsgemäßen Lackzusammensetzung sind bevorzugt.

Ferner sind die erfindungsgemäßen Verbindungen hervorragend geeignet, um als Wurzelkanalfüllungs- und/oder versiegelungsmaterial eingesetzt zu werden.

In einer bevorzugten Ausgestaltung betrifft die Erfindung eine härtbare dentale Zusammensetzung zum Füllen und/oder Versiegeln eines Wurzelkanals bestehend aus oder umfassend:
(a) 5 Gew.-% oder mehr einer oder mehrerer erfindungsgemäßer Verbindungen oder einer erfindungsgemäßen Mischung, vorzugsweise in einer der als bevorzugt bzw. besonders bevorzugt gekennzeichneten Ausgestaltungen,
(b-1) 5 Gew.-% oder mehr von ein, zwei oder mehr weiteren polymerisierbaren Monomeren aus der Gruppe bestehend aus Acrylaten und Methacrylaten, vorzugsweise mit einem Polyetherpolyol-Strukturelement, dabei bevorzugt ein oder mehrere Polyethylenglykoldi(meth)acrylate mit 4 bis 10 Ethylenoxideinheiten,
(b-2) 35 - 80 Gew.-% einer Füllstoffkomponente umfassend einen, zwei oder mehrere röntgenopake Füllstoffe, und
(b-3) ein oder mehrere Initiatoren und/oder Katalysatoren,
   sowie gegebenenfalls weitere Additive,
   wobei die Gesamtmenge der Monomere der Komponenten (a) und (b-1) vorzugsweise 20 Gew.-% oder mehr beträgt,
   und/oder
   wobei die Gesamtmenge der röntgenopaken Füllstoffe der Komponente (b-2) vorzugsweise im Bereich von 30 bis 75 Gew.-%, bevorzugt im Bereich von 40 bis 70 Gew.-% liegt, weiter bevorzugt im Bereich von 50 bis 70 Gew.-%, besonders bevorzugt im Bereich von 55 bis 65 Gew.-%
   wobei sich die Gewichtsprozentangaben jeweils auf die Gesamtmasse der Zusammensetzung beziehen.

Um eine weiter verbesserte Haftung an Zahnschmelz und/oder Dentin zu erreichen, können erfindungsgemäße Verbindungen vorzugsweise mit ein oder mehreren haftfördernden Additiven kombiniert werden.

Bevorzugt sind daher erfindungsgemäße Gemische, die als Komponente (b-6) ein oder mehrere haftfördernde Additive enthalten.

Weiter bevorzugt sind erfindungsgemäße Gemische umfassend
(a) eine oder mehrere erfindungsgemäße Verbindungen oder eine erfindungsgemäße Mischung, vorzugsweise in einer der als bevorzugt bzw. besonders bevorzugt gekennzeichneten Ausgestaltungen, und
(b-6) ein oder mehrere haftfördernde Additive, ausgewählt aus der Gruppe bestehend aus polymerisierbaren oder nicht polymerisierbaren Säuren oder Carbonsäureanhydriden, vorzugsweise aus der Gruppe bestehend aus Phosphorsäuren, Phosphonsäuren, Carbonsäuren und deren Salze, Carbonsäureestern und Carbonsäureanhydriden,
   bevorzugt in einer Menge im Bereich von 0,1 bis 5 Gew.-%, weiter bevorzugt in einer Menge im Bereich von 0,5 bis 2,5 Gew.-%, jeweils bezogen auf die Gesamtmasse der Zusammensetzung.

Vorzugsweise sind die ein oder mehreren haftfördernde Additive der Komponente (b-6) ausgewählt aus der Gruppe bestehend aus
10-(Meth)acryloyloxydecyldihydrogenphosphat (10-MDP), 2-(Meth)acryloyloxyethyl-dihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogen-phosphat, 2-(Meth)acryloyloxynonyldihydrogenphosphat, 11-(Meth)acryloyloxyun-decyldihydrogenphosphat, 20-(Meth)acryloyloxyeicosyldihydrogenphosphat, 1,3-Di(meth)acyloyloxypropyl-2-dihydrogenphosphat, 2-(Meth)acryloyloxyethylphenyl-dihydrogenphosphat, Di(2-(meth)acyloyloxyethyl)pyrophosphat, Di(2-(meth)acyloyloxypropyl)pyrophosphat, Di(2-(meth)acyloyloxybutyl)pyrophosphat, Di(2-(meth)acyloyloxypentyl)pyrophosphat, das Di(2-(meth)acyloyloxyhexyl)pyrophosphat, Di(2-(meth)acyloyloxydecyl)pyrophosphat, Mono-, Di- und/oder Triester der Phosphorsäure, welche durch Umsetzung von Hydroxy-C2-C8-alkylmethacrylat (dabei vorzugsweise Hydroxyethylmethacrylat) oder Glyceryldimethacrylat mit Phosphoroxychlorid erhalten werden, Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäureethylendiamintetraessigsäureester.

Dabei wiederum bevorzugte haftfördernde Additive der Komponente (b-6) sind 10-(Meth)acryloyloxydecyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat.

Ebenfalls bevorzugt sind erfindungsgemäße härtbare dentale Zusammensetzungen zum Füllen und/oder Versiegeln eines Wurzelkanals (wie oben definiert), welche als Komponente (b-6) ein oder mehrere haftfördernde Additive enthalten, da die Haftung an Dentin für solche Zusammensetzungen relevant ist. Dabei gelten die als bevorzugt bzw. besonders bevorzugt gekennzeichneten Ausgestaltungen, auch hier entsprechend.

### Beispiele

Anhand der folgenden Beispiele wird die Erfindung weiter erläutert. Sofern nicht anders angegeben, beziehen sich alle Angaben auf das Gewicht. Es werden dabei folgende branchenübliche Abkürzungen verwendet:
BHT = 2,6-Di-tert.butyl-4-methylphenol
UDMA = Urethandimethacrylat (7,7,9-Trimethyl-4,13-dioxo-5,12-diazahexadecan-1,16-dioxydimethacrylat)
CC = Campherchinon
DABE = Ethyl-p-N,N-dimethylaminobenzoat
Bis-GMA = Bisphenol-A-Glycidyl-Methacrylat
TEDMA = Triethylenglykoldimethacrylat

Für die im Folgenden eingesetzte Katalysatorlösung wurden 0,50 g Dibutylzinn(II)dilaurat in 9,50 g Toluol gelöst.

### Beispiel 1: Synthese der Verbindung der Formel (1) (nicht erfindungsgemäß)

1,90 g (9,68 mmol) 3(4), 8(9)-Bis(hydroxymethyl)tricyclo[5.2.1.0^{2,6}]decan wurden in 10 mL Tetrahydrofuran gelöst und mit 0,04 g BHT und 0,105 g der Katalysatorlösung versetzt. Unter Rühren wurden 3,00 g (19,34 mmol, 2 Äquivalente) Isocyanatoethylmethacrylat, gelöst in 10 mL THF, zugetropft. Nach beendeter Zugabe wurde der Tropftrichter durch einen Rückflusskühler ausgetauscht und das Reaktionsgemisch auf 60 °C erwärmt und der Fortschritt der Reaktion mittels IR-Spektroskopie verfolgt. Nach 24 Stunden konnte keine Isocyanatbande mehr detektiert werden. Das Lösungsmittel wurde am Rotationsverdampfer entfernt. Das Urethan der Formel (1) wurde in einer Ausbeute von 3,40 g (6,74 mmol, 70%) als leicht gelbliches Öl erhalten.

### Beispiel 2: Synthese der Verbindung der Formel (2)

0,95 g (4,84 mmol) 3(4), 8(9)-Bis(hydroxymethyl)tricyclo[5.2.1.0^{2,6}]decan wurden in 10 mL Toluol gelöst und mit 0,04 g BHT und 0,103 g der Katalysatorlösung versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

### Beispiel 3: Synthese der Verbindung der Formel (26) (nicht erfindungsgemäß)

1,88 g (9,67 mmol) 3 (4), 8(9)-Bis(Aminomethyl)tricyclo[5.2.1.0^{2,6}]decan wurden in 10 mL Tetrahydrofuran gelöst und mit 0,04 g BHT und 0,104 g der Katalysatorlösung versetzt. Unter Rühren wurden 3,00 g (19,34 mmol, 2 Äquivalente) Isocyanatoethylmethacrylat, gelöst in 10 mL THF, zugetropft. Nach beendeter Zugabe wurde der Tropftrichter durch einen Rückflusskühler ausgetauscht und das Reaktionsgemisch auf 60 °C erwärmt und der Fortschritt der Reaktion mittels IR-Spektroskopie verfolgt. Nach 3 Stunden konnte keine Isocyanatbande mehr detektiert werden. Das Lösungsmittel wurde am Rotationsverdampfer entfernt. Die Verbindung der Formel (26) wurde in einer Ausbeute von 3,90 g (7,76 mmol, 80%) als leicht gelbliches Öl erhalten.

### Beispiel 4: Synthese der Verbindung der Formel (27)

0,94 g (4,84 mmol) 3(4), 8(9)-Bis(Aminomethyl)tricyclo[5.2.1.0^{2,6}]decan wurden in 10 mL Toluol gelöst und mit 0,04 g BHT und 0,104 g der Katalysatorlösung versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 100 °C erwärmt und der Fortschritt der Reaktion mittels IR-Spektroskopie verfolgt. Nach 2 Tagen konnte keine Isocyanatbande mehr detektiert werden. Das Lösungsmittel wurde am Rotationsverdampfer entfernt. Das Biuret (Formel (27) wurde in einer Ausbeute von 3,90 g (4,79 mmol, 99%) als leicht gelbliches Öl erhalten.

### Beispiel 5: Synthese der Verbindung AX1

1,19 g (6,46 mmol) 1,3,5-Trihydroxyadamantan wurden in 10 mL Tetrahydrofuran gelöst und mit 0,04 g BHT und 0,105 g der Katalysatorlösung versetzt. Unter Rühren wurden 3,00 g (19,34 mmol, 3 Äquivalente) Isocyanatoethylmethacrylat, gelöst in 10 mL THF, zugetropft. Nach beendeter Zugabe wurde der Tropftrichter durch einen Rückflusskühler ausgetauscht und das Reaktionsgemisch auf 60 °C erwärmt und der Fortschritt der Reaktion mittels IR-Spektroskopie verfolgt. Nach 6 Tagen konnte keine Isocyanatbande mehr detektiert werden. Das Lösungsmittel wurde am Rotationsverdampfer entfernt. Verbindung AX1 wurde in einer Ausbeute von 3,61 g (5,69 mmol, 88%) als kristalliner Feststoff erhalten.

### Beispiel 6: Synthese der Verbindung AX2

0,60 g (3,26 mmol) 1,3,5-Trihydroxyadamantan wurden in 20 mL Toluol gelöst und mit 0,04 g BHT und 0,101 g der Katalysatorlösung versetzt. Unter Rühren wurden 3,03 g (19,54 mmol, 6 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 6 Tagen konnte keine Isocyanatbande mehr detektiert werden. Das Lösungsmittel wurde am Rotationsverdampfer entfernt. Das Allophanat AX2 wurde in einer Ausbeute von 3,52 g (3,16 mmol, 97%) als weisser Feststoff erhalten.

### Beispiel 7: Synthese der Verbindung der Formel (35)

0,59 g (3,07 mmol) 1,3-Dicarboxyadamantan wurden in 20 mL Toluol gelöst und mit 0,04 g BHT und 0,105 g der Katalysatorlösung versetzt. Unter Rühren wurden 0,93 g (5,99 mmol, 2 Äquivalente) Isocyanatoathylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 2 Tagen konnte keine Isocyanatbande mehr detektiert werden. Das Lösungsmittel wurde am Rotationsverdampfer entfernt. Die Verbindung AX3 wurde in einer Ausbeute von 1,34 g (3,00 mmol, 98%) als weisser Feststoff erhalten.

### Beispiel 8: Synthese einer erfindungsgemäßen Mischung (NCO : OH = 1,25 : 1,00)

1,50 g (7,64 mmol) 3(4), 8(9)-Bis(hydroxymethyl)tricyclo[5.2.1.0^{2.6}]decan wurden in 10 mL Toluol gelöst und mit 0,04 g BHT und 0,101 g der Katalysatorlösung versetzt. Unter Rühren wurden 2,94 g (19,10 mmol, 2,5 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 4 Tagen wurde keine Isocyanatbande mehr detektiert. Das Lösungsmittel wurde am Rotationsverdampfer entfernt. Das Produkt wurde in einer Ausbeute von 4,42 g als leicht gelbliches Öl erhalten.

### Beispiel 9: Messung der Festigkeit und Wasseraufnahme

Um den positiven Einfluss der chemischen Struktur der erfindungsgemäßen Monomere auf die Festigkeit und Wasseraufnahme zu zeigen, wurden Probekörper aus verschiedenen beispielhaften Harzmischungen vorbereitet und deren Wasseraufnahme und Biegefestigkeit gemessen. Die Zusammensetzungen der Harzmischungen (in Gew.-%) sowie die Ergebnisse der Messungen sind in der folgenden Tabelle aufgelistet.

| | Vergleichsbeispiel A | Vergleichsbeispiel B | Vergleichsbeispiel C | D* | Vergleichsbeispiel E | F* | G* | H* | I* | J* |
|---|---|---|---|---|---|---|---|---|---|---|
| UDMA | 59,55 | 59,55 | 59,55 | 59,55 | 59,55 | 59,55 | 59,55 | 59,55 | 59,55 | 59,55 |
| TEDMA | 19,85 | 39,85 | 19,85 | 19,85 | 19,85 | 19,85 | 19,85 | 19,85 | 19,85 | 19,85 |
| erfindungsgemäßes Monomer* | 0 | 0 | 0 | 20,00 | 0 | 20,00 | 20,00 | 20,00 | 20,00 | 20,00 |
| Monomer der Formel (1)* | 0 | 0 | 20,00 | 0 | 0 | 0 | 0 | 0 | 0 | 0 |
| Monomer der Formel (26)* | 0 | 0 | 0 | 0 | 20,00 | 0 | 0 | 0 | 0 | 0 |
| Bis-GMA | 20,00 | 0 | 0 | 0 | 0 | 0 | 0 | 0 | 0 | 0 |
| CC | 0,24 | 0,24 | 0,24 | 0,24 | 0,24 | 0,24 | 0,24 | 0,24 | 0,24 | 0,24 |
| DABE | 0,36 | 0,36 | 0,36 | 0,36 | 0,36 | 0,36 | 0,36 | 0,36 | 0,36 | 0,36 |
| Biegefestigkeit [MPa] | 92,9 | 85,7 | 86,5 | 102,9 | 100,9 | 109,1 | 101,3 | 110,5 | 99,8 | 96,8 |
| Wasseraufnahme [µg/mm³] | 29,08 | 36,41 | 26,15 | 18,17 | 32,54 | 21,38 | 24,15 | 20,17 | 22,72 | 23,0 |

| | | | | | | | | | | |
|---|---|---|---|---|---|---|---|---|---|---|
| * Folgende Monomere wurden jeweils eingesetzt: Vergleichsbeispiel C: Verbindung der Formel (1) Beispiel D: Verbindung der Formel (2) Vergleichsbeispiel E: Verbindung der Formel (26) Beispiel F: Verbindung der Formel (27) Beispiel G: Verbindung AX1 Beispiel H: Verbindung AX2 Beispiel I: Verbindung der Formel (35) Beispiel J: Monomer-Mischung aus Beispiel 8 | | | | | | | | | | |

Die Bestimmung der Biegefestigkeit wurde in Anlehnung an die ISO4049 durchgeführt. In einer Form aus PTFE wurden Probekörper mit den Maßen 2 x 2 x 50 mm hergestellt. Diese wurden anschließend für 60 Minuten in einem Lichtkasten polymerisiert. Nach der Entformung wurden die Probekörper bis zur Messung 24 Stunden bei 37°C in destilliertem Wasser gelagert. Die Messung der Biegefestigkeit erfolgte mit einer Universalprüfmaschine bei einer Traversengeschwindigkeit von 0,75 mm/min und einer Einspannlänge von 25 mm bis zum Bruch.

Für die Wasseraufnahme wurden ebenfalls Probekörper der Maße 2 x 2 x 50 mm³ wie oben beschrieben vorbereitet und im Lichtkasten für 60 Minuten polymerisiert. Anschließend wurden das Gewicht, sowie die genauen Dimensionen der Probekörper auf 0,01 mm genau bestimmt und daraus das Volumen berechnet. Die so bereiteten Probekörper wurden für 72 Stunden bei 37°C in destilliertem Wasser gelagert. Nachdem die Oberflächen mit einem Zellstofftuch getrocknet wurden, wurde das Gewicht erneut bestimmt. Durch Division der Gewichtszunahme durch das Probekörpervolumen kann die Wasseraufnahme in µg/mm³ ermittelt werden.

Aus dem Vergleich der Resultate ist ersichtlich, dass Harzmischungen umfassend die erfindungsgemäßen Monomere mindestens in einer der Eigenschaften Biegefestigkeit und Wasseraufnahme günstigere Werte aufweisen als die Harzmischungen gemäß dem Stand der Technik. In den meisten Beispielen sind die Harzmischungen umfassend erfindungsgemäße Monomere sogar in beiden Parametern dem Stand der Technik überlegen.

### Weitere Beispiele erfindungsgemäßer Gemische und daraus durch Härten erhaltener Erzeugnisse:

### Beispiel D1: dentale Lackzusammensetzung

Das folgende erfindungsgemäße Beispiel zeigt eine dentale Lackzusammensetzung.

| Dentale Lackzusammensetzung | Gewichtsteile |
|---|---|
| Phosphinoxidinitiator | 1,998 |
| UV-Stabilisator | 0,029 |
| BHT | 0,017 |
| Methylmethacrylat | 9,52 |
| Dipentaerythritolpentaacrylat | 9,52 |
| Monomer der Formel (2) | 19,05 |
| Nano-SiO₂ (mittlere Partikelgröße 50 nm) | 59,87 |

| | |
|---|---|
| Biegefestigkeit [MPa] | 112 |
| E-Modul [MPa] | 3735 |
| KW [°] trockener Zahnschmelz | 25,6 |
| Wasseraufnahme [µg/mm³] | 32,17 |

### Beispiel D2: dentales Kompositmaterial

Das folgende erfindungsgemäße Beispiel zeigt ein dentales Kompositmaterial. Dieses Kompositmaterial ist als dentales Versiegelungsmaterial, insbesondere für die Versiegelung von Fissuren, von Grübchen, kariöser Läsionen und zur Reparatur kleiner Defekte an Kunststoff- und Amalgamfüllungen oder zur Oberflächenversiegelung von Restaurationen geeignet.

| Dentales Kompositmaterial | Gewichtsteile |
|---|---|
| | |
| Monomer der Formel (2) | 21,38 |
| UDMA | 17,35 |
| TEDMA | 12,44 |
| | |
| UV-Stabilisator | 0,25 |
| DABE | 0,22 |
| Katalysator (CC) | 0,15 |
| BHT | 0,09 |
| Glaskeramik 0,7 µm (silanisiert) | 26,85 |
| Reaktives Aluminiumsilikatglas (<10 µm) | 4,13 |
| Aerosil | 1,65 |
| Titandioxid (silanisiert) | 0,62 |
| Nanofüllstoff (silanisiert) | 14,87 |

| | |
|---|---|
| Biegefestigkeit [MPa] | 138 |
| E-Modul [MPa] | 6319 |
| KW [°] trockener Zahnschmelz | 29,5 |
| Wasseraufnahme [µg/mm³] | 11,91 |
| Acta-Abrasion | 75 µm |

### Beispiel D3: hochgefülltes dentales Kompositmaterial

Das folgende erfindungsgemäße Beispiel zeigt ein dentales hochgefülltes Kompositmaterial. Dieses Kompositmaterial ist als Füllungs-, Unterfüllungs-, Befestigungs- und/oder Stumpfaufbaumaterial, als provisorisches Kronen- und/oder Brückenmaterial, als Prothesen- und/oder Unterfütterungsmaterial oder als Flow-Material geeignet.

| Hochgefülltes Kompositmaterial | Gewichtsteile |
|---|---|
| (a) Füllstoffe | |
| (a1) SiO₂-Nanopartikel (d₅₀ = 50 nm, silanisiert) | 22,62 |
| (a2) Mikropartikel, erste Fraktion | 52,44 |
| (d₅₀ = 1,5 µm, silanisiertes Dentalglas) | |
| (a2) Mikropartikel, zweite Fraktion (d₅₀ = 0,7 µm, silanisiertes Dentalglas) | 14,86 |
| (b) Monomere | |
| (b1) Verbindung der Formel (2) | 7,60 |
| (b2) UDMA | 1,90 |
| (b2) TEDMA | 0,43 |
| (c) CC | 0,05 |
| (c) DABE | 0,075 |
| (d) BHT | 0,014 |
| (d) UV-Absorber | 0,071 |

| | |
|---|---|
| Vickers-Mikrohärte [MHV] | 162,5 |
| ACTA [µm] | 22,6 |
| Polymerisationsschrumpfung [%] | 1,49 |
| Quotient (MHV/(ACTA*Polymerisationsschrumpfung)) [100/µm] | 4,83 |

### Beispiel D4: Wurzelkanalfüll - und -versiegelungsmaterial

Das folgende erfindungsgemäße Beispiel zeigt eine dualhärtende dentale Zusammensetzung, die zum Füllen und/oder Versiegeln von Wurzelkanälen geeignet ist.

Es werden dabei folgende Bezeichnungen bzw. Abkürzungen verwendet:
PEG-400-DMA = Polyethylenglykol-400-dimethacrylat
Nano-SiO₂ = silanisierte SiO₂-Partikel (40 nm)
GK = hochröntgenopakes Zr-haltiges Glas (d50 = 5 µm; BET-Oberfläche: 0,5 m²/g)
Silanisierte Kieselsäure = Kieselsäure mit einer mittleren Partikelgröße von 13 nm (BET-Oberfläche: 160 m²/g ± 25 m²/g)

Die beide jeweiligen Pasten A und B flossen gut aus und waren gut miteinander mischbar.

### Pasten A und B des Wurzelkanalversiegelungsmaterials

| | Paste A Gewichtsteile | Paste B Gewichtsteile |
|---|---|---|
| Nano-SiO₂ | 6,16 | 6,16 |
| Verbindung der Formel (2) | 12,30 | 12,32 |
| PEG-400-DMA | 12,30 | 12,32 |
| GK | 64,57 | 64,69 |
| Silanisierte Kieselsäure | 4,11 | 4,11 |
| Allylthioharnstoff | 0,28 | 0,00 |
| CC | 0,05 | 0,00 |
| DABE | 0,07 | 0,00 |
| gamma-Terpinen | 0,16 | 0,00 |
| Cumolhydroperoxid (88%) in Cumol | 0,00 | 0,40 |

Die beiden Pasten A und B wurden zu gleichen Volumenanteilen miteinander vermischt (1:1 (v/v)).

Die Ergebnisse der Messungen für die jeweiligen Wurzelkanalversiegelungsmaterialien sind in der nachfolgenden Tabelle aufgelistet.

| | |
|---|---|
| Biegefestigkeit [MPa] | 7,1 |
| E-Modul [MPa] | 105 |
| Wasseraufnahme [µg/mm³] | 14 |
| Wasseraufnahme | 0,57% |
| Löslichkeit [µg/mm³] | 2 |
| Löslichkeit | 0,07% |
| Flow (ISO 6876) | 29 mm |
| Röntgenopazität (Al) | 6,35 mm |
| Abbindezeit | 22 min |
| Verarbeitungszeit | 43 min |

## Patentansprüche

1. Verbindung der Struktur Q(YZₑ)_{b} mit ein, zwei, drei, vier oder mehr funktionellen Gruppen, welche ausgewählt sind aus der Gruppe bestehend aus *N*-Acylharnstoff, Allophanat und Biuret,
wobei hier und nachfolgend gilt:
- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei keines, eines, zwei oder mehr der nicht durch Substituenten YZₑ substituierten Wasserstoffatome dieses polyalicyclischen Strukturelements Q durch Alkylgruppen, Alkoxygruppen, Halogenatome oder Trifluormethylgruppen substituiert ist bzw. sind;
- b ist eine natürliche Zahl ausgewählt aus der Gruppe der natürlichen Zahlen 2, 3, 4;
- jedes Z bedeutet ein Strukturelement, das unabhängig von etwaigen weiteren Strukturelementen Z ausgewählt ist aus der Gruppe bestehend aus
-O-(C=O)-CH=CH₂, -O-(C=O)-C(CH₃)=CH₂,
-(C=O)-CH=CH₂, -(C=O)-C(CH₃)=CH_{2;}
-CH=CH₂ und -C(CH₃)=CH₂;
- jeder Index e ist eine natürliche Zahl, die unabhängig von etwaigen weiteren Indizes e ausgewählt ist aus der Gruppe der natürlichen Zahlen 1, 2, 3 und 4;
- jedes Y bedeutet ein Strukturelement, welches in der Struktur Q(YZₑ)_{b} das polyalicyclische Strukturelement Q mit e Strukturelementen Z verknüpft und ein Strukturelement enthält oder aus diesem besteht, ausgewählt aus der Gruppe bestehend aus 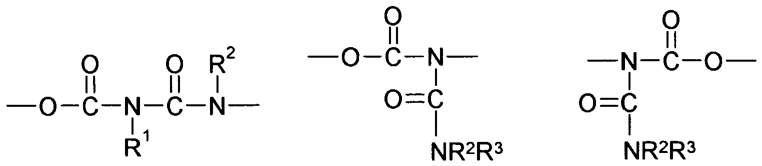 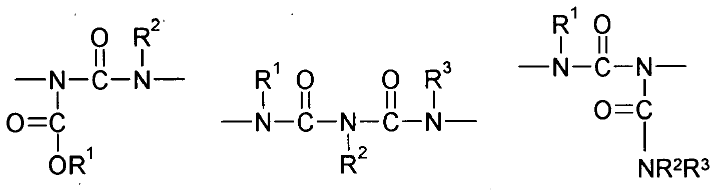 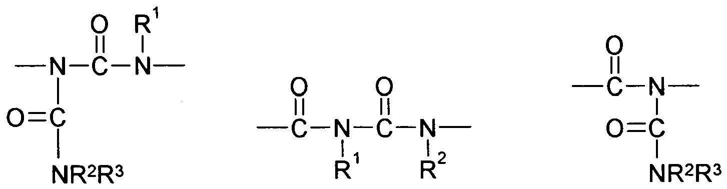 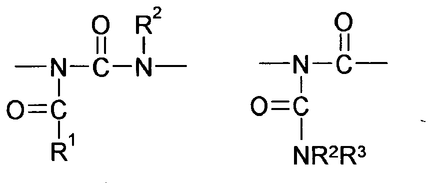
wobei R¹, R² und R³ sonstige Reste der Verbindung bedeuten, wobei die jeweils im Formelbild links angeordnete Bindung dem Strukturelement Q und die rechts angeordnete Bindung dem Strukturelement Z näher ist.

2. Verbindung nach Anspruch 1, wobei die Verbindung herstellbar ist durch Umsetzung eines ersten Umsetzungsproduktes, welches das Umsetzungsprodukt ist aus einer ersten Reaktion von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH
mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH,
wobei gilt:
- R bedeutet jeweils unabhängig von etwaigen weiteren R ein Wasserstoffatom oder einen Alkylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,
wobei die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

3. Verbindung nach Anspruch 2, wobei die Verbindung ein zweites Umsetzungsprodukt ist aus einer Reaktion von dem obigen ersten Umsetzungsprodukt mit
C) einer weiteren Verbindung gemäß A) oder B), wobei jede weitere Verbindung gemäß A) bzw. B) dieselbe Bedeutung hat wie in der ersten Reaktion,
und/oder
wobei die Verbindung ein drittes Umsetzungsprodukt ist aus einer Reaktion von dem obigen zweiten Umsetzungsprodukt mit
D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

4. Verbindung nach Anspruch 1, 2 oder 3, umfassend ein Strukturelement ausgewählt aus der Gruppe bestehend aus 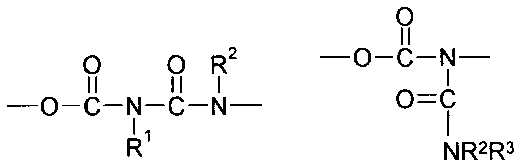 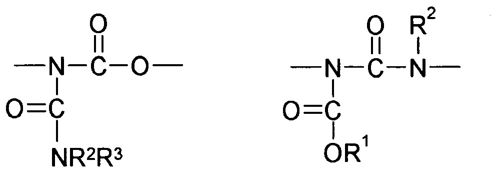 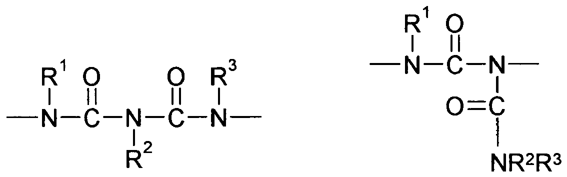 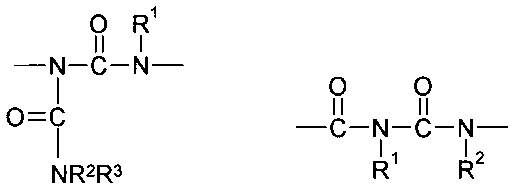 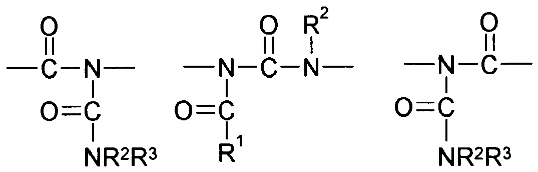 wobei R¹, R² und R³ sonstige Reste der Verbindung bedeuten und Q die oben genannte Bedeutung hat und der Index n ausgewählt ist aus der Gruppe bestehend aus 0 und 1.

5. Verbindung nach einem der vorangehenden Ansprüche, wobei Q ein polyalicyclisches Strukturelement bedeutet, vorzugsweise ein gesättigtes polyalicyclisches Strukturelement, das ausgewählt ist aus der Gruppe bestehend aus bicyclischen und tricyclischen Kohlenwasserstoffresten.

6. Verbindung nach einem der vorangehenden Ansprüche, wobei mindestens ein Strukturelement YZₑ unabhängig von dem oder den weiteren Strukturelementen YZₑ ausgewählt ist und wobei vorzugsweise sämtliche Strukturelemente YZₑ ausgewählt sind aus der Gruppe bestehend aus 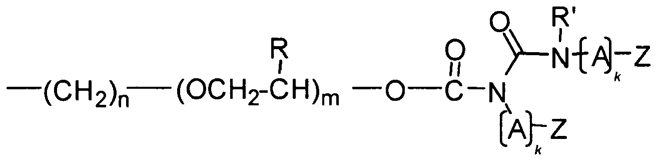 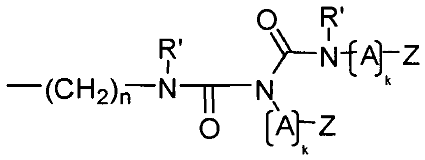 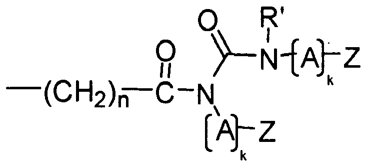 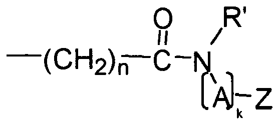 wobei Z, R, m sowie n die obige Bedeutung haben und worin ferner gilt:
- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

7. Verbindung nach einem der Ansprüche 1 bis 5, wobei mindestens ein Strukturelement YZₑ unabhängig von dem oder den weiteren Strukturelementen YZₑ ausgewählt ist und wobei vorzugsweise sämtliche Strukturelemente YZₑ ausgewählt sind aus der Gruppe bestehend aus 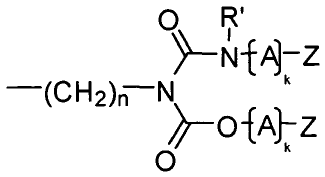 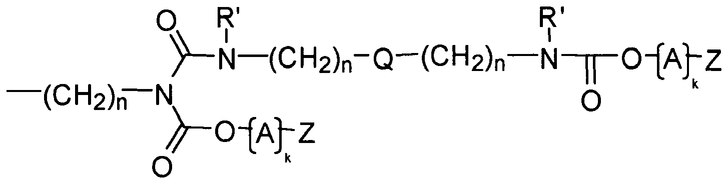 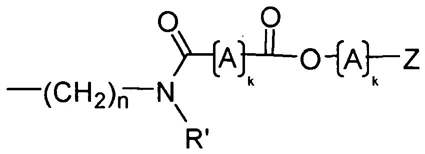 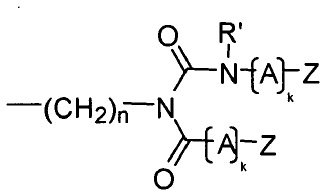 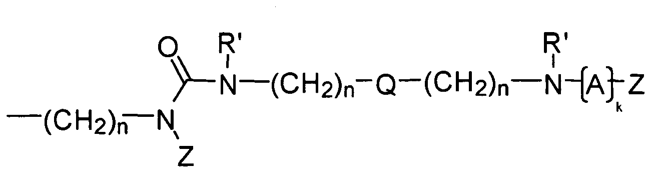 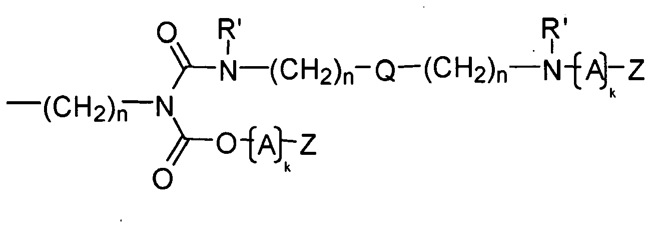 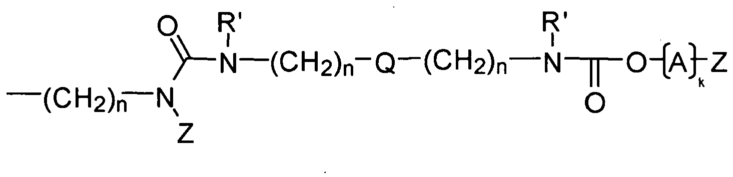 wobei jedes Q unabhängig von etwaigen weiteren Strukturelementen Q die obige Bedeutung hat, und
wobei Z und n die oben genannte Bedeutung haben und wobei ferner gilt:
- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-(A)ₖ-Z, wobei A, Z und k wiederum die obigen Bedeutungen haben.

8. Verbindung nach einem der Ansprüche 6 oder 7, wobei jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, wobei die gegebenenfalls vorhandenen Heteroatome vorzugsweise gewählt sind aus der Gruppe bestehend aus N und O.

9. Verfahren zur Herstellung einer Verbindung Q(YZₑ)_{b}, vorzugsweise nach einem der vorangehenden Ansprüche, oder einer Mischung umfassend zumindest eine Verbindung Q(YZₑ)_{b}, mit folgenden Schritten:
In einer ersten Reaktion Umsetzen von
A) einer Verbindung der Struktur QG_{b}, worin jedes G eine reaktive Gruppe bedeutet, die unabhängig von weiteren Gruppen G ausgewählt ist aus der Gruppe bestehend aus (-CH₂)ₙ-NH₂, (-CH₂)ₙ-(OCH₂-CHR)ₘ-OH, (-CH₂)ₙ-NCO und (-CH₂)ₙ-COOH
mit
B) zwei oder mehr gleichen oder verschiedenen Verbindungen MZₑ, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, -NH₂, -OH, -NCO und -COOH
zu einem ersten Umsetzungsprodukt,
in einer zweiten Reaktion Umsetzen des ersten Umsetzungsproduktes mit
C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
zu einem zweiten Umsetzungsprodukt
und gegebenenfalls in einer dritten Reaktion Umsetzen des zweiten Umsetzungsproduktes mit
D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.
wobei Q, b, Y, Z, e, M, R, m und n jeweils die oben genannte Bedeutung haben, und
wobei das Verhältnis der Gesamtzahl von NCO-Gruppen zu der Gesamtzahl von - NH₂, -OH und -COOH in der Gesamtzahl von Verbindungen gemäß A) und B) in der ersten, der zweiten und gegebenenfalls der dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

10. Mischung umfassend ein, zwei oder mehr unterschiedliche Verbindungen nach einem der Ansprüche 1 - 8, herstellbar nach einem Verfahren gemäß Anspruch 9.

11. Härtbares Gemisch, umfassend
(a) eine oder mehrere Verbindungen nach einem der Ansprüche 1 - 8 oder eine Mischung nach Anspruch 10,
und
(b) einen oder mehrere weitere Bestandteile gewählt aus der Gruppe bestehend aus
(b-1) von Bestandteil (a) verschiedene Monomere, welche mit Bestandteil (a) co-polymerisierbar sind, bevorzugt gewählt aus der Gruppe bestehend aus Acrylaten und Methacrylaten,
(b-2) einen oder mehrere Füllstoffe, vorzugsweise einen oder mehrere nanoskalige Füllstoffe,
(b-3) Photoinitiatoren und Initiatoren für die chemische Aushärtung,
(b-4) Polymerisationsinhibitoren,
und
(b-5) Lösungsmittel.

12. Erzeugnis, erhältlich durch Härten einer Verbindung nach einem der Ansprüche 1 - 8, einer Mischung nach Anspruch 10 oder eines Gemisches nach Anspruch 11.

13. Verbindung nach einem der Ansprüche 1 - 8, Mischung nach Anspruch 10, Gemisch nach Anspruch 11 oder Erzeugnis nach Anspruch 12 zur Anwendung in einem dentalen Verfahren als Dentalmaterial, insbesondere als dentales Füllungsmaterial, dentales Unterfüllungsmaterial, als fließfähiges Kompositmaterial (Flow-Material), als Fissurenversiegler, als Wurzelkanalfüll- und -versiegelungsmaterial, als provisorisches Kronenmaterial, als provisorisches Brückenmaterial und/oder als Stumpfaufbaumaterial.

14. Verwendung einer Verbindung nach einem der Ansprüche 1 - 8, einer Mischung nach Anspruch 10 oder eines Gemisches nach Anspruch 11
- in oder zur Herstellung von Kleb-, Farb-, Lack-, Anstrichs- oder Beschichtungszusammensetzungen, Vergussmassen, Dichtungsmassen, Spachtelmassen, Laminierharzen, Formmassen, Bindemitteln oder Gießharzen,
- zum Einkapseln elektrischer oder elektronischer Bauteile,
- zur Herstellung von magnetischen Aufzeichnungsmaterialien, mikromechanischen Teilen, optischen Schaltern, Glasfaserbeschichtungen, dreidimensionalen Gegenständen mittels Stereolithographie, Druckplatten oder Verbundwerkstoffen,
- für photographische Reproduktionsverfahren oder holographische Aufnahmematerialien,
- als Haftmittel.

15. Verfahren zur Herstellung eines Erzeugnisses, vorzugsweise eines dentalen Erzeugnisses, mit folgenden Schritten:
(i) Bereitstellen einer Verbindung nach einem der Ansprüche 1 - 8, einer Mischung nach Anspruch 10 oder eines Gemisches nach Anspruch 11 als erste Komponente,
(ii) gegebenenfalls Herstellen einer Zubereitung durch Vermischen der bereitgestellten ersten Komponente mit einer oder mehreren weiteren Komponenten, vorzugsweise mit einem oder mehreren weiteren dentalen Materialien,
(iii) Härten des oder der Bestandteile aus Schritt (i) oder der Zubereitung gemäß Schritt (ii), wobei das Härten vorzugsweise chemisch und/oder durch Licht induziert und/oder thermisch induziert erfolgt.

## Claims

1. A compound of the structure Q(YZₑ)_{b} with one, two, three, four or more functional groups that are selected from the group consisting of N-acyl urea, allophanate and biuret,
wherein here and below the following applies:
- Q represents a saturated or olefinically unsaturated polyalicyclic structure element selected from the group consisting of bicyclic, tricyclic, tetracyclic, pentacyclic and hexacyclic hydrocarbon residues, wherein none, one, two or more of the hydrogen atoms of this polyalicyclic structure element Q not substituted by substituents YZₑ is or are substituted by alkyl groups, alkoxy groups, halogen atoms or trifluoromethyl groups;
- b is an integer selected from the group of integers 2, 3, 4;
- each Z represents a structure element which, independently of any further structure elements Z, is selected from the group consisting of
-O-(C=O)-CH=CH₂, -O-(C=O)-C(CH₃)=CH₂,
-(C=O)-CH=CH₂, -(C=O)-C(CH₃)=CH₂;
-CH=CH₂ and -C(CH₃)=CH₂;
- each index e is an integer which, independently of any further indices e, is selected from the group of integers 1, 2, 3 and 4;
- each Y represents a structure element which, in the structure Q(YZₑ)ₙ, links the polyalicyclic structure element Q with e structure elements Z and contains or consists of a structure element selected from the group consisting of 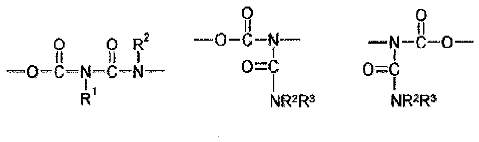 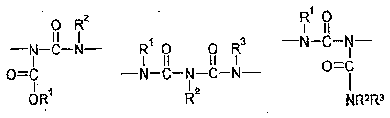 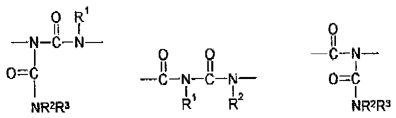 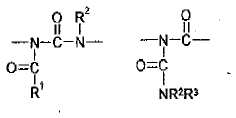
wherein R¹, R² and R³ represent other residues of the compound, wherein in each case the bond arranged on the left of the graphic formula is closer to the structure element Q and the bond arranged on the right is closer to the structure element Z.

2. The compound according to claim 1, wherein the compound can be produced by reacting a first reaction product, which is the reaction product of a first reaction of
A) a compound of structure QG_{b} in which each G represents a reactive group which, independently of further groups G, is selected from the group consisting of (-CH₂)ₙ-NH₂, (-CH₂)ₙ-(OCH₂-CHR)ₘ-OH, (-CH₂)ₙ-NCO and (-CH₂)ₙ-COOH
with
B) two or more identical or different compounds MZₑ, wherein M represents a structure element which in each case has one or more grouping(s) that react with the reactive groups G selected from the group consisting of -NH, -NH₂, -OH, -NCO and -COOH,
wherein the following applies:
- R, in each case independently of any further R, represents a hydrogen atom or an alkyl residue;
- m is an integer selected from the group of integers from 0 to 10,
- each index n is an integer which, independently of any further indices n, is selected from the group consisting of 0 and 1,
wherein the compound is a second reaction product from a reaction of the above first reaction product with
C) a further compound according to A) or B), wherein each further compound according to A) or B) has the above meaning, independently of the meaning of A) and/or B) in the first reaction,
or
the compound is a third reaction product from a reaction of the above second reaction product with
D) a further compound according to A) or B), wherein each further compound according to A) or B) has the above meaning, independently of the meaning of A) and/or B) in the first and/or second reaction.

3. The compound according to claim 2, wherein the compound is a second reaction product from a reaction of the above first reaction product with
C) a further compound according to A) or B), wherein each further compound according to A) or B) has the same meaning as in the first reaction,
and/or
wherein the compound is a third reaction product from a reaction of the above second reaction product with
D) a further compound according to A) or B), wherein each further compound according to A) or B) has the same meaning as in the first and/or the second reaction, preferably as in the first and the second reaction.

4. The compound according to claim 1, 2 or 3, comprising a structure element selected from the group consisting of 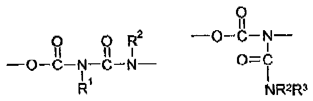 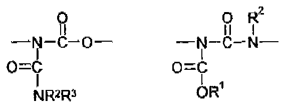 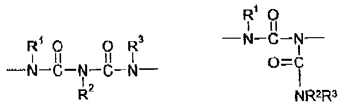 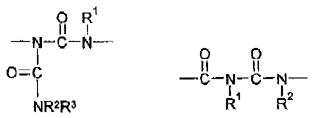 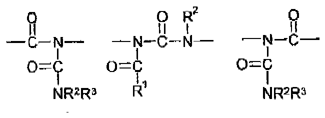 wherein R¹, R² and R³ represent other residues of the compound and Q has the above-mentioned meaning and the index n is selected from the group consisting of 0 and 1.

5. The compound according to one of the preceding claims, wherein Q represents a polyalicyclic structure element, preferably a saturated polyalicyclic structure element, which is selected from the group consisting of bicyclic and tricyclic hydrocarbon residues.

6. The compound according to one of the preceding claims, wherein at least one structure element YZₑ is selected independently of the other structure element(s) YZₑ and wherein preferably all structure elements YZₑ are selected from the group consisting of 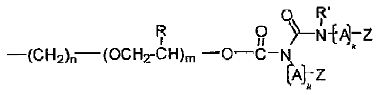 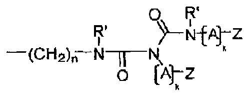 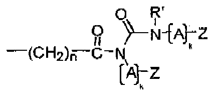 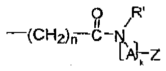 wherein Z, R, m and n have the meaning given above and wherein the following also applies:
- each A represents an organic structure element,
- each index k is an integer which, independently of any further indices k, is selected from the group consisting of 0 and 1;
- each R' represents a structure element which, independently of any further structure elements R', is selected from the group consisting of H and a structure element (C=O)-NH-(A)ₖ-Z, wherein A, Z and k in turn have the meanings given above.

7. The compound according to one of claims 1 to 5, wherein at least one structure element YZₑ is selected independently of the other structure element(s) YZₑ and wherein preferably all structure elements YZₑ are selected from the group consisting of 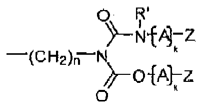 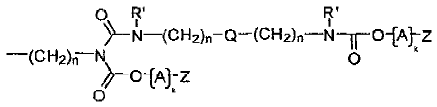 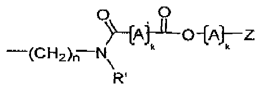 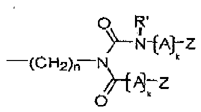 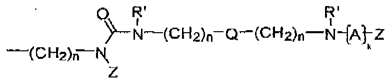 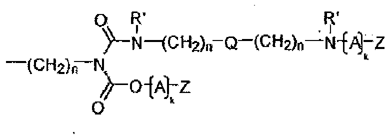 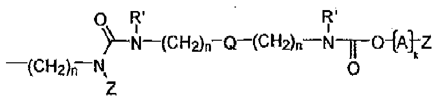 wherein each Q, independently of any further structure elements Q, has the above meaning, and
wherein Z and n have the above-mentioned meaning and wherein the following also applies:
- each A represents an organic structure element,
- each index k is an integer which, independently of any further indices k, is selected from the group consisting of 0 and 1;
- each R' represents a structure element which, independently of any further structure elements R', is selected from the group consisting of H and a structure element (C=O)-NH-(A)ₖ-Z, wherein A, Z and k in turn have the above meanings.

8. The compound according to one of claims 6 or 7, wherein each structure element A, independently of any further structure elements A, is selected from the group consisting of linear, branched or ring-comprising structure elements with 1 to 25 C atoms and 0 to 10 heteroatoms, wherein the heteroatoms that are optionally present are preferably selected from the group consisting of N and O.

9. A method of producing a compound Q(YZₑ)_{b}, preferably according to one of the preceding claims, or a mixture comprising at least one compound O(YZₑ)_{b}, with the following steps:
in a first reaction, reacting
A) a compound of structure QG_{b}, in which each G represents a reactive group which, independently of further groups G, is selected from the group consisting of (-CH₂)ₙ-NH₂, (-CH₂)ₙ-(OCH₂-CHR)ₘ-OH, (-CH₂)ₙ-NCO and (-CH₂)ₙ-COOH
with
B) two or more identical or different compounds MZₑ, wherein M represents a structure element which in each case has one or more grouping(s) that react with the reactive groups G selected from the group consisting of -NH, -NH₂, -OH, -NCO and -COOH
to form a first reaction product,
in a second reaction, reacting the first reaction product with
C) a further compound according to A) or B), wherein each further compound according to A) or B) has the above meaning, independently of the meaning of A) and/or B) in the first reaction,
to form a second reaction product,
and optionally in a third reaction, reacting the second reaction product with
D) a further compound according to A) or B), wherein each further compound according to A) or B) has the above meaning, independently of the meaning of A) and/or B) in the first and/or second reaction,
wherein Q, b, Y, Z, e, M, R, m and n in each case have the above meanings,
and
wherein the ratio of the total number of NCO groups to the total number of -NH₂, -OH and -COOH in the total number of compounds according to A) and B) in the first, the second and optionally the third reaction is greater than or equal to 1, preferably in the range of 1.1:1 to 5:1, more preferably in the range of 1.25:1 to 4:1, particularly preferably in the range of 1.5:1 to 3:1 and most preferably in the range of 2:1 to 2.6.1.

10. A mixture comprising one, two or more different compounds according to one of claims 1 - 8, 8, which can be produced by a method according to claim 9.

11. A curable blend, comprising:
(a) one or more compounds according to one of claims 1 - 8 or a mixture according to claim 10,
and
(b) one or more further components selected from the group consisting of
(b-1) monomers differing from component (a), which are copolymerisable with component (a), preferably selected from the group consisting of acrylates and methacrylates,
(b-2) one or more fillers, preferably one or more nanoscale fillers,
(b-3) photoinitiators and initiators for chemical curing,
(b-4) polymerisation inhibitors
and
(b-5) solvents.

12. A product obtainable by curing a compound according to one of claims 1 - 8, a mixture according to claim 10 or a blend according to claim 11.

13. A compound according to one of claims 1 - 8, a mixture according to claim 10, a blend according to claim 11 or a product according to claim 12 for use in a dental procedure as a dental material, in particular as a dental filling material, a dental cavity lining material, a flowable composite (flow material), a fissure sealant, a root canal filling and sealing material, a temporary crown material, a temporary bridge material and/or a core build-up material.

14. Use of a compound according to one of claims 1 - 8, a mixture according to claim 10 or a blend according to claim 11
- in or for the production of adhesive, colour, lacquer, paint or coating compositions, encapsulating compounds, sealants, knifing fillers, laminating resins, moulding compositions, binders or casting resins,
- for the encapsulation of electrical or electronic components,
- for the production of magnetic recording materials, micromechanical parts, optical switches, glass fibre coatings, three-dimensional objects by means of stereolithography, printing plates or composite materials,
- for photographic reproduction methods or holographic recording materials,
- as adhesives.

15. A method of producing a product, preferably a dental product, with the following steps:
(i) preparing a compound according to one of claims 1 - 8, a mixture according to claim 10 or a blend according to claim 11 as the first component,
(ii) optionally producing a preparation by mixing the first prepared component with one or more further components, preferably with one or more further dental materials,
(iii) curing the component(s) from step (i) or the preparation according to step (ii), wherein the curing preferably takes place chemically and/or is light induced and/or thermally induced.

## Revendications

1. Composé de structure (Q(YZₑ)_{b} comportant un, deux, trois ou quatre groupes fonctionnels, ou plus, lesquels sont choisis parmi le groupe constitué de N-acylurée, d'allophanate et de biuret,
dans lequel s'applique ce qui suit :
- Q représente un élément structurel polyalicyclique saturé ou oléfiniquement insaturé, choisi parmi le groupe constitué de résidus hydrocarbures bicycliques, tricycliques, tétracycliques, pentacycliques et hexacycliques, sachant qu'aucun, un seul, deux ou plus des atomes d'hydrogène de cet élément structurel polyalicyclique Q, non substitués par des substituants YZₑ, est ou sont substitué(s) par des groupes aryle, des groupes alcoxy, des atomes d'halogène ou des groupes trifluorométhyle ;
- b est un nombre naturel choisi parmi le groupe constitué des nombres naturels 2, 3, 4 ;
- chaque Z représente un élément structurel qui est choisi indépendamment des éventuels autres éléments structurels Z, parmi le groupe constitué de :
-O-(C=O)-CH=CH₂, -O-(C=O)-C(CH₃)=CH₂ ;
-(C=O)-CH=CH₂, -(C=O)-C(CH₃)=CH₂ ;
-CH=CH₂ et -C(CH₃)=CH₂ ;
- chaque indice e est un nombre naturel, qui est choisi indépendamment des éventuels autres indices e, parmi le groupe constitué des nombres naturels 1, 2, 3 et 4 ;
- chaque Y représente un élément structurel, qui combine dans la structure Q(YZₑ)_{b} l'élément structurel polyalicyclique à e éléments structurels Z et qui contient un élément structurel ou est constitué de ce dernier, choisi parmi le groupe constitué de 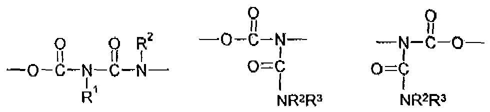 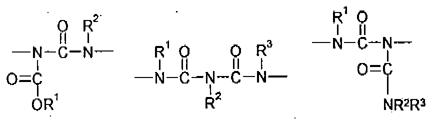 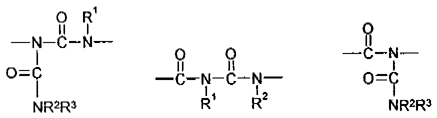 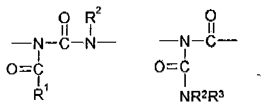
dans lequel R¹, R² et R³ représentent d'autres résidus du composé, sachant que la liaison disposée à gauche respectivement sur l'image des formules est plus proche de l'élément structurel Q et que la liaison disposée à droite est plus proche de l'élément structurel Z.

2. Composé selon la revendication 1, sachant que le composé peut être produit par la mise en réaction d'un premier produit de réaction, qui est le produit de réaction obtenu d'une première réaction de
A) un composé de structure QG_{b}, où chaque G représente un groupe réactif, qui est choisi indépendamment d'autres groupes G, parmi le groupe constitué de (-CH₂)ₙ-NH₂, (-CH₂)ₙ-(OCH₂-CHR)ₘ-OH, (-CH₂)ₙ-NCO et (-CH₂)ₙ-COOH
avec
B) deux composés MZₑ ou plus, identiques ou différents, dans lesquels M représente un élément structurel qui présente respectivement un ou plusieurs groupements réactifs par rapport aux groupes réactifs G, choisi parmi le groupe constitué de -NH, -NH₂, -OH, - NCO et -COOH,
dans lequel:
- R représente, respectivement indépendamment des éventuels autres R, un atome d'hydrogène ou un résidu alkyle ;
- m est un nombre naturel choisi parmi le groupe constitué des nombres naturels allant de 0 à 10 ;
- chaque indice n est un nombre naturel, qui est choisi indépendamment d'éventuels autres indices n, parmi le groupe constitué de 0 et de 1,
dans lequel le composé est un deuxième produit de réaction obtenu d'une réaction du premier produit de réaction ci-dessus avec
C) un autre composé selon A) ou B), dans lequel chaque autre composé selon A) ou B) a la signification ci-dessus, indépendamment de la signification de A) ou de B) dans la première réaction,
ou
que le composé est un troisième produit de réaction obtenu d'une réaction du deuxième produit de réaction ci-dessus avec
D) un autre composé selon A) ou B), sachant que chaque autre composé selon A) ou B) a la signification ci-dessus, indépendamment de la signification de A) ou de B) dans la première ou la deuxième réaction.

3. Composé selon la revendication 2, sachant que le composé est un deuxième produit de réaction obtenu d'une réaction du premier produit de réaction ci-dessus avec
C) un autre composé selon A) ou B), sachant que chaque autre composé selon A) ou B) a la même signification que dans la première réaction,
et/ou
sachant que le composé est un troisième produit de réaction obtenu d'une réaction avec le deuxième produit de réaction ci-dessus avec
D) un autre composé selon A) ou B), sachant que chaque autre composé selon A) ou B) a la même signification que dans la première et/ou la deuxième réaction, de préférence comme dans la première et la deuxième réaction.

4. Composé selon la revendication 1, 2 ou 3, comportant un élément structurel choisi parmi le groupe constitué de : 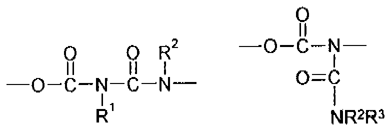 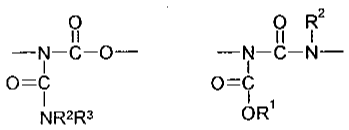 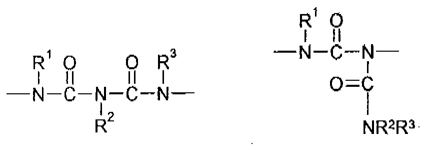 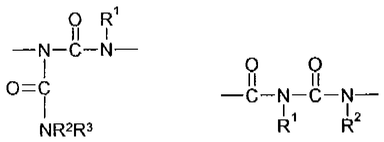 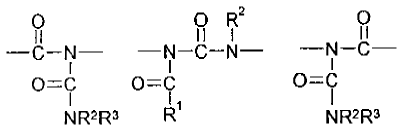 dans lequel R¹, R² et R³ représentent les autres résidus du composé et Q a la signification évoquée ci-dessus et l'indice n est choisi parmi le groupe constitué de 0 et de 1.

5. Composé selon l'une quelconque des revendications précédentes, dans lequel Q représente un élément structurel polyalicyclique, de préférence un élément structurel polyalicyclique saturé, qui est choisi parmi le groupe constitué des résidus d'hydrocarbures bicycliques et tricycliques.

6. Composé selon l'une quelconque des revendications précédentes, dans lequel au moins un élément structurel YZₑ est choisi indépendamment de l'élément structurel ou des autres éléments structurels YZₑ, et que de préférence tous les éléments structurels YZₑ sont choisis parmi le groupe constitué de : 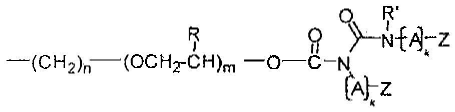 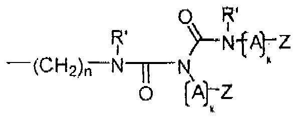 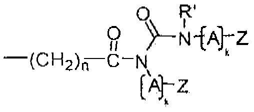 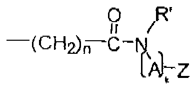 dans lesquels Z, R, m ainsi que n ont la signification exposée ci-dessus et qu'en outre s'applique ce qui suit :
- chaque A représente un élément structurel organique ;
- chaque indice k est un nombre naturel, qui est choisi indépendamment des éventuels autres indices k parmi le groupe constitué de 0 et 1 ;
- chaque R' représente un élément structurel, qui est choisi indépendamment des éventuels autres éléments structurels R' parmi le groupe constitué de H et d'un élément structurel (C=O)-NH-(A)ₖ-Z, sachant que A, Z et k ont à nouveau la signification exposée ci-dessus.

7. Composé selon l'une quelconque des revendications 1 à 5, dans lequel au moins un élément structurel YZₑ est choisi indépendamment de l'élément structurel ou des autres éléments structurels YZₑ, et que de préférence tous les éléments structurels YZₑ sont choisis parmi le groupe constitué de : 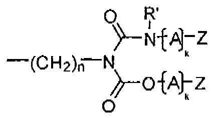 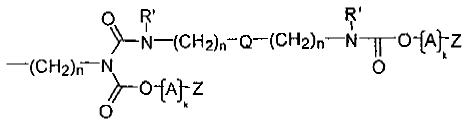 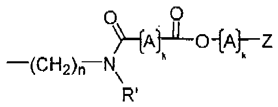 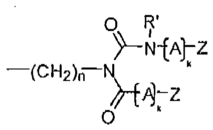 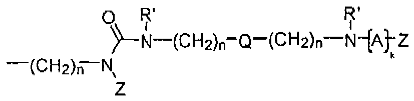 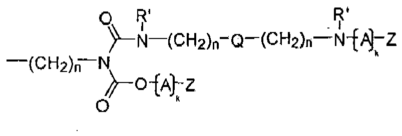 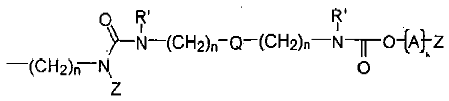 dans lesquels chaque Q a indépendamment des éventuels autres éléments structurels Q la signification exposée ci-dessus, et
dans lesquels Z et n ont la signification évoquée ci-dessus et qu'en outre s'applique ce qui suit :
- chaque A représente un élément structurel organique,
- chaque indice k est un nombre naturel qui est choisi indépendamment des éventuels autres indices k parmi le groupe constitué de 0 et de 1 ;
- chaque R' représente un élément structurel qui est choisi indépendamment des éventuels autres éléments structurels R' parmi le groupe constitué de H et d'un élément structurel (C=O)-NH-(A)ₖ-Z, sachant que A, Z et k ont à nouveau la signification exposée ci-dessus.

8. Composé selon l'une quelconque des revendications 6 ou 7, sachant que chaque élément structurel A est choisi indépendamment des éventuels autres éléments structurels A parmi le groupe constitué des éléments structurels linéaires, ramifiés ou comportant des composés cycliques avec 1 à 25 atomes de carbone et 0 à 10 hétéroatomes, sachant que les hétéroatomes éventuellement présents sont de préférence choisis parmi le groupe constitué de N et de O.

9. Procédé de production d'un composé Q(YZₑ)_{b}, de préférence selon l'une quelconque des revendications précédentes, ou d'un mélange comportant au moins un composé Q(YZₑ)_{b}, comportant les étapes suivantes consistant à :
dans une première réaction, faire réagir
A) un composé de structure QG_{b}, où chaque G représente un groupe réactif, qui est choisi indépendamment des autres groupes G parmi le groupe constitué de (-CH₂)ₙ-NH₂, (-CH₂)ₙ-(OCH₂-CHR)ₘ-OH, (-CH₂)ₙ-NCO et (-CH₂)ₙ-COOH
avec
B) deux composés MZₑ ou plus, identiques ou différents , dans lesquels M représente un élément structurel, qui présente respectivement un ou plusieurs groupements réactifs par rapport aux groupes G réactifs, choisi parmi le groupe constitué de -NH, -NH₂, -OH,-NCO et -COOH
pour obtenir un premier produit de réaction,
dans une deuxième réaction faire réagir le premier produit de réaction avec
C) un autre composé selon A) ou B), sachant que chaque autre composé selon A) ou B) a la signification ci-dessus, indépendamment de la signification de A) ou B) dans la première réaction,
pour obtenir un deuxième produit de réaction,
et éventuellement dans une troisième réaction, faire réagir le deuxième produit de réaction avec
D) un autre composé selon A) ou B), sachant que chaque autre composé selon A) ou B) a la signification ci-dessus, indépendamment de la signification de A) ou B) dans la première ou la deuxième réaction,
dans lequel Q, b, Y, Z, e, M, R, m et n ont respectivement la signification évoquée ci-dessus,
et
sachant que le rapport du nombre total de groupes NCO avec le nombre total de -NH₂, -OH et -COOH dans le nombre total de composés selon A) et B) est dans les première, deuxième et éventuellement troisième réactions est supérieur ou égal à 1, et se situe de préférence dans la plage de 1,1:1 à 5:1, de manière davantage préférée dans la plage de 1,25:1 à 4:1, de manière particulièrement préférée dans la plage de 1,5:1 à 3:1, et de manière préférée entre toutes dans la plage de 2:1 à 2,5:1.

10. Mélange comportant un ou deux composés différents, ou plus, selon l'une quelconque des revendications 1 à 8, pouvant être produit selon un procédé selon la revendication 9.

11. Mélange durcissable, comportant
(a) un ou plusieurs composés selon l'une quelconque des revendications 1 à 8 ou un mélange selon la revendication 10,
et
(b) un ou plusieurs autres composants choisis parmi le groupe constitué
(b-1) de monomères différents du composant (a), lesquels peuvent être copolymérisés avec le composant (a), et sont de préférence choisis parmi le groupe constitué d'acrylates et de méthacrylates,
(b-2) d'une ou de plusieurs matières de charge, de préférence d'une ou de plusieurs matières de charges nanométriques,
(b-3) de photo-initiateurs et d'initiateurs pour le durcissement chimique,
(b-4) d'inhibiteurs de polymérisation,
et
(b-5) de solvants.

12. Produit obtenu par durcissement d'un composé selon l'une quelconque des revendications 1 à 8, d'un mélange selon la revendication 10 ou d'un mélange selon la revendication 11.

13. Composé selon l'une quelconque des revendications 1 à 8, mélange selon la revendication 10, mélange selon la revendication 11 ou produit selon la revendication 12 à utiliser dans un procédé dentaire comme matériau dentaire, en particulier comme matériau de remplissage dentaire, matériau de sous-remplissage dentaire, matériau composite à écoulement libre (Flow-material), produit de jointement de fissures, matériau de remplissage et de jointement de canal de racine, matériau de couronne provisoire, matériau de bridge provisoire et/ou matériau de reconstitution de moignon.

14. Utilisation d'un composé selon l'une quelconque des revendications 1 à 8, d'un mélange selon la revendication 10 ou d'un mélange selon la revendication 11
- dans ou pour la production de compositions de collage, de couleurs, de vernis, de peinture ou de revêtement, de masses de remplissage, de masses d'étanchéité, de mastics, de résines de laminage, de matières de moulage, de liants ou de résines de coulée,
- pour encapsuler des composants électriques ou électroniques,
- pour produire des matériaux d'enregistrement magnétiques, des pièces micromécaniques, des commutateurs optiques, des revêtements à base de fibres de verre, des objets tridimensionnels au moyen de la stéréolithographie, des plaques d'impression ou des matériaux composites,
- pour des procédés de reproduction photographique ou pour des matériaux de réception holographiques,
- en tant qu'adhésif.

15. Procédé de production d'un produit, en particulier d'un produit dentaire, comportant les étapes suivantes consistant à :
(i) fournir un composé selon l'une quelconque des revendications 1 à 8, un mélange selon la revendication 10 ou un mélange selon la revendication 11 comme premiers composants ;
(ii) éventuellement produire une préparation en mélangeant les premiers composants fournis avec un ou plusieurs autres composants, de préférence avec un ou plusieurs autres matériaux dentaires,
(iii) durcir le ou les composants issus de l'étape (i) ou la préparation selon l'étape (ii), sachant que le durcissement se fait de préférence par voie chimique et/ou de manière induite par la lumière et/ou par la température.
